(19) Europäisches Patentamt
European Patent Office
Office européen des brevets

(11) EP 1 507 794 B1

(12) **FASCICULE DE BREVET EUROPEEN**

(45) Date de publication et mention de la délivrance du brevet:
**23.08.2006 Bulletin 2006/34**

(21) Numéro de dépôt: **03756015.8**

(22) Date de dépôt: **28.05.2003**

(51) Int Cl.:
*C07K 5/08* (2006.01) *C07K 5/10* (2006.01)
*C07K 14/705* (2006.01) *C07K 14/525* (2006.01)
*A61K 38/04* (2006.01) *A61K 38/16* (2006.01)
*A61P 31/00* (2006.01) *A61P 33/00* (2006.01)
*A61P 35/00* (2006.01) *A61P 37/00* (2006.01)

(86) Numéro de dépôt international:
**PCT/FR2003/001613**

(87) Numéro de publication internationale:
**WO 2003/102207 (11.12.2003 Gazette 2003/50)**

(54) **NOUVELLES MOLECULES MULTIMERIQUES, LEUR PROCEDE DE PREPARATION, ET LEUR UTILISATION POUR LA PREPARATION DE MEDICAMENTS**

NEUE MULTIMERISCHE MOLEKÜLE, VERFAHREN ZU DEREN HERSTELLUNG, UND DEREN VERWENDUNG ZUR HERSTELLUNG VON ARZNEIMITTELN

NOVEL MULTIMERIC MOLECULES, THE PREPARATION METHOD THEREOF AND USE OF SAME FOR THE PREPARATION OF MEDICAMENTS

(84) Etats contractants désignés:
**DE ES FR GB**

(30) Priorité: **30.05.2002 FR 0206631**

(43) Date de publication de la demande:
**23.02.2005 Bulletin 2005/08**

(73) Titulaire: **CENTRE NATIONAL DE LA RECHERCHE SCIENTIFIQUE**
**75794 Paris Cedex 16 (FR)**

(72) Inventeurs:
- **GUICHARD, Gilles François Roger**
**67202 Wolfisheim (FR)**
- **FOURNEL, Sylvie Victorine Lucienne**
**67200 Strasbourg (FR)**
- **BIANCO, Alberto**
**67000 Strasbourg (FR)**
- **HOEBEKE, Johan Félicien**
**F-67300 Schiltigheim (FR)**
- **MULLER, Sylviane**
**67600 Strasbourg (FR)**

(74) Mandataire: **Grosset-Fournier, Chantal Catherine**
**Grosset-Fournier & Demachy,**
**54, rue Saint-Lazare**
**75009 Paris (FR)**

(56) Documents cités:
**WO-A-01/49866** **WO-A-02/00893**
**WO-A-99/52877**

- **G AVERSA ET AL.: "CD40 ligand-CD40 interaction in Ig isotype switching in mature and immature human B cells" SEMINARS IN IMMUNOLOGY., vol. 6 , 1994, pages 295-301, XP002237774 W.B. SAUNDERS COMPANY, PA., US ISSN: 1044-5323**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 507 794 B1

Printed by Jouve, 75001 PARIS (FR)

## Description

L'invention a pour objet de nouvelles molécules multimériques, leur procédé de préparation, ainsi que leur utilisation pour la préparation de médicaments.

L'invention a également pour objet des molécules capables d'activer ou d'inhiber la réponse immunitaire.

L'importance du couple CD40/CD40L dans la réponse immunitaire a amené de nombreux groupes à utiliser des anticorps dirigés contre ces deux molécules à des fins thérapeutiques, de manière à inhiber ou activer le système immunitaire. L'administration d'anticorps anti-CD40L a donné des résultats encourageants dans le traitement de maladies auto-immunes comme l'encéphalomyélite allergique expérimentale murine (un modèle de la sclérose en plaques humaine)(Howard et al., 1999) ou dans le traitement de rejet d'allogreffes rénales chez les singes (Kirk et al., 1999). Dans ces deux cas, les anticorps ont inhibé une activité néfaste du système immunitaire. Inversement, l'utilisation d'anticorps anti-CD40 agonistes a permis d'une part, d'améliorer fortement la réponse à des vaccins anti-tumoraux peptidiques chez la souris (Diehl et al., 1999) et d'autre part, d'augmenter l'efficacité des cellules T $CD4^+$ dans la lutte contre des tumeurs murines (Sotomayor et al., 1999 ; Lode et al., 2000). Une régression de tumeur dans des modèles murins a été mise en évidence après injection de cellules dendritiques (CDs) transformées par un adénovirus codant le CD40L (Kikuchi et al., 2000). Enfin, une activation des cellules dendritiques par l'interaction de leur molécule CD40 avec CD40L est capable de protéger des souris d'une infection par un parasite, *Trypanosoma Cruzi* (Chaussabel et al., 1999). Dans tous ces travaux, la valence particulière de la molécule CD40L, qui s'associe sous forme de trimère pour former avec le CD40 des complexes hexavalents, rend difficile la production d'anticorps fonctionnels capables d'interférer avec les fonctions du couple CD40/CD40L. Le développement des adénovirus codant pour CD40L répond en partie à cet inconvénient. Cependant, leur utilisation n'est pas sans poser de problèmes chez l'homme. Enfin, la valence particulière du système rend difficile la découverte de molécules de synthèse capables d'interférer avec l'interaction CD40/CD40L.

La demande internationale WO 99/52877 décrit des ligands agonistes ou antagonistes de récepteur multimérique qui ne sont pas des anticorps, ledit récepteur étant notamment CD40. Les composés ainsi obtenus sont des ligands du récepteur multimérique et ils comprennent un composé espaceur qui est substitué par au moins deux résidus de liaison du récepteur. Ledit composé espaceur peut être toute molécule ayant un centre di- ou trisubstitué. Cependant, ce document D1 décrit de nombreuses molécules sans les définir précisément, par exemple par leur formule chimique globale, et il décrit uniquement les espaceurs di- ou trisubstitués des composés multimériques sans donner de détails sur la structure précise de la partie protéique desdits composés multimériques. Enfin, les ligands de CD40 préparés dans le cadre de ce document sont des ligands dimériques.

Le document D2 décrit les interactions entre CD40L et CD40 dans l'activation des lymphocytes B et T. Il ne concerne donc en rien la préparation d'un mime multimérique synthétique d'un ligand naturel.

L'invention a pour but de fournir des ligands multimériques conçus pour interférer dans des interactions protéine-protéine.

La présente invention a également pour but la préparation de molécules pouvant interférer avec des interactions multivalentes protéines-protéines.

La présente invention a également pour but la préparation de molécules pouvant moduler l'activité des membres des familles du TNF et du TNF-R.

La présente invention a pour but de fournir une molécule de synthèse agissant sur le système CD40/CD40L.

La présente invention a également pour but de fournir des molécules pouvant agir comme adjuvants ou immunosuppresseurs.

La présente invention concerne une molécule multimérique capable de mimer, avec une activité agoniste ou antagoniste, un ligand multimérique protéique naturel.

La présente invention concerne également une molécule multimérique capable de produire des effets différents de ceux produits par le ligand multimérique naturel appartenant à la famille du TNF, pouvant être bénéfiques dans une pathologie.

Par "molécule capable de mimer un ligand avec une activité agoniste", on désigne une molécule capable de reproduire une partie ou la totalité des fonctions du ligand naturel.

Par "molécule capable de mimer un ligand avec une activité antagoniste", on désigne des molécules une molécule capable d'inhiber une partie ou la totalité des fonctions du ligand naturel.

Par "ligand multimérique protéique naturel", on désigne toute protéine active sur son récepteur sous forme multimérique, à savoir homo-dimérique, homo-trimérique, homo-tétramérique ou homo-oligomérique, par autoassemblage non covalent.

La présente invention concerne une molécule multimérique telle que définie ci-dessus, capable de mimer un ligand de récepteur de la superfamille du TNF.

La "superfamille du TNF" désigne une famille de molécules ayant des caractéristiques structurales ou fonctionnelles proches de celles du TNF, ces molécules étant essentiellement impliquées dans la réponse immunitaire.

Selon un mode de réalisation avantageux de l'invention, la molécule multimérique de l'invention est caractérisée

en ce que le ligand est un ligand de la molécule CD40.

La molécule CD40 est une molécule transmembranaire de 48 kDa qui appartient à la superfamille des "récepteurs au TNF". Elle est exprimée de manière constitutive par les cellules présentatrices de l'antigène telles que les cellules dendritiques, les monocytes et les lymphocytes B. Elle interagit de manière trivalente avec CD40L (CD154) exprimé sur les cellules T activées, les leucocytes (monocytes/macrophages, cellules NK, basophiles, éosinophiles), les plaquettes activées ainsi que sur des cellules non hématopoïétiques (cellules musculaires lisses, cellules épithéliales, cellules endothéliales). A la surface de ces différentes cellules, son expression est inductible et persistante contrairement à son expression sur les cellules T activées qui n'est que transitoire. L'interaction CD40/CD40L est centrale dans le développement et le contrôle des réponses immunitaires humorales et cellulaires.

La présente invention concerne une molécule multimérique telle que définie ci-dessus, caractérisée en ce qu'elle répond à la formule générale suivante :

$$A\text{-}X_n$$

dans laquelle :

- n est égal à 3, 4, 5 ou 6,
- A est un groupe chimique, fonctionnalisé par au moins trois fonctions amines ou fonctions COOH,
- X représente un groupe -D, -B-D ou -B(D)-D', dans lequel :

  * B est un bras espaceur,
  * -D et -D' sont des peptides ou pseudopeptides correspondant à une séquence dérivée du ligand, choisie parmi les résidus formant l'interface avec le récepteur du ligand, laquelle est susceptible d'interagir avec le récepteur.

Le groupe chimique A est un groupe chimique fonctionnalisé de telle sorte qu'il permet la liaison avec le groupe X. A est également appelé "molécule coeur".

Par "molécule coeur", on désigne un groupe chimique présentant un rôle central dans la présentation des n groupes X dans la molécule multimérique.

Par "bras espaceur", on désigne une chaîne organique utilisée pour éloigner le groupe D à la distance désirée de A.

Par "peptides ou pseudopeptides", on désigne un enchaînement de résidus d'acides aminés naturels ou non naturels, reliés entre eux par des liaisons amide. Un pseudopeptide est obtenu par remplacement d'une ou plusieurs liaisons amide dans le peptide par une liaison chimique de nature différente.

Par "séquence dérivée du ligand, choisie parmi les résidus formant l'interface avec le récepteur du ligand", on définit une séquence peptidique appartenant à la séquence primaire du ligand et dont le nombre d'acides aminés est compris entre 3 et 10, et dont des études structurales (diffraction des rayons X, résonance magnétique nucléaire, modélisation moléculaire, mutagenèse dirigée) ont montré qu'au moins un des acides aminés la composant est en interaction non covalente (liaison_ hydrogène, interaction cation-pi, pont salin, interaction hydrophobe, van der Waals) avec un résidu d'acide aminé du récepteur.

La présente invention concerne également une molécule multimérique, caractérisée en ce qu'elle répond à la formule générale suivante :

$$A\text{-}X_n$$

dans laquelle :

- n est égal à 3, 4, 5 ou 6,
- A est un groupe chimique, fonctionnalisé par au moins trois fonctions amines ou fonctions COOH ou fonctions SH ou fonctions S-Npys (S-nitro-pyridinesulfényle) ou fonctions S-Pys (S-pyridinesulfényle), et est notamment différent d'une protéine,
- X représente un groupe -D, -B-D ou -B(D)-D', dans lequel :

  * B est un bras espaceur,
  * -D et -D' représentent des peptides ou pseudopeptides correspondant à une séquence dérivée d'un ligand, choisie parmi les résidus formant l'interface avec le récepteur du ligand, laquelle séquence est susceptible d'interagir avec le récepteur, ledit ligand étant choisi parmi les ligands de récepteurs de la superfamille du TNF, et notamment parmi les ligands suivants : EDA, CD40L, FasL, OX40L, AITRL, CD30L, VEGI, LIGHT, 4-1 BBL, CD27L, LT$\alpha$, TNF, LT$\beta$, TWEAK, APRIL, BLYS, RANKL et TRAIL.

La présente invention concerne également une molécule multimérique, caractérisée en ce qu'elle répond à la formule générale suivante :

$$A\text{-}X_n$$

dans laquelle :

- n est égal à 3, 4, 5 ou 6,
- A est un groupe chimique, fonctionnalisé par au moins trois fonctions amines ou fonctions COOH, et est notamment différent d'une protéine,
- X représente un groupe -D, -B-D ou -B(D)-D', dans lequel :

  * B est un bras espaceur,
  * -D et -D' représentent des peptides ou pseudopeptides correspondant à une séquence dérivée d'un ligand, choisie parmi les résidus formant l'interface avec le récepteur du ligand, laquelle séquence est susceptible d'interagir avec le récepteur, ledit ligand étant choisi parmi les ligands de récepteurs de la superfamille du TNF, et notamment parmi les ligands suivants : EDA, CD40L, FasL, OX40L, AITRL, CD30L, VEGI, LIGHT, 4-1BBL, CD27L, LTα, TNF, LTβ, TWEAK, APRIL, BLYS, RANKL et TRAIL.

L'expression "-D et -D' correspondant à une séquence dérivée d'un ligand, laquelle séquence est susceptible d'interagir avec le récepteur" peut être définie comme suit : les séquences -D et -D' sont choisies sur la base de données structurales disponibles (diffraction des rayons X ou modélisation moléculaire et mutagenèse dirigée) pour leurs implication dans l'interaction avec le récepteur correspondant. Ainsi dans la protéine naturelle, tout ou partie des résidus de ces séquences sont en interaction (liaison hydrogène, liaison hydrophobe, liaison H, liaison cation-Pi, ponts salin, interactions de van der Waals) avec une partie du ou des récepteurs correspondants. Néanmoins les peptides isolés -D ou -D' peuvent avoir des affinités pour le récepteur trop faibles pour être mesurées. Le gain d'affinité sera apporté par la multimérisation telle que décrite dans la présente invention.

Les ligands de la superfamille du TNF sont choisis dans la liste fournie par Locksley et al. (2001) et sont notamment les suivants :

| Nom du ligand | Références |
|---|---|
| EDA | Laurikkala J, *Development* (2002) May;**129**(10):2541-53 |
| CD40L | J. Singh et al., *Protein Science* (1998), **7,** 1124-1135 |
| FasL | Starling et al., *Biochemistry* (1998), **37**, 3723-3726 |
| OX40L | Weinberg AD., *Trends Immunol* (2002) Feb;**23**(2):102-9 (review) |
| AITRL | Kwon B et al., *J Biol Chem* (1999) Mar 5; **274**(10):6056-61 |
| CD30L | Opat S, Gaston JS. *Autoimmunity* (2000); **33**(1):45-60 (review) |
| VEGI (ou TL1) | Migone TS et al., *Immunity* (2002) Mar; **16**(3):479-92 |
| LIGHT | Wang J et al., *J Immunol* (2001) Nov 1;**167**(9):5099-105 |
| 4-1BBL | Kwon B, *Trends Immunol* (2002) Aug;**23**(8):378-80 |
| CD27L | Jacquot S., *Immunol Res* (2000); **21**(1):23-30 |
| LTα (TNFB) | Banner et al., *Cell* (1993), **73,** 431-445 |
| TNF | Eck et al., *J. Biol. Chem.* (1989), **264**, 17595-17605 |
| Ltβ | Browning JL, *Cell* (1993) Mar 26; **72**(6):847-56 |
| TWEAK | Chicheportiche Y et al., *J Biol Chem* (1997) Dec 19; **272**(51):32401-10 |
| APRIL | Yu G et al., *Nat Immunol* (2000) Sep; **1**(3):252-6 |
| BLYS | Liu Y et al., *Cell* (2002) Feb 8; **108**(3):383-94 |
| RANKL | Ito S et al., *J Biol Chem* (2002) Feb 22; **277**(8):6631-6 |
| TRAIL | Mongkolsapaya J et al., *Nat Struct Biol* (1999) Nov; **6**(11):1048-53 |

Selon un mode de réalisation avantageux de la présente invention, la molécule de l'invention est caractérisée en ce que -D et -D' représentent des peptides dérivés du ligand du récepteur CD40 humain ou murin (CD40L), lesdits peptides appartenant à la séquence primaire du ligand CD40L de CD40 et dont le nombre d'acides aminés est compris entre 3 et 10.

Une molécule multimérique avantageuse de l'invention est une molécule multimérique caractérisée en ce que A présente une symétrie $C_3$.

On définit une molécule de symétrie $C_3$ de la manière suivante : une molécule appartient au groupe $C_3$ si elle possède un axe d'ordre 3 (cf. définition dans "Physical Chemistry", PW Atkins, Oxford University Press, 1998, p430).

La présente invention concerne une molécule multimérique telle que définie ci-dessus, caractérisée en ce que :

- soit A est un radical branché de symétrie $C_3$ de formule générale suivante :

$$Y\left[(CH_2)_m-Z-(CH_2)_{m'}-V-\right]_3$$

dans laquelle :

* m et m' sont des nombres entiers compris de 1 à 5,
* V représente un groupe -NH- ou -CO- formant une liaison amide avec X,
* Z représente un atome d'oxygène ou un groupe $CH_2$,
* Y représente soit un atome d'azote, soit un groupe R-C- soit un groupe R-CONH-C-, dans lesquels R peut être un groupe alkyle avec 1 à 10 atomes de carbone, un groupe alkényle avec 1 à 10 atomes de carbone, un groupe alkynyle avec 1 à 10 atomes de carbone, un groupe aryle avec 5 à 12 atomes de carbone, un groupe aralkyle avec 5 à 14 atomes de carbone ou un groupe hétéroaryle avec 1 à 10 atomes de carbone, lesdits groupes pouvant être non substitués ou substitués par 1 jusqu'à 6 substituants choisis parmi les groupes -COOH, $-NH_2$, $-CONH_2$ ou alkoxy,

- soit A est un radical $C_3$ cyclique répondant à l'une des formules générales suivantes :

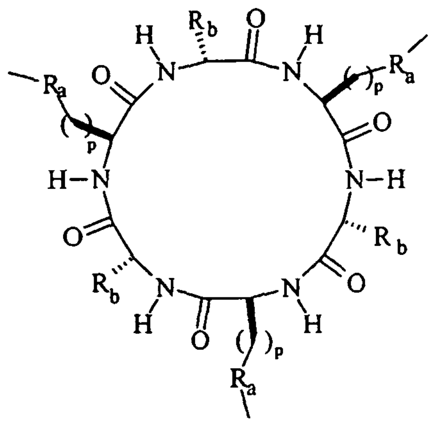

Ia

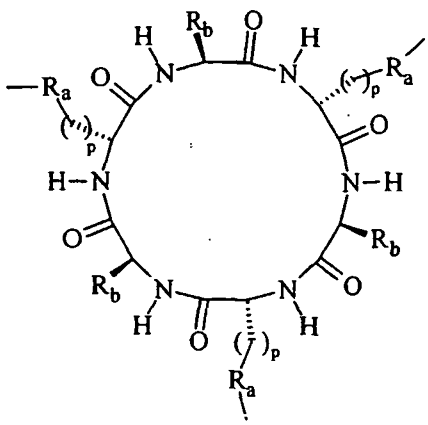

Ib

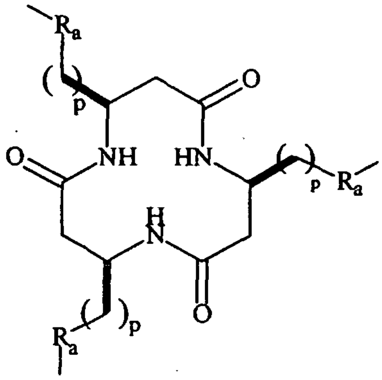

II

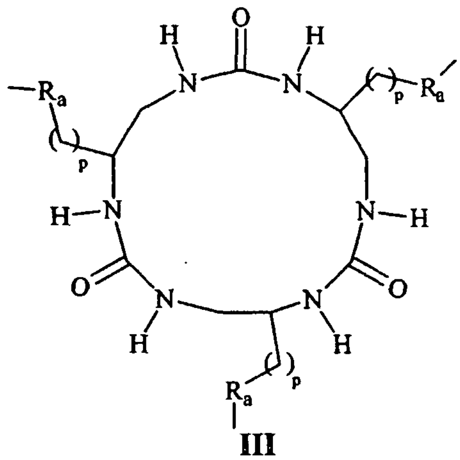

III

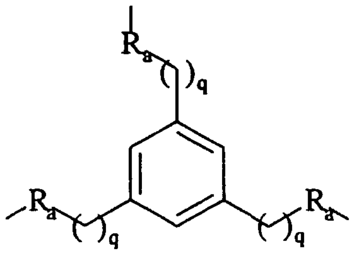

IV

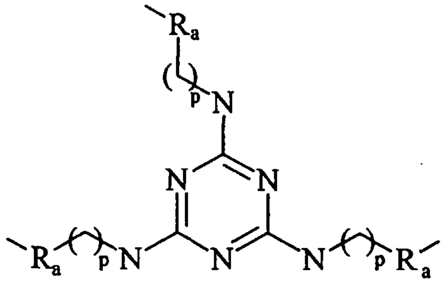

V

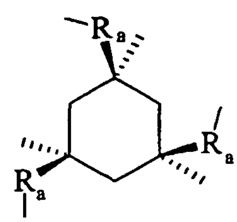

VI

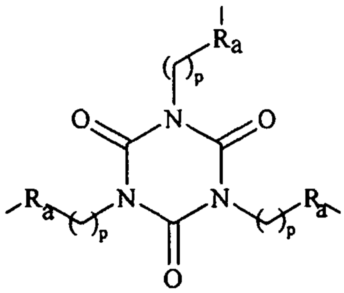

VIa

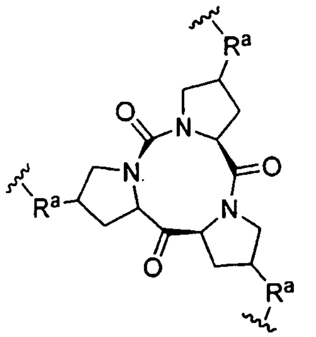

VIb

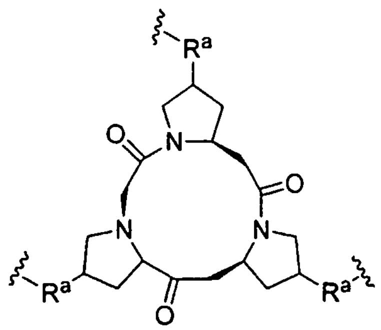

VIc

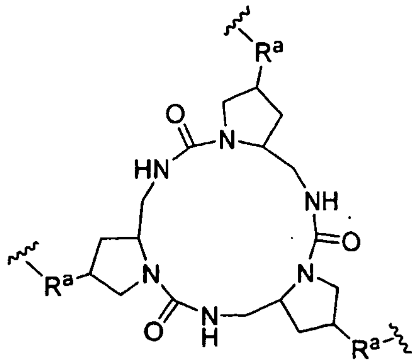

VId

dans lesquelles :

* $R_a$ représente soit un groupe -NH- soit un groupe -CO- formant une liaison amide avec X,
* $R_b$ représente la chaîne latérale d'un acide aminé protéinogénique,
* p est un nombre entier compris de 1 à 4,
* q est un nombre entier compris de 0 à 4,

- soit A est un radical branché non symétrique répondant aux formules générales suivantes :

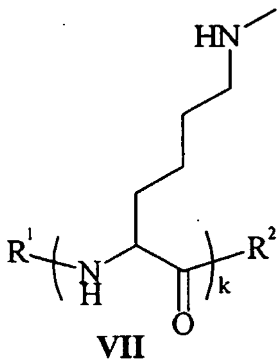

VII

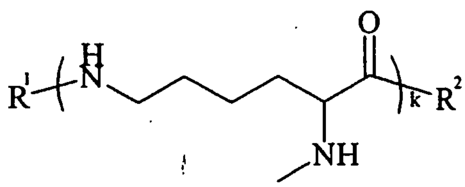

VIII

dans lesquelles :

* k représente 3, 4, 5 ou 6,
* $R^1$ représente soit un atome d'hydrogène, soit un résidu d'acide aminé choisi parmi les acides aminés protéinogéniques, soit un groupement RCO-, ROCO- ou RNHCO-, R étant tel que défini ci-dessus,
* $R^2$ représente soit un groupe $-NH_2$, soit un groupe -NHR, soit un résidu d'acide aminé choisi parmi les acides aminés protéinogéniques, R étant tel que défini ci-dessus,

- B répond à l'une des formules générales suivantes :

$$-R^3-Y-R^4$$

ou

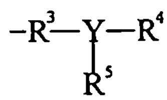

dans lesquelles :

* Y représente une chaîne alkyle $C_1$-$C_{10}$ ou un groupement alkynyle ou alkényle ou aryle ou aralkyle ou hétéroaryle,

* $R^3$ représente soit un groupe -NH- lorsque V ou $R_a$ est un groupe -CO-, soit un groupe -CO- lorsque V ou $R_a$ est un groupe -NH-,
* $R^4$ et $R^5$ représentent indépendamment l'un de l'autre un groupe -CO- ou un groupe -NH-,

- -D et -D' sont des peptides ou pseudopeptides correspondant à une séquence dérivée du ligand qui est en interaction avec le récepteur.

La présente invention concerne également une molécule telle que définie ci-dessus, caractérisée en ce que -D et -D' représentent des résidus dérivés du ligand du récepteur CD40 humain ou murin (CD40L), choisis parmi les suivants :

$Lys^{143}$-Gly-$Tyr^{145}$, $Tyr^{145}$-Gly-$Lys^{143}$,
$Lys^{143}$-Gly-Tyr-$Tyr^{146}$, $Tyr^{146}$-Tyr-Gly-$Lys^{143}$,
Lys-Pro-Arg, Lys-ψ($CH_2NH$)Pro-Arg,
$Arg^{200}$-Phe-Glu-Arg-Ile-Leu-Leu-$Arg^{207}$,
$Arg^{207}$-Leu-Leu-Ile-Arg-Glu-Phe-$Arg^{200}$,
$Arg^{200}$-Phe-Glu-Arg-$Ile^{204}$, $Ile^{204}$-Arg-Glu-Phe-$Arg^{200}$,
$Arg^{203}$-Ile-Leu-Leu-$Arg^{207}$, $Arg^{207}$-Leu-Leu-Ile-$Arg^{203}$,
$Cys^{218}$-Gly-Gln-Gln-Ser-$Ile^{223}$, $Ile^{223}$-Ser-Gln-Gln-Gly-$Cys^{218}$,
$Gly^{200}$-Ser-Glu-Arg-Ile-Leu-Leu-$Lys^{207}$,
$Lys^{207}$-Leu-Leu-Ile-Arg-Glu-Ser-$Gly^{200}$,
$Gly^{200}$-Ser-Glu-Arg-$Ile^{204}$, $Ile^{204}$-Arg-Glu-Ser-$Gly^{200}$,
$Arg^{203}$-Ile-Leu-Leu-$Lys^{207}$, $Lys^{207}$-Leu-Leu-Ile-$Arg^{203}$,
$Cys^{218}$-Glu-Gln-Gln-Ser-$Val^{223}$, $Val^{223}$-Ser-Gln-Gln-Glu-$Cys^{218}$,

ou parmi des peptides hybrides constitués d'au moins deux acides aminés consécutifs de deux des séquences définies ci-dessus, notamment les peptides de séquences $Arg^{203}$-$Ile^{204}$-$Tyr^{145}$-$Tyr^{146}$ ou $Arg^{203}$-$Ile^{204}$-$Tyr^{146}$-$Tyr^{145}$-$Gly^{144}$-$Lys^{143}$,
ou parmi des fragments des séquences susmentionnées,
les acides aminés pouvant être indifféremment de configuration L ou D.

Selon un mode de réalisation avantageux de la présente invention, la molécule telle que définie ci-dessus est caractérisée en ce que A répond à l'une des formules suivantes :

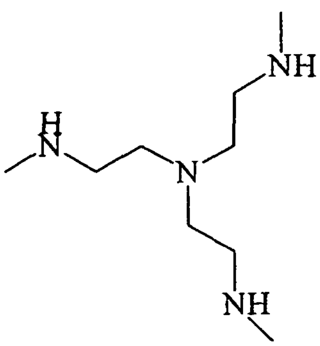

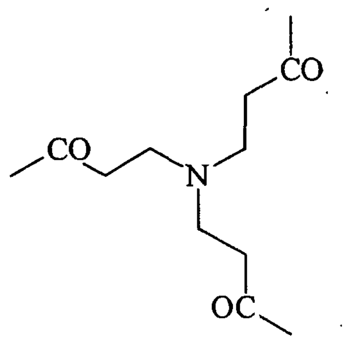

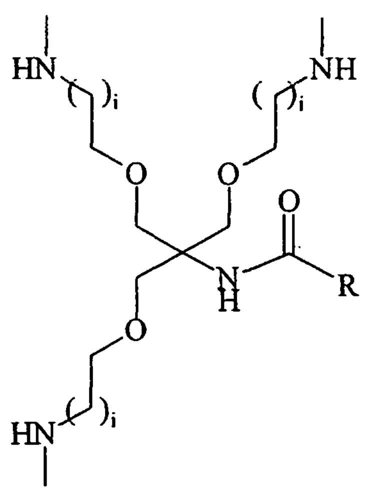

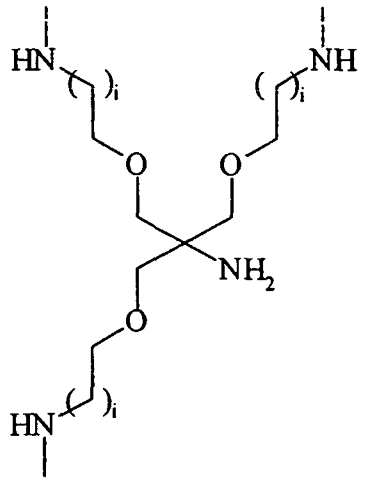

dans lesquelles i représente un nombre entier supérieur ou égal à 1.

Une molécule avantageuse de la présente invention est une molécule telle que définie ci-dessus, de formule suivante : H-Lys-Gly-Tyr-Tyr,

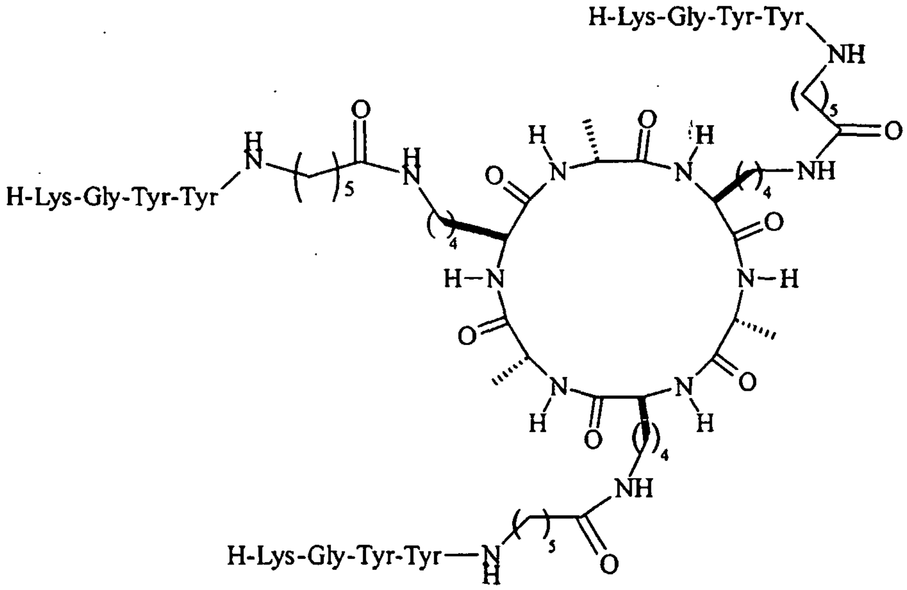

**Ic**

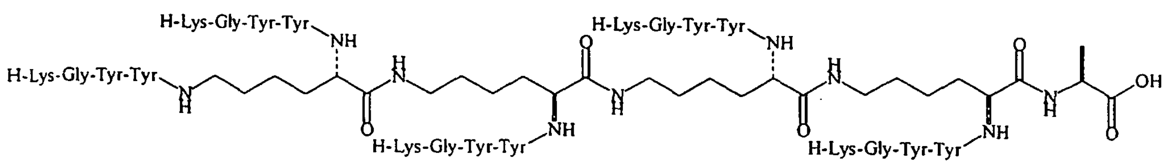

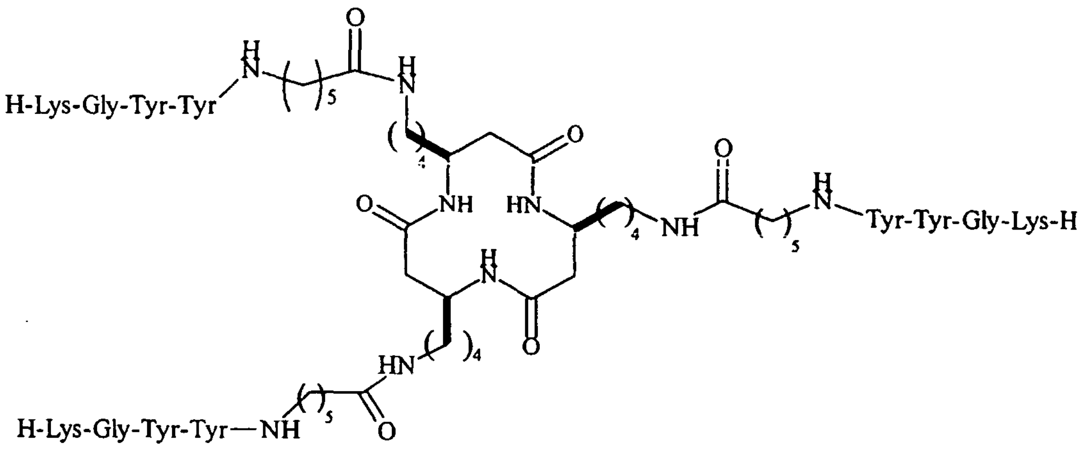

**IIa**

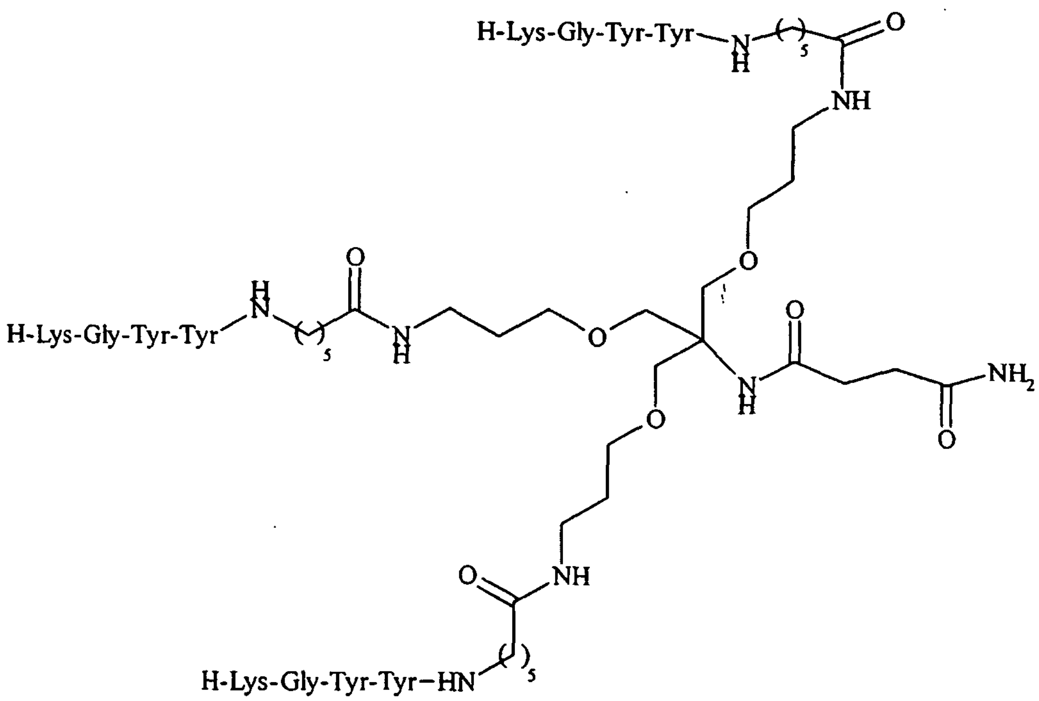

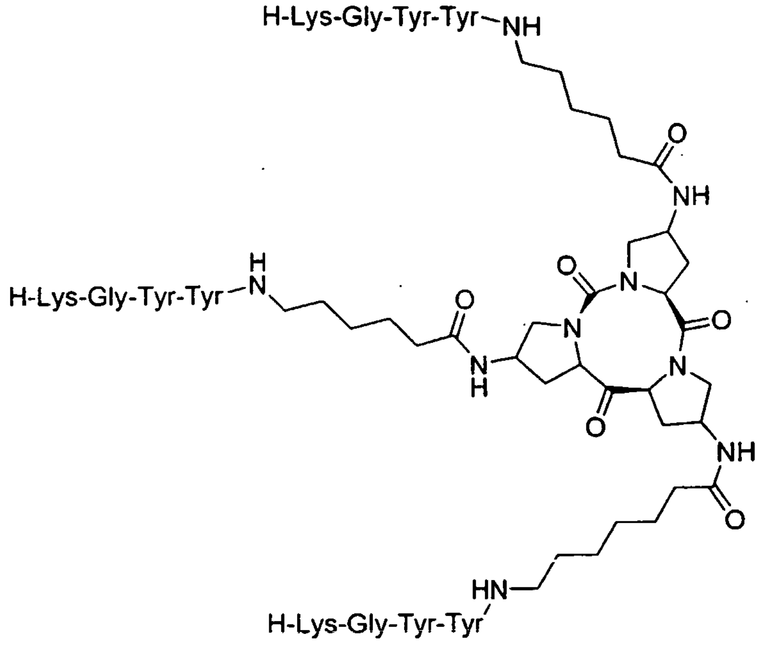

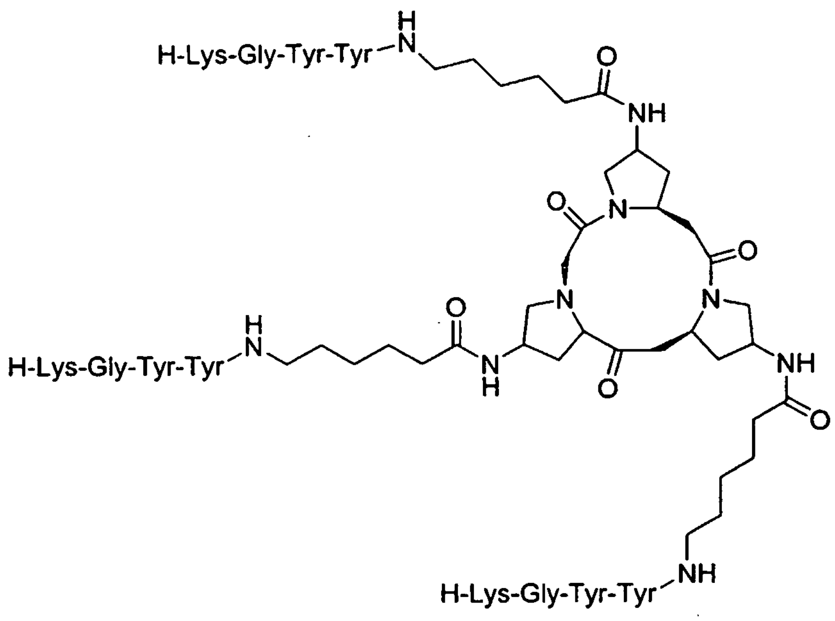

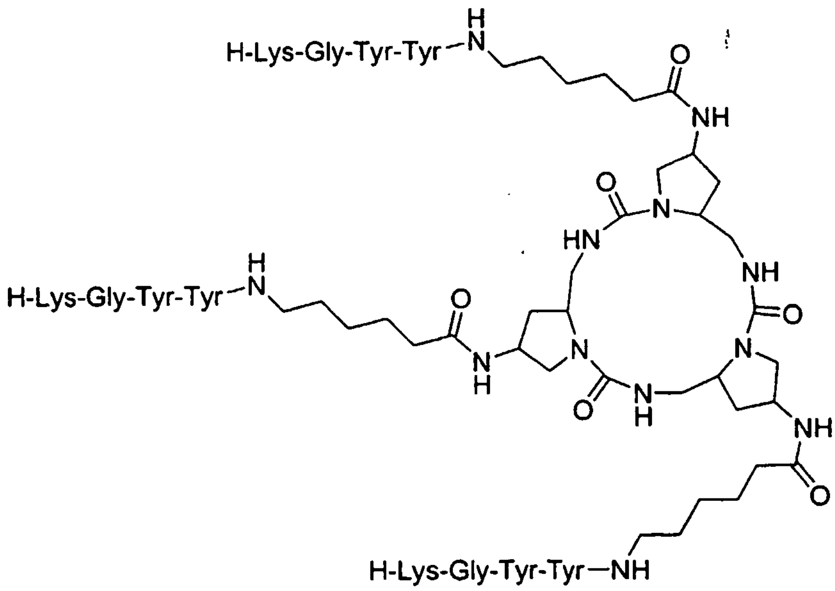

La présente invention concerne également une composition pharmaceutique caractérisée en ce qu'elle comprend, à titre de substance active une molécule multimérique telle que définie ci-dessus, en association avec un vecteur pharmaceutiquement acceptable.

La présente invention concerne également une composition vaccinale, caractérisée en ce qu'elle comprend, à titre de substance active une molécule multimérique telle que définie ci-dessus, en association avec un adjuvant pharmaceutiquement acceptable.

La présente invention concerne également l'utilisation de molécules multimériques telles que définies ci-dessus, pour la préparation d'un médicament destiné au traitement de pathologies impliquant l'inhibition ou l'activation de la réponse immunitaire.

La réponse immunitaire doit être inhibée au cours des maladies inflammatoires (rhumatismes inflammatoires), des maladies auto-immunes, des réactions d'hypersensibilités en général et des allergies en particulier, des rejets de greffes, des réactions du greffon contre l'hôte.

La réponse immunitaire doit être activée dans les vaccinations en général, dans l'immunothérapie des cancers, dans les maladies bactériennes ou virales induisant une immunosuppression (rougeole, SIDA, virus de l'herpes, cytomégalovirus...).

La présente invention concerne également l'utilisation telle que mentionnée ci-dessus, pour la préparation d'un médicament destiné au traitement de pathologies impliquant l'inhibition de la réponse immunitaire, telles que les rejets de greffes ou les maladies auto-immunes.

Les maladies impliquant l'inhibition de la réponse immunitaire comprennent les maladies auto-immunes telles que les diabètes, la sclérose en plaques, le lupus érythémateux disséminé ou l'arthrite rhumatoïde, les rejets de greffes, notamment dans le cadre d'allogreffes, de xénogreffes ou les réactions du greffon contre l'hôte, ainsi que les réactions d'hypersensibilités telles que les allergies, notamment le rhume des foins et les dermatites atopiques, ou les granulomes.

Les composés selon la présente invention, utilisés dans le cadre de l'inhibition de la réponse immunitaire, peuvent être administrés par voie intraveineuse, par les voies muqueuses (orales, aériennes, nasales, vaginales), par voie sous-cutanée, intradermique ou épicutanée.

La présente invention concerne également une composition pharmaceutique, caractérisée en ce qu'elle comprend un composé selon la présente invention, pour le traitement de pathologies impliquant l'inhibition de la réponse immunitaire, lequel composé est présent dans la composition pharmaceutique en quantités telles qu'il peut être administré à raison d'environ 100 ng à environ 5 mg par jour et par individu.

La présente invention concerne également l'utilisation telle que définie ci-dessus, pour la préparation d'un médicament destiné au traitement de pathologies impliquant l'augmentation de la réponse immunitaire, telles que les cancers ou les infections parasitaires, bactériennes ou virales.

Les cas impliquant l'activation de la réponse immunitaire comprennent les vaccinations en général, notamment les vaccins contre la grippe ou contre les maladies infantiles, l'immunothérapie des cancers, notamment dans le cadre de mélanomes ou de cancers à métastases, ou les maladies bactériennes ou virales induisant une immunosuppression, notamment dans le cadre de la rougeole, du SIDA, du virus de l'herpès ou du cytomégalovirus.

Les composés selon la présente invention, utilisés dans le cadre de l'activation de la réponse immunitaire,

peuvent être administrés par voie intraveineuse, par les voies muqueuses (orales, aériennes, nasales, vaginales), par voie sous-cutanée, intradermique ou épicutanée.

La présente invention concerne également une composition pharmaceutique, caractérisée en ce qu'elle comprend un composé selon la présente invention, pour le traitement de pathologies impliquant l'activation de la réponse immunitaire, lequel composé est présent dans la composition pharmaceutique en quantités telles qu'il peut être administré à raison d'environ 100 ng à environ 5 mg par jour et par individu.

La présente invention concerne également l'utilisation telle que définie ci-dessus, pour la préparation d'un médicament destiné au traitement de maladies non liées au système immunitaire, telles que les lymphomes, l'athérosclérose ou les thromboses.

La présente invention concerne un procédé de préparation sur support solide d'une molécule multimérique telle que définie ci-dessus, dans laquelle A est un radical $C_3$ cyclique et répond à l'une des formules Ia, Ib, II, VIb, VIc ou VId telles que définies ci-dessus, ledit procédé étant caractérisé en ce qu'il comprend les étapes suivantes :

- la formation d'un précurseur linéaire de A, lequel précurseur est constitué d'un enchaînement d'acides aminés formant une chaîne peptidique en croissance, synthétisée par des cycles successifs de couplage entre des résidus d'acides aminés N-protégés, dont trois portent un groupe $R_a$ de type amine, et la fonction amine de la chaîne peptidique en croissance, et de déprotection, le premier résidu d'acide aminé étant accroché sur un support solide,
- la cyclisation du précurseur linéaire de A protégé susmentionné,
- le clivage des susdits groupes protecteurs, pour libérer les susdites fonctions amines protégées,
- le couplage des trois fonctions amines libérées avec un bras espaceur B N-protégé,
- la déprotection du bras espaceur B et le couplage des fonctions amines libérées du bras espaceur B, avec un peptide D déjà formé ou formé in situ par l'assemblage séquentiel des résidus d'acides aminés correspondant au peptide D, et
- le clivage de la molécule du support solide, après la suppression de tous les groupements protecteurs présents sur les chaînes latérales fonctionnalisées du peptide D, afin d'obtenir la molécule multimérique selon l'invention.

Les composés comprenant un groupe A cyclique présentant une symétrie $C_3$ et répondant aux formules générales Ia, Ib, II, VIb, VIc ou VId sont obtenus par synthèse sur support solide selon le procédé décrit ci-après. Le groupe A est d'abord construit sur support solide par synthèse de son précurseur linéaire et cyclisation. Ainsi, un premier résidu d'acide aminé, dont la fonction acide est convenablement protégée (ester d'allyle par exemple), est accroché sur le support par une réaction d'amination réductrice, en utilisant une résine fonctionnalisée par un aldéhyde (résines commerciales). Le précurseur linéaire de A est ensuite assemblée par cycles successifs de couplage (de manière classique en synthèse de peptide) avec un acide aminé N-protégé (N-Fmoc-Xaa-OH par exemple, Xaa représentant un acide aminé ou un peptide en croissance quelconque) et de déprotection (pipéridine 20% dans du DMF pour le clivage d'un groupe Fmoc). Les techniques de lavage et de filtration de la résine ainsi que de déprotection du groupement Fmoc sont celles couramment utilisées en synthèse peptidique en phase solide. Les trois acides aminés portant une chaîne $R_a$ (cf. formules Ia, Ib ou II) sont fonctionnalisés sur leur chaîne latérale par une fonction amine qui est protégée par un groupement protecteur orthogonal aux autres (TEOC ou méthyltrityl par exemple). A la fin de l'assemblage, le dernier groupement N-protecteur est clivé (en présence de pipéridine 20% dans du DMF dans le cas d'un groupement Fmoc) et la protection de l'ester C-terminal est clivée. Le précurseur linéaire est alors cyclisé "tête à queue" ("head to tail") en présence d'un réactif de couplage (classique en synthèse peptidique) et d'une base tertiaire comme la DIEA ou la collidine par exemple. La réaction de cyclisation peut être suivie par un test colorimétrique tel que le test de Kaiser (Kaiser et al., 1970). A la fin de la cyclisation, les groupements protecteurs des acides aminés possédant une fonction amine protégée sont clivés et le bras espaceur, convenablement protégé (Fmoc-Ahx (acide 6-amino hexanoïque)-COOH par exemple), est couplé en présence d'un agent de couplage sur les trois fonctions amines libres. A l'issue de ce couplage, le groupement protecteur du bras espaceur est clivé et le peptide D est assemblé par des méthodes classiques de synthèse peptidique. A la fin de la synthèse et une fois le dernier groupe protecteur enlevé, la molécule est clivée de la résine, par exemple par traitement à l'acide trifluoroacétique, lyophilisée après précipitation à l'éther et purifiée par HPLC préparative en phase inverse sur une colonne C18 par exemple.

La présente invention concerne également un procédé de préparation en solution d'une molécule multimérique telle que définie ci-dessus, dans laquelle A est un radical $C_3$ cyclique et répond à l'une des formules Ia, Ib, II, VIb, VIc ou VId telles que définies ci-dessus, ledit procédé étant caractérisé en ce qu'il comprend les étapes suivantes :

- la formation d'un précurseur linéaire de A, lequel précurseur est constitué d'un enchaînement d'acides aminés formant une chaîne peptidique en croissance, synthétisée par des cycles successifs de couplage entre des résidus d'acides aminés N-protégés, dont trois portent un groupe $R_a$ de type amine, et la fonction amine de la chaîne peptidique en croissance, et de déprotection,
- la cyclisation du précurseur linéaire de A protégé susmentionné,

- le clivage des susdits groupes protecteurs, pour libérer les susdites fonctions amines protégées,
- le couplage des trois fonctions amines libérées avec un peptide -D-B correspondant à un bras espaceur B lié à un peptide D protégé,
- la déprotection des groupements protecteurs présents sur le peptide D, afin d'obtenir la molécule multimérique selon l'invention.

La présente invention concerne un procédé de préparation d'une molécule multimérique telle que définie ci-dessus, dans laquelle A est un radical $C_3$ branché et répond à l'une des formules IV, V, VI ou VIa telles que définies ci-dessus, ledit procédé étant caractérisé en ce qu'il comprend les étapes suivantes :

- le couplage des trois fonctions amines du radical A de formule IV, V, VI ou VIa avec un bras espaceur B protégé,
- la déprotection du bras espaceur B,
- l'assemblage du bras espaceur B déprotégé avec des acides aminés protégés entrant dans la constitution d'un peptide D, par des cycles successifs de couplage, de purification et de déprotection des acides aminés susmentionnés,
- la déprotection du dernier acide aminé entrant dans la constitution du peptide D, afin d'obtenir la molécule multimérique selon l'invention.

Les composés de l'invention comprenant un groupe A qui est un radical branché de symétrie $C_3$, et correspondant notamment aux formules IV, V, VI ou VIa, peuvent être synthétisés en phase solide ou en solution selon la procédure détaillée ci-après.

Lorsque le radical A est fonctionnalisé par des fonctions amines, le bras espaceur convenablement protégé (Boc-Ahx-OH par exemple) est couplé en présence d'un réactif de couplage selon les procédés de synthèse peptidique sur les trois fonctions amines de A. A l'issue de ce couplage dont la réaction peut être suivie par chromatographie sur couche mince, le produit est isolé et purifié sur colonne de silice selon les techniques classiques de synthèse organique. Le groupement Boc est ensuite clivé par l'acide trifluoroacétique, et le peptide D est assemblé en solution par étapes successives de couplage, purification et déprotection du groupement Boc. A la fin de la synthèse, les groupements protecteurs sur les chaînes latérales sont clivés par hydrogénation catalytique ou bien par traitement à l'acide fluorhydrique HF. La molécule trimérique est ensuite purifiée par HPLC préparative en phase inverse sur une colonne C18 par exemple.

Lorsque le radical A est fonctionnalisé par des fonctions acides carboxyliques, le bras espaceur convenablement protégé (hexaméthylène diamine mono protégée par un groupement Boc par exemple) est couplé en présence d'un réactif de couplage selon les procédés de synthèse peptidique sur les trois fonctions acide carboxylique de A. A l'issue de ce couplage dont la réaction peut être suivie par chromatographie sur couche mince, le produit est isolé et purifié sur colonne de silice selon les techniques classiques de synthèse organique. Le groupement Boc est ensuite clivé par l'acide trifluoroacétique, et le peptide D est assemblé en solution par étapes successives de couplage, purification et déprotection du groupement Boc. A la fin de la synthèse, les groupements protecteurs sur les chaînes latérales sont clivés par hydrogénation catalytique ou bien par traitement à l'acide fluorhydrique HF. La molécule trimérique est ensuite purifiée par HPLC préparative en phase inverse sur une colonne C18 par exemple.

La présente invention concerne un procédé de préparation sur support solide d'une molécule multimérique telle que définie ci-dessus, dans laquelle A est un radical branché non symétrique répondant à l'une des formules VII ou VIII telles que définies ci-dessus, ledit procédé étant caractérisé en ce qu'il comprend les étapes suivantes :

- le greffage d'une lysine sur un support solide, chacune des deux fonctions amines de la lysine, respectivement en position $\alpha$ et $\varepsilon$, étant protégée respectivement par des groupes protecteurs différents et orthogonaux,
- l'allongement de la chaîne peptidique formée à partir de la lysine, à la longueur désirée, par couplages et déprotections successifs

    * soit uniquement des fonctions amines en position $\alpha$ afin d'obtenir le radical A de formule VII, avec des fonctions amines protégées en position $\varepsilon$,
    * soit uniquement des fonctions amines en position $\varepsilon$ afin d'obtenir le radical A de formule VIII, avec des fonctions amines protégées en position $\alpha$,

- le couplage des fonctions amines déprotégées en position $\varepsilon$ dans le radical A de formule VII ou en position $\alpha$ dans le radical A de formule VIII, avec un bras B protégé,
- l'assemblage du bras espaceur B déprotégé avec un peptide D déjà formé ou formé in situ par l'assemblage séquentiel des résidus d'acides aminés correspondant au peptide D, et
- le clivage de la molécule ainsi obtenue du support solide, après la suppression de tous les groupements protecteurs

présents sur les chaînes latérales fonctionnalisées du peptide D.

Les composés de l'invention comprenant un groupe A qui est un radical branché non symétrique, peuvent être synthétisés en phase solide selon la procédure détaillée ci-après.

On utilise une résine Fmoc-Xaa-Wang (Xaa peut être n'importe quel acide aminé ou peptide en croissance) ou une résine Fmoc-Rink-amide commerciale. Après clivage du groupement Fmoc avec de la pipéridine 25% dans du DMF (diméthylformamide)(2 × 15 min), l'acide aminé, portant la chaîne amine fonctionnalisée, convenablement protégée de manière orthogonale notamment par un groupement Fmoc ou un groupement Mtt (méthyltrityle) dans le cas d'une fonction amine (Fmoc-Lys(Mtt)-OH par exemple), est activé avec un agent de couplage en présence d'une base tertiaire et couplé sut la résine. Le groupement Mtt ou Fmoc est clivé sélectivement par traitement avec une solution (6 ml) de 85% de dichlorométhane (DCM), de 10% de triisopropylsilane (TIS), de 5% d'acide trifluoroacétique (TFA) (3 × 3 min) (cas du Mtt), ou de pipéridine 25% dans du DMF (cas du Fmoc). La chaîne est allongée à la longueur désirée (k étant un nombre entier compris entre 3 et 6) par couplages et déprotections successives en utilisant le même acide aminé (Fmoc-Lys(Mtt)-OH par exemple). Après déprotection du dernier groupement Fmoc ou Mtt, le reste des groupements protecteurs (Mtt ou Fmoc) est clivé et le bras espaceur convenablement protégé (Fmoc-Ahx-COOH par exemple) est couplé en présence d'un agent de couplage sur les fonctions amines libres. A l'issue de ce couplage, le groupement protecteur du bras espaceur est clivé et le peptide D est assemblé par des méthodes classiques de synthèse peptidique. A la fin de la synthèse et une fois le dernier groupe protecteur enlevé, le peptide est clivé de la résine (traitement à l'acide trifluoroacétique par exemple), lyophilisé après précipitation à l'éther et purifié par HPLC préparative en phase inverse sur une colonne C18 par exemple.

## DESCRIPTION DES FIGURES

La Figure 1A représente le modèle moléculaire du complexe trivalent CD40-CD40L.

La Figure 1B représente des résidus de CD40L situés à l'interface et essentiels à l'interaction avec CD40. Les résidus portant une étoile appartiennent à la deuxième sous-unité CD40L formant l'interface.

La Figure 2 représente la structure des ligands trimériques de l'invention.

Les Figure 3A, 3B, 3C et 3D représentent l'expression de CD95 à la surface des cellules du lymphome de Burkitt BL41 lors de l'interaction CD40-CD40L, mesurée par cytométrie de flux. Les surfaces grisées représentent le contrôle isotypique et la courbe gris clair représente la fluorescence proportionnelle à la quantité d'anti-CD95 fixé sur les cellules.

La Figure 3A représente l'expression de CD95 lorsque les cellules BL41 susmentionnées ($5.10^5$/ml) sont incubées avec des fibroblastes 3T6 non transfectés.

La Figure 3B représente l'expression de CD95 lorsque les cellules BL41 susmentionnées ($5.10^5$/ml) sont incubées avec des fibroblastes 3T6 transfectés par CD40L.

La Figure 3C représente l'expression de CD95 lorsque les cellules BL41 susmentionnées ($5.10^5$/ml) sont incubées avec des fibroblastes 3T6 non transfectés, en présence d'anticorps anti-CD40L.

La Figure 3D représente l'expression de CD95 lorsque les cellules BL41 susmentionnées ($5.10^5$/ml) sont incubées avec des fibroblastes 3T6 transfectés par CD40L, en présence d'anticorps anti-CD40L.

La Figure 4A représente l'inhibition de l'incorporation de thymidine tritiée des cellules du lymphome de Burkitt BL41 ($4.10^5$ cellules/ml) après 24h de culture en présence de différentes concentrations de ligands (L4, L7, L7-1, L7-2 ou L7-3) ou de CD40L soluble (CD40Ls). Les colonnes noires correspondent à une concentration égale à 50 μM ; les colonnes avec des hachures horizontales correspondent à une concentration égale à 5 μM ; les colonnes blanches correspondent à une concentration égale à 0,5 μM ; les colonnes grises correspondent à une concentration égale à 100 nM et les colonnes quadrillées correspondent à une concentration égale à 50 nM.

La thymidine tritiée a été ajoutée à une concentration de 1μci/puits ($0,8.10^5$ cellules/puits) pendant les 8 dernières heures de la culture et l'incorporation de thymidine tritiée mesurée à l'aide d'un compteur β en scintillation gazeuse.

La Figure 4B représente le pourcentage d'apoptose spécifique des cellules du lymphome de Burkitt BL41 ($4.10^5$ cellules/ml) après 24h de culture en présence de différentes concentrations des ligands L4, L4bis, L8, L7, L9 et L11. Les colonnes noires correspondent à une concentration égale à 50 μM ; les colonnes blanches correspondent à une concentration égale à 25 μM ; les colonnes avec des hachures horizontales correspondent à une concentration égale à 12,5 μM et les colonnes avec des hachures obliques correspondent à une concentration égale à 6 μM.

L'apoptose est mesurée en cytométrie de flux après marquage avec de l'annexine V FITC et de l'iodure de propidium. Les cellules marquées avec l'annexine V seule ou avec l'annexine V et l'iodure de propidium sont considérées comme apoptotiques. Le pourcentage d'apoptose spécifique est calculé par la formule suivante :

$$[(\% \text{ apoptose avec le ligand} - \% \text{ apoptose sans ligand})/(100 - \% \text{ apoptose sans ligand})] \times 100$$

La Figure 5A représente le pourcentage de souris MRL-$^{lpr}$ (Koopman et al., 1998) vivantes à différents âges après injection intraveineuse de 100 µL/souris de PBS contenant ou non 100µg de ligand L4. Les injections ont eu lieu à l'âge de 7, 9 et 11 semaines. La courbe en trait plein correspond à une injection de PBS sans le ligand L4 et la courbe en trait pointillé correspond à une injection de PBS avec le ligand L4.

La Figure 5B représente le pourcentage de souris MRL-$^{lpr}$ présentant des anticorps anti-ADN (évalué par un test ELISA) dans leur sérum à différents âges après le même traitement que dans la Figure 5A. Les colonnes blanches correspondent à une injection de PBS sans le ligand L4 et les colonnes noires correspondent à une injection de PBS avec le ligand L4.

La Figure 5C représente le pourcentage de souris MRL-$^{lpr}$ présentant une protéinurie positive dans leurs urines à différents âges après le même traitement que dans la figure 5A. Les colonnes blanches correspondent à une injection de PBS sans le ligand L4 et les colonnes noires correspondent à une injection de PBS avec le ligand L4.
* La totalité des souris traitées par le ligand L4 sont mortes.

**PRÉPARATION DES COMPOSÉS L1, L2, L3 ET L4 :**

**A) PRÉPARATION DU COMPOSÉ L1**

Le composé L1 répond à la formule suivante :

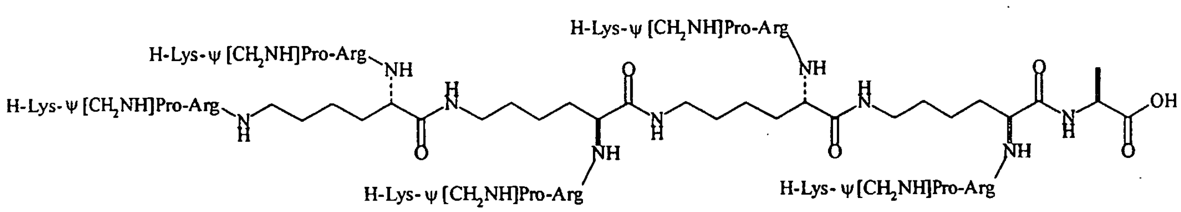

Le composé L1 est synthétisé en phase solide sur une résine Wang-Ala-Fmoc commerciale sur une échelle de 200 µmol. Après clivage du groupement Fmoc avec 25% de pipéridine dans la DMF (diméthyleformamide) (2 × 15 min), l'acide aminé Fmoc-Lys(Mtt)-OH (3 équivalents) activé avec Bop (benzotriazol-1-yloxy-tris(diméthylamino)phosphonium hexafluorophosphate)(3 équivalents), HOBt (1-hydroxybenzotriazole)(3 équivalents) et DIEA (diisopropyléthylamine)(9 équivalents) dans la DMF est couplé à la résine (2 × 15 min). Les techniques de lavage et de filtration de la résine ainsi que de déprotection du groupement Fmoc sont celles couramment utilisées en synthèse peptidique en phase solide. Le groupement Mtt (4-méthyltrityle) est clivé avec une solution (6 ml) de 85% DCM (dichlorométhane), 10% TIS (triisopropylsilane), 5% TFA (acide trifluoroacétique) (3 × 3 min). Le deuxième et le troisième Fmoc-Lys(Mtt)-OH sont couplés avec la même stratégie et les mêmes quantités de réactifs ci-dessus. La quatrième lysine est couplée sous forme de Fmoc-Lys(Fmoc)-OH. Après déprotection du groupement Fmoc avec 25% pipéridine dans la DMF (2 × 15 min), les acides aminés Fmoc-Arg(Pbf)-OH et Fmoc-Pro-OH (15 équivalents) activés avec Bop (15 équivalents), HOBt (15 équivalents) et DIEA (45 équivalents) dans la DMF sont couplés pendant 15 minutes (chaque couplage est répété 2 fois). La liaison amide réduite entre la lysine et la proline est formée sur la résine en utilisant la réaction d'amination réductrice de l'aldéhyde Boc-Lys(Boc)-CHO (2,5 équivalents) en présence de $NaBH_3CN$ (2,5 équivalents) dans de la DMF contenant 1% d'acide acétique (2 × 1h). Le produit est clivé de la résine avec une solution (5 ml) de 80% TFA, 10% DCM, 10% TIS pendant 2 heures. Après précipitation dans de l'éther froid, le produit brut est lyophilisé dans un mélange $H_2O$/acétonitrile/acide acétique (80/15/5). Le produit est contrôlé par spectrométrie de masse (MALDI-MS (Matrix assisted laser desorption ionisation mass spectrometry)) et HPLC analytique et purifiée par HPLC préparatif sur colonne $C_{18}$ en utilisant un gradient d'acétonitrile dans l'eau.

**B) PRÉPARATION DU COMPOSÉ L2**

Le composé L2 répond à la formule suivante :

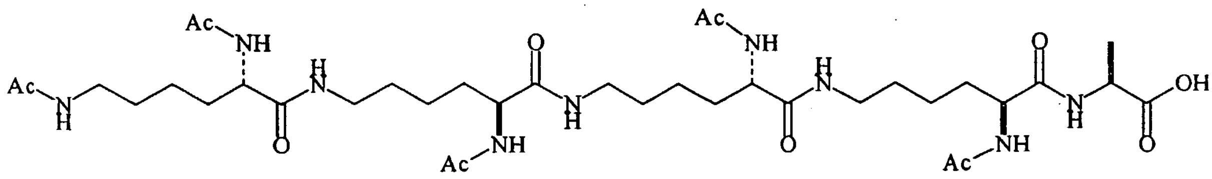

Le composé L2 est synthétisé comme L1 jusqu'à la quatrième lysine. Après déprotection du groupement Fmoc avec 25% de pipéridine dans la DMF (2 × 15 min), les groupes aminés libres sont acétylés avec une solution d'anhydride acétique (1 ml) dans du DCM (2 ml) en présence de DIEA (1 ml) pendant 15 minutes. Le produit est clivé de la résine avec une solution (5 ml) de 80% TFA, 10% DCM, 10% TIS pendant 2 heures. Après précipitation dans de l'éther froid, le produit brut est lyophilisé dans un mélange $H_2O$/acétonitrile/acide acétique (80/15/5). Le produit est contrôlé par spectrométrie de masse (MALDI-MS) et HPLC analytique et purifiée par HPLC préparatif sur colonne $C_{18}$ en utilisant un gradient d'acétonitrile dans l'eau.

**C) PRÉPARATION DU COMPOSÉ L3**

Le composé L3 répond à la formule suivante :

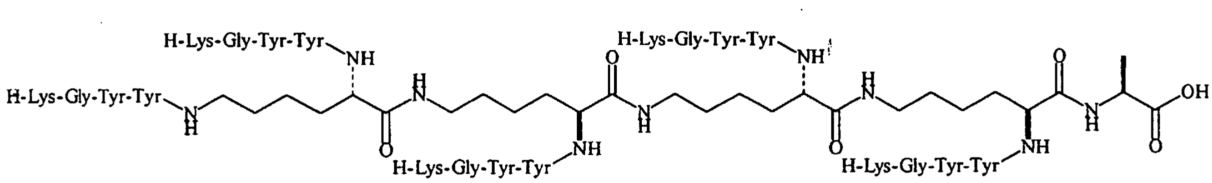

Le composé L3 est synthétisé comme L1 jusqu'à la quatrième lysine. Après déprotection du groupement Fmoc avec 25 % pipéridine dans la DMF (2 × 15 min), les acides aminés Fmoc-Tyr(tBu)-OH, Fmoc-Tyr(tBu)-OH, Fmoc-Gly-OH et Fmoc-Lys(Boc)-OH (15 équivalents) activés avec Bop (15 équivalents), HOBt (15 équivalents) et DIEA (45 équivalents) dans la DMF sont couplés pendant 15 minutes (chaque couplage est répété 2 fois). Le produit est clivé de la résine avec une solution (5 ml) de 80% TFA, 10% DCM, 10% TIS pendant 2 heures. Après précipitation dans de l'éther froid, le produit brut est lyophilisé dans un mélange $H_2O$/acétonitrile/acide acétique (80/15/5). Le produit est contrôlé par spectrométrie de masse (MALDI-MS) et HPLC analytique et purifiée par HPLC préparatif sur colonne $C_{18}$ en utilisant un gradient d'acétonitrile dans l'eau.

**D) PRÉPARATION DES COMPOSÉS L4 ET L4bis**

Le composé L4 répond à la formule suivante :

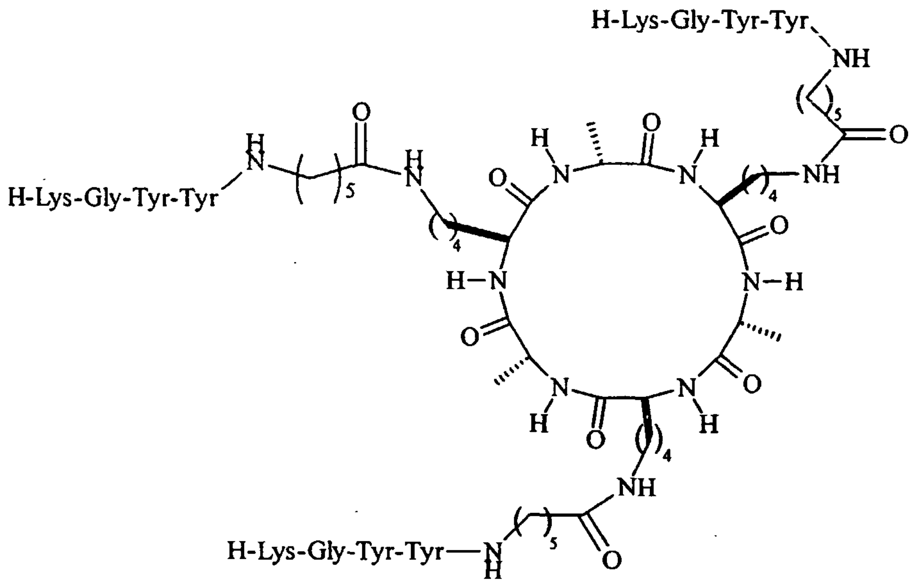

Le composé L4bis répond à la formule suivante :

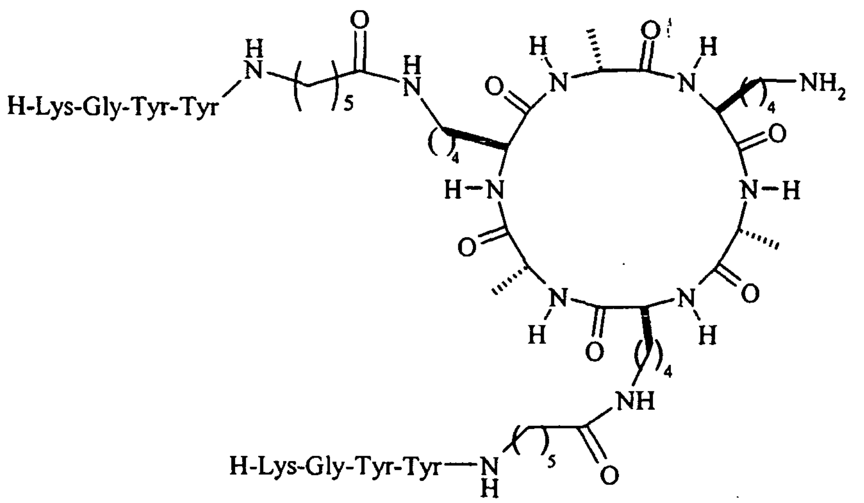

**1) Préparation des synthons**

**a) Boc-(D)Ala-OAll**

L'acide aminé Boc-D-Ala-OH (1,89 g, 10 mmol) est solubilisé dans 70 ml d'ACN (acétonitrile) et la solution refroidie à 0°C. Après avoir ajouté 1,5 ml (1,2 équivalents) de DBU (1,8-diazabicyclo[5,4,0]undecen-7-ene), une solution de bromure d'allyle (0,72 ml, 1 équivalent), dans 10 ml d'acétonitrile, est ajoutée goutte à goutte pendant environ 15 minutes. La réaction, suivie par chromatographie en couche mince (CCM) se déroule à température ambiante pendant 21 heures. Après évaporation de l'acétonitrile, le produit brut est dissous dans l'acétate d'éthyle et la solution organique est lavée avec $NaHCO_3$ 5%, $H_2O$, $KHSO_4$ 1N et $H_2O$. Après séchage sur $Na_2SO_4$ et évaporation de la phase organique, le produit est récupéré sous forme d'huile avec un rendement de 73%. Le produit est caractérisé par spectroscopie RMN et FT-IR, et utilisé pour la réaction suivante sans purifications ultérieures.

**b) HCl×H-(D)Ala-OAll**

L'acide aminé Boc-D-Ala-OAll (1,67 g ; 7,3 mmol) est solubilisé dans une solution de 5 ml de HCl (4 M) dans du dioxane sous atmosphère d'argon pendant 30 minutes. La réaction est suivie par CCM. Après évaporation de la solution acide, le produit est obtenu solide sous vide avec un rendement de 90%. Le produit est caractérisé par spectroscopie RMN et FT-IR, et utilisé pour la réaction suivante sans purifications ultérieures.

**c) Fmoc-Lys(Mtt)-OAll**

L'acide aminé Fmoc-Lys(Mtt)-OH (625 mg, 1 mmol) est solubilisé dans 5 ml de dichlorométhane (DCM) en présence de HOBt (1 équivalent) et EDC×HCl (EDC : 1-éthyl-3-(3-diméthylaminopropyl)carbodiimide méthiodide)(1,1 équivalents). Après 5 minutes, l'allyle alcool (1 équivalent) et la DMAP (4-diméthyl aminopyridine)(0,1 équivalents) sont ajoutés. Après 27 heures, 0,5 équivalents de HOBt, EDC×HCl, allyle alcool et 0,1 équivalents de DMAP sont ajoutés. La réaction se déroule au total pendant 44 heures. Après évaporation du dichlorométhane, le produit brut est dissous dans l'acétate d'éthyle et la solution organique est lavée avec $NaHCO_3$ 5%, $H_2O$, $KHSO_4$ 1N et $H_2O$. Après séchage sur $Na_2SO_4$ et évaporation de la phase organique, le produit est récupéré su forme d'huile avec un rendement de 94%. Le produit est caractérisé par spectroscopie RMN et FT-IR, et utilisé pour la réaction suivante sans purifications ultérieures.

**d) H-Lys(Mtt)-OAll**

Fmoc-Lys(Mtt)-OAll (240 mg, 0,36 mmol) est solubilisé dans 20% de DEA (diéthyle amine) dans du DCM (10 ml). La réaction, suivie par CCM, se déroule pendant 5 heures après avoir ajouté encore 3 ml de DEA. Après évaporation de la solution, le produit brut est solubilisé dans le diéthyle éther et extrait avec une solution de $KHSO_4$ 1 N. La solution acide est basifiée avec $NaHCO_3$ solide et le produit ré-extrait avec de l'acétate d'éthyle. La phase organique est lavée avec $H_2O$, séchée sur $N_2SO_4$ et évaporée. Le produit est récupéré sous forme d'huile avec un rendement quantitatif, et utilisé pour la réaction suivante.

**2) Amination réductrice sur support solide**

La réaction d'amination réductrice est effectuée en phase solide sur une résine 2-(3,5-dimethoxy-4-formylphenoxy)éthyle polystyrène commerciale, sur une échelle de 62 μmol. HCl×H-D-Ala-OAll (10 équivalents) et $NaBH_3CN$ (10 équivalents) sont solubilisés dans la DMF et ajoutés à la résine. La réaction, suivie par spectrophotométrie FT-IR, se déroule pendant 24 heures sous agitation.

**3) Préparation du dipeptide linéaire, précurseur de L4 et L4bis**

L'acide aminé Fmoc-Lys(Mtt)-OH (5 équivalents) est solubilisé dans 1 ml de dichlorométhane en présence du collidine (14 équivalents) et activé avec du triphosgène (1,65 équivalents) pendant 1 minute. La solution est ensuite ajoutée à la résine (62 μmol) et la réaction de couplage se déroule pendant 30 minutes. La résine est lavée de manière extensive au DCM, à la DMF et séchée à l'éther.

**4) Préparation du tripeptide linéaire précurseur de L4 et L4bis**

Le conjugué dipeptide-résine (62 μmol) est traité avec $Pd(Ph_3)_4$ (2 équivalents), solubilisé dans 2 ml d'une solution de DCM:AcOH (acide acétique):NMM (N-méthyle morpholine) en rapport 1850:100:50 pendant 6 h sous argon. La réaction est suivie par spectrophotométrie FT-IR. Ensuite, à l'acide aminé H-Lys(Mtt)-OAll (5,8 équivalents), solubilisé dans 1,5 ml de DMF et 600 μl de DCM, sont ajoutés HATU [O-(7-azabenzotriazol-1-yl)-1,1,3,3-tetramethyluranium hexafluorophosphate] (4 équivalents) HOAt (7-azabenzotriazol) (4 équivalents), $CuCl_2$ (0,5 équivalents) et collidine (9 équivalents). La solution est ajoutée à la résine (62 μmol) et la réaction de couplage se déroule pendant 2 heures. La résine est lavée de manière extensive à la DMF, au DCM, au méthanol, séchée à l'éther et contrôlée par spectrophotométrie FT-IR.

**5) Préparation de l'hexapeptide cyclique L4 et L4bis**

Après déprotection du groupement Fmoc avec 25 % de pipéridine dans la DMF (2 × 15 min), les acides aminés Fmoc-D-Ala-OH, Fmoc-Lys(Mtt)-OH et Fmoc-D-Ala-OH (5 équivalents) activés avec Bop (5 équivalents), HOBt (5 équivalents) et DIEA (15 équivalents) dans la DMF sont couplés pendant 1 heure (chaque couplage est répété 2 fois). Le

groupement allyle est clivé avec $Pd(Ph_3)_4$ (2 équivalents), solubilisé dans 2 ml d'une solution de DCM:AcOH:NMM en rapport 1850:100:50 pendant 6 h sous argon. Le groupement Fmoc est clivé avec une solution 25% de pipéridine dans la DMF (2 × 15 min). Le dérivé hexapeptide-résine linéaire avec les extrémités N- et C-terminales libres est cyclisé en présence de HOAt (4 équivalents), DIC (diisopropylcarbodiimide) (4,4 équivalents) dans une solution de DMF/DCM 5: 2 (2,1 ml) pendant 3 heures. Le groupement Mtt est clivé avec une solution (2 ml) de 85% DCM, 10% TIS, 5% TFA (3 × 2 min). Les acides aminés Fmoc-Ahx-OH (H-Ahx-OH : acide 6-amino hexanoïque), Fmoc-Tyr(tBu)-OH, Fmoc-Tyr (tBu)-OH, Fmoc-Gly-OH et Fmoc-Lys(Boc)-OH (15 équivalents) activés avec Bop (15 équivalents), HOBt (15 équivalents) et DIEA (45 équivalents) dans la DMF sont couplés pendant 1 heure (chaque couplage est répété 2 fois). Le peptide cyclique est clivé de la résine avec une solution (3 ml) de 90% TFA, 5% $H_2O$, 5% TIS pendant 2 heures. Le clivage est répété une deuxième fois dans les mêmes conditions pendant 3 heures. Après précipitation dans de l'éther froid, on obtient à la fois le produit L4 et le sous-produit L4bis qui sont ensuite lyophilisés dans un mélange $H_2O$/acétonitrile/ acide acétique (80/15/5). Les produits sont contrôlés par spectrométrie de masse (MALDI-MS) et HPLC analytique, purifiés et séparés par HPLC préparative sur colonne $C_{18}$ en utilisant un gradient d'acétonitrile dans l'eau.

**E) PRÉPARATION DU COMPOSÉ L7**

Le ligand L7 répond à la formule suivante :

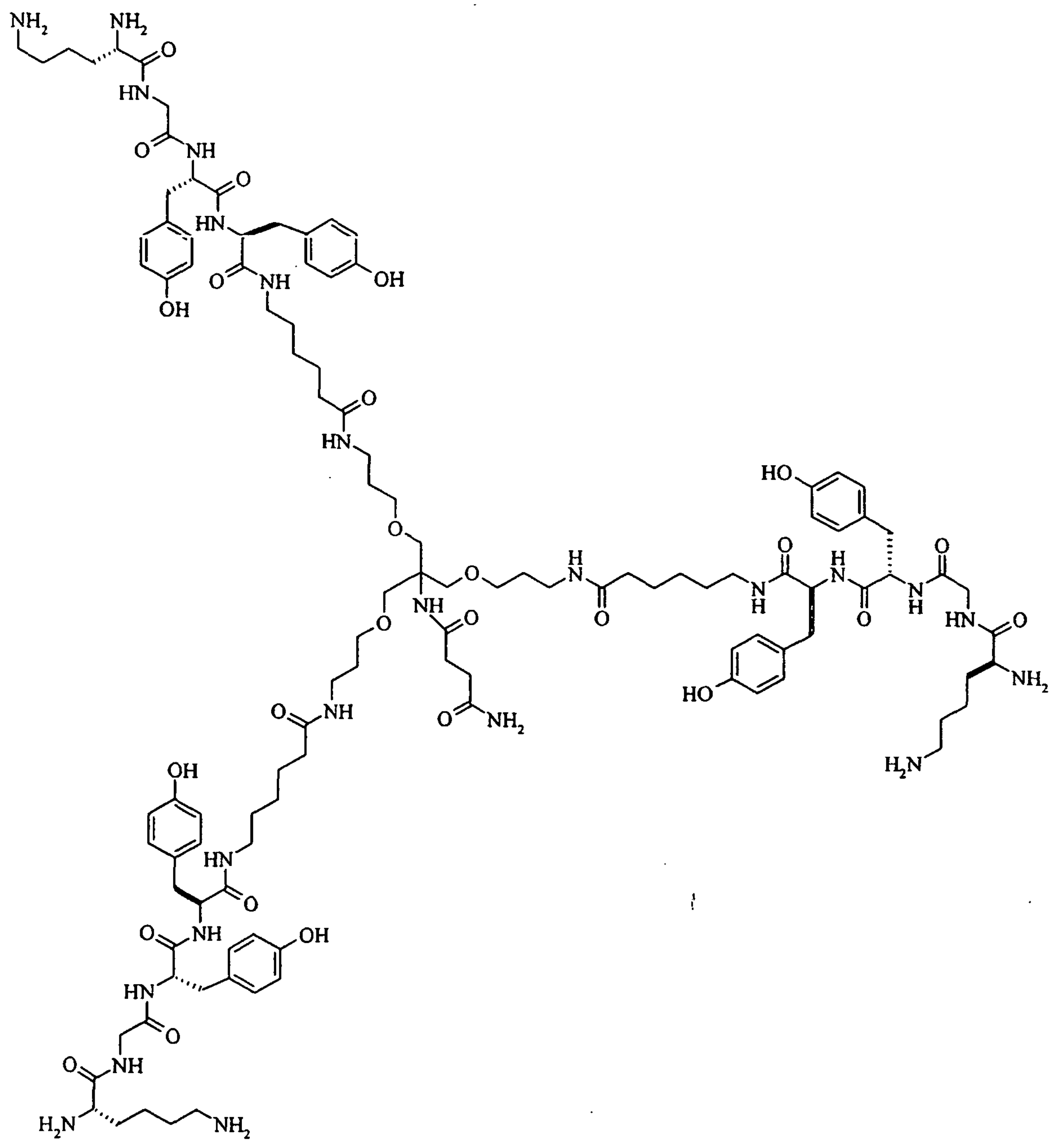

**Schéma réactionnel : Préparation du monomère 6 (produit intermédiaire)**

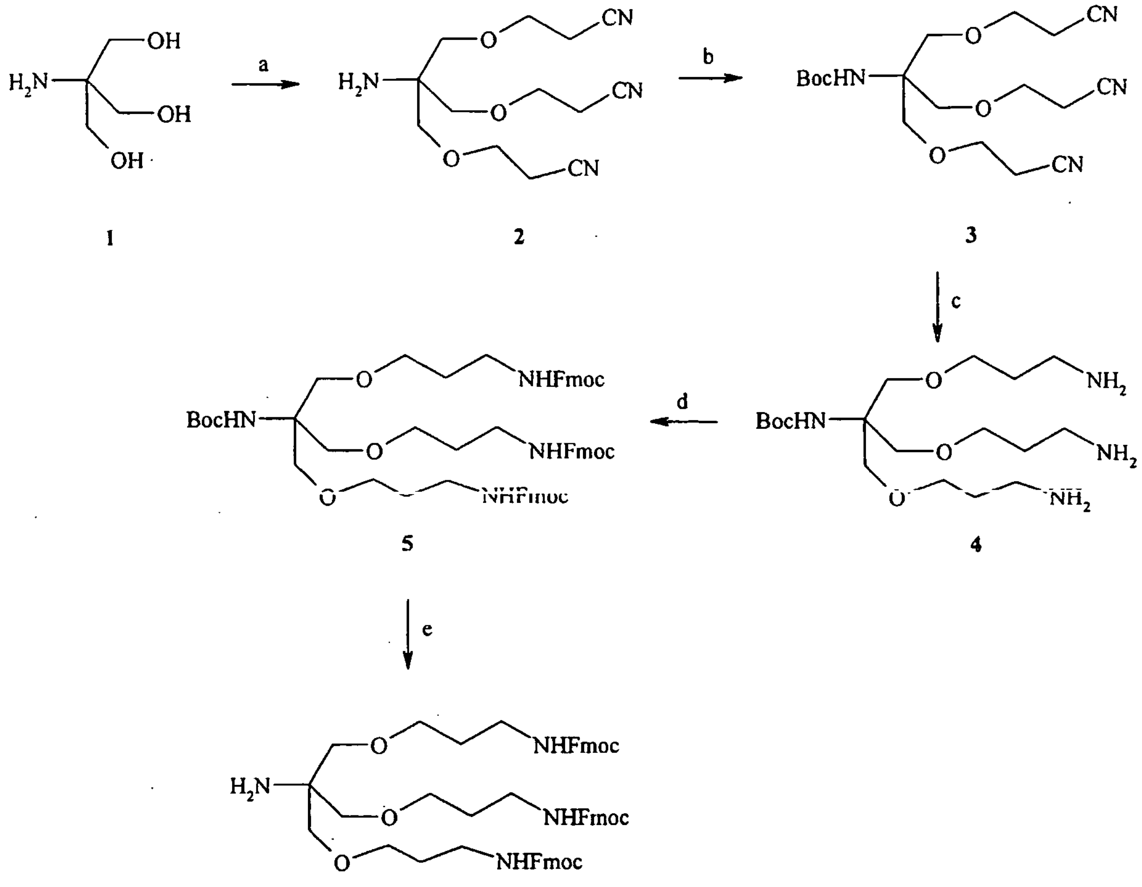


1 2 3 4 5

6
Monomère

**a.** 2g (1 éq.) de tris(hydroxyméthyl)aminométhane **1** ont été dissous dans du THF sous agitation et 40% de KOH aqueux a été ajouté, et ensuite 4,34 ml (4 éq.) d'acrylnitrile ont été ajoutés. Le mélange réactionnel a été maintenu à température ambiante sous agitation pendant 24 heures. Après évaporation d'une partie du THF, de l'eau a été ajoutée et ensuite extraite avec du dichlorométhane (DCM), séchée sur du sulfate de sodium. Après que le solvant ait été supprimé par évaporation, le produit brut **2** a été obtenu. Il a été ensuite purifié par chromatographie (EA/HEX/MeOH = 3,5/3,5/1). Masse : 2,5g ; Rendement : 54%.

**b.** 2 g (1 éq.) du composé **2** ont été dissous dans du THF sous agitation, et refroidis avec un bain de glace. 2,14 g (1,5 éq.) de $(Boc)_2O$ ont été dissous dans du THF dans un autre flacon, qui ont ensuite été ajoutés lentement dans le milieu réactionnel, après avoir ajouté 2,3 ml (1,5 éq.) de DIEA au milieu réactionnel. L'ensemble a été conservé à température ambiante et sous agitation pendant la nuit. Après évaporation d'une partie du THF, on a ajouté de l'acétate d'éthyle et lavé séquentiellement avec $KHSO_4$(1N) $H_2O$, $NaHCO_3$(st.) et NaCl (st.). Le produit **3** a ensuite été purifié par chromatographie (DCM/EA = 10/1). Poids : 2,39g ; Rendement : 71%.

**c.** Le composé **3** a été dissous dans MeOH/$CHCl_3$ (55/1). On a ajouté 10% de $PtO_2$ et ensuite le flacon a été balayé avec de l'hydrogène et maintenu sous atmosphère d'hydrogène pendant la nuit. Après filtration et evaporation du solvant, le composé **4** a été obtenu et séché sous vide.

**d.** Le composé **4** a été dissous dans l'eau sous agitation. 6 éq. de $NaHCO_3$ ont été ajoutés sous forme de poudre. 3,6 éq. de FmocOSu ont été dissous dans du THF et ajoutés au mélange réactionnel. La réaction a duré 4 heures et ensuite le THF a été évaporé. Le mélange restant a été extrait avec du DCM, et ensuite la phase organique a été lavée avec une solution 1 N de $KHSO_4$. Après avoir été séché sur du sulfate de sodium, le solvant a été supprimé par évaporation. Le composé final a été purifié par chromatographie (acétate d'éthyle/Hexane/acide acétique = 3/7/0,5).

**e.** Le composé **5** a été dissous dans du TFA pur sous agitation pendant 30 minutes, et ensuite le TFA a été retiré par évaporation pour donner le composé **6** (monomère). Le rendement global des deux dernières étapes est 73,3%.

Les réactions sur résines ont été effectuées dans une seringue sous agitation.

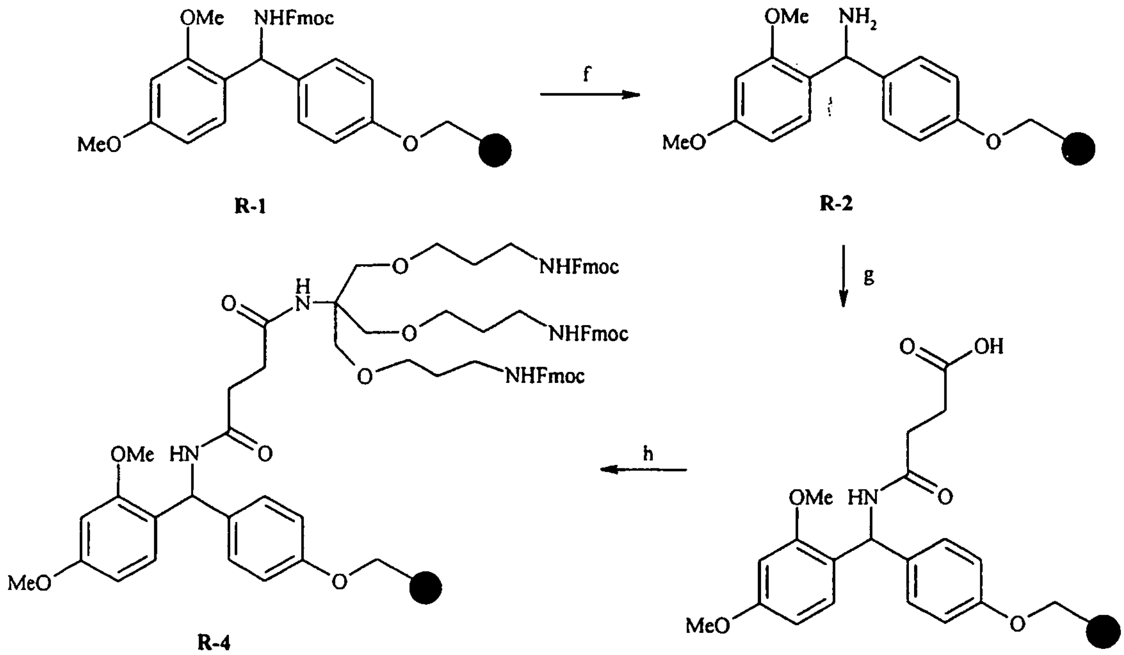


R-1

R-2

R-4

R-3

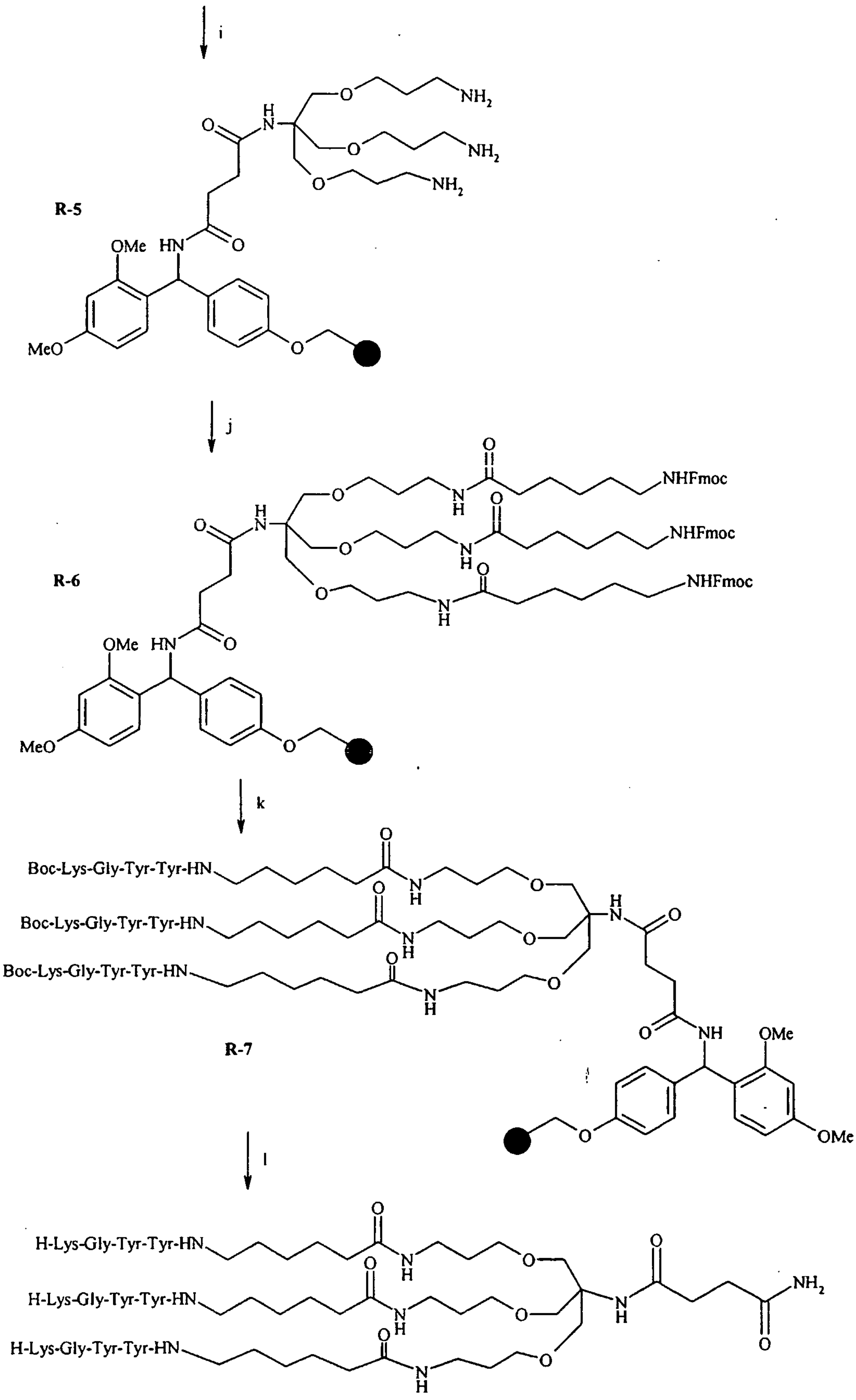


R-5

R-6

R-7

L-7

**f.** La résine amide de Rink **R-1** avec une charge de 0,62 mmol/g a été gonflée dans du diméthylformamide (DMF). Le groupe Fmoc a été retiré par 25% pipéridine/DMF. La réaction a été effectuée deux fois et chaque fois pendant 20 minutes.

**g.** La résine **R-2** a été gonflée dans du DCM et 10 éq. d'acide succinique et 1 éq. de pyridine ont été ajoutés. La réaction a été poursuivie jusqu'à ce que le test à la ninhydrine soit négatif. La résine a été filtrée et lavée avec du DCM et du DMF.

**h.** La résine **R-3** a été gonflée dans du DMF et ensuite 6 éq. de composé **6** (monomère 1), 5 éq. de BOP (benzotriazol-1-yloxy-tris(diméthylamino)phosphonium hexafluorophosphate), 5 éq. de HoBt (1-hydroxybenzotriazole) et 15 éq. de DIEA (diisopropyléthylamine) ont été ajoutés séquentiellement. Six heures plus tard, la résine a été filtrée et lavée avec du DMF, du DCM, de l'éther et séchée sous vide pour donner **R-4.** La charge est de 0,24 mmol/g après dosage UV quantitatif du groupement fluorényle, et la conversion globale est 84%.

**i.** Le groupe Fmoc a été retiré par 25% pipéridine/DMF pour donner **R-5.** La réaction a été effectuée deux fois et à chaque fois pendant 20 minutes.

**j.** La résine **R-5** a été gonflée dans du DMF. 15 éq. d'acide Fmoc-6-aminocaproïque, 15 éq. de BOP, 15 éq. de HoBt et 60 éq. de DIEA ont été ajoutés et couplés pendant 4h pour donner **R-6.** La réaction a été vérifiée par le test à la ninhydrine.

**k.** Le groupe Fmoc a été retiré par 25% pipéridine/DMF. 15 éq. de Fmoc-Xaa-OH, 15 éq. de BOP et 15 éq. de HoBt ont été dissous dans du DMF, et ensuite la solution a été ajoutée dans la résine, après avoir ajouté 60 éq. de DIEA. Pour chaque acide aminé, la réaction de couplage a été effectuée deux fois et à chaque fois pendant 30 minutes. Enfin, la résine **R-7** a été vérifiée par le test à la ninhydrine.

**l.** Le peptide synthétisé a été clivé avec TFA/$H_2O$ (95/5) pendant 30 minutes et ensuite la résine a été filtrée. La solution a été récupérée et précipitée dans de l'éther froid. Après avoir retiré l'éther, le peptide final **L7** a été séché et purifié par CLHP et analysé par spectrométrie de masse.

Des analogues de L-7 ont été synthétisés en suivant le même schéma de synthèse mais en changeant la séquence peptidique pour l'interaction avec CD40 :

Il s'agit de la séquence acétylée : Ac-Lys-Gly-Tyr-Tyr- pour donner la construction **L7-1**

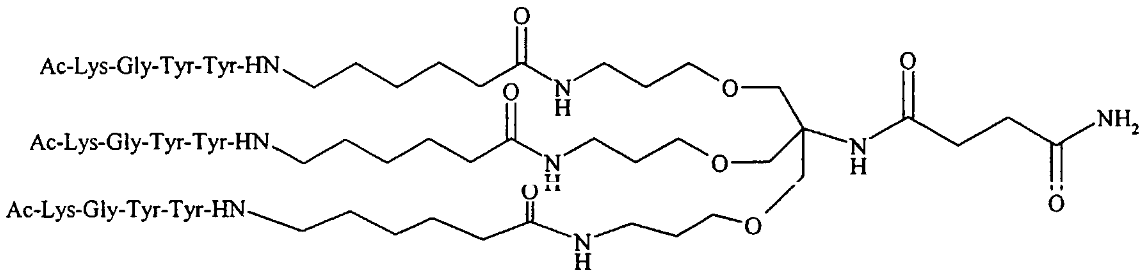

et de la séquence inversée : H-Tyr-Tyr-Gly-Lys- pour donner la construction **L7-2**

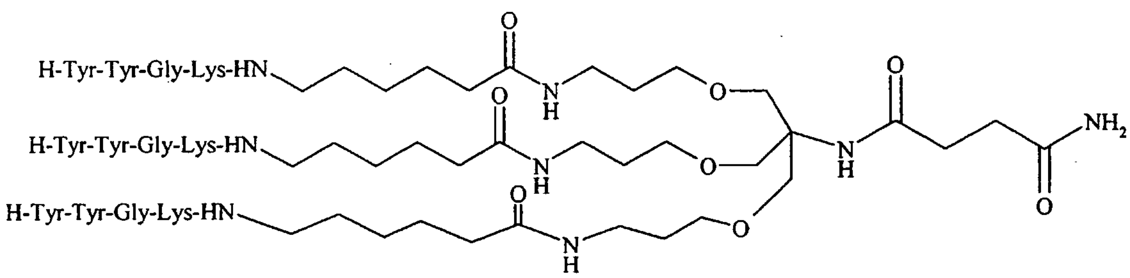

ou d'une modification du bras espaceur : remplacement de l'acide aminohexanoïque par une glycine : **L7-3**

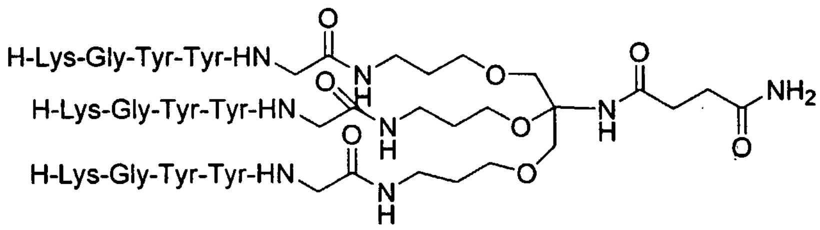

L-7-3

**F) PRÉPARATION DU COMPOSÉ L11**

Le ligand L11 répond à la formule suivante :

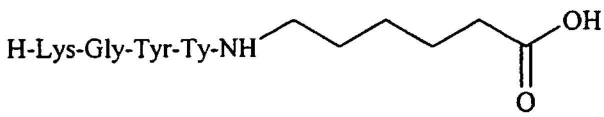

**Schéma réactionnel :**

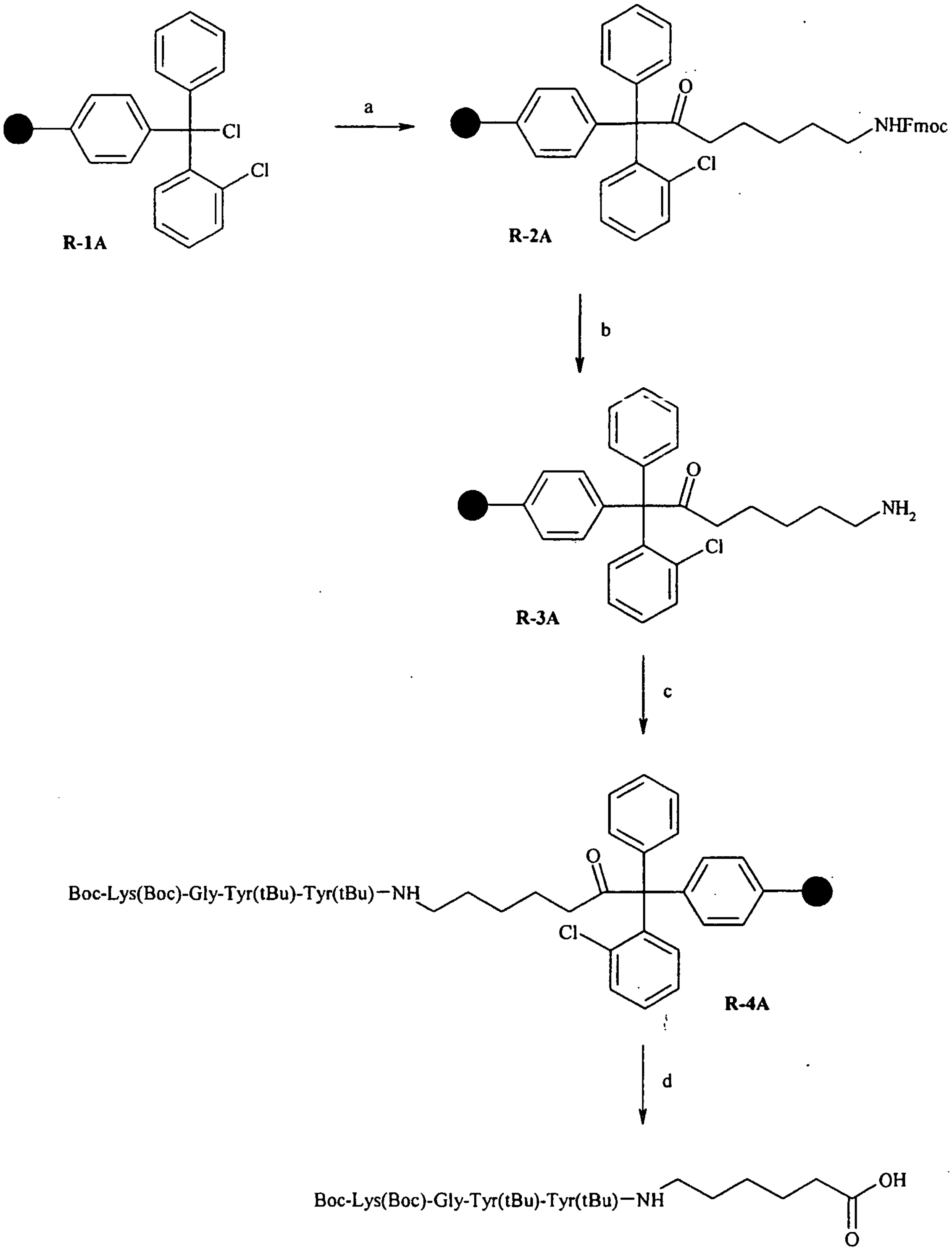


R-1A

R-2A

R-3A

R-4A

1-A

a) acide Fmoc-6-aminocaproïque, DIEA, DCM; b) 25% pipéridine/DMF ; c) Fmoc-Xaa-OH BOP, HoBt, DIEA, DMF; d) 1,1,1,3,3,3-hexafluoro-2-propanol/DCM (60/40)

**1) Préparation du pentapeptide protégé : Boc-Lys(Boc)-Gly-Tyr(tBu)-Tyr(tBu)-Ahx-OH**

Les réactions sur résine ont été effectuées dans une seringue et sous agitation.

1,5 g (1 éq.) de résine de 2-chlorotritylchlorure **(R1-A)** (0,96 mmol/g) ont été lavés deux fois avec du DCM distillé dans une seringue sous agitation. Un mélange de 1,02 g (2 éq.) d'acide Fmoc-6-aminocaproïque et 1,5 ml (6 éq.) de DIEA dans du DCM a été préparé et ajouté à la résine. Trois heures après, la résine a été filtrée et lavée avec du DCM. Elle a été de nouveau gonflée dans du méthanol sous agitation pendant 1 heure. Elle est ensuite filtrée et lavée avec du DMF, IpOH (isopropanol), du DCM, de l'éther. Elle est ensuite séchée sous vide pour donner **R-2A.**

La résine **R-2A** a été traitée avec une solution de 25% pipéridine/DMF pendant 20 minutes et la résine a ensuite été filtrée pour donner **R-3A** et la solution a été récupérée. La réaction a été effectuée deux fois. La charge de la résine 2 était 0,6 mmol/g par dosage UV quantitatif de la solution récupérée et la conversion de la réaction de couplage est 81 %.

Procédé général pour le couplage des acides aminés : 5 éq. de Fmoc-Xaa-OH, 5 éq. de BOP et 5 éq. de HoBt ont été dissous dans du DMF et ensuite le mélange a été ajouté à la résine qui a été prégonflée dans du DMF. 15 éq. de DIEA ont été ajoutés dans le système réactionnel. La réaction de couplage a été effectuée deux fois et à chaque fois pendant 30 minutes. La résine a été testée par le test à la ninhydrine.

Après répétition de ces procédés de déprotection et de couplage en utilisant les acides aminés nécessaires à la synthèse du peptide, la résine **R-4A** a été obtenue.

La résine **R-4A** a été mise dans une seringue. Un mélange de 1,1,1,3,3,3-hexafluoro-2-propanol et de DCM (60/40) a été ajouté à la résine. La seringue a été maintenue sous agitation pendant 2 heures et ensuite la résine a été filtrée et la solution récupérée. Après évaporation du solvant, le produit restant a été précipité dans de l'éther froid et ensuite purifié par CLHP pour donner **1-A.**

2) Le peptide **1-A** a été traité avec du TFA en présence d'eau et de triisopropylsilane (95:5:5) pour donner **L11** qui a été précipité dans de l'éther froid, centrifugé, lyophilisé et purifié par CLHP en phase inverse (RP-CLHP).

## G) PRÉPARATION DU COMPOSÉ L9

**1) Préparation de Z-beta-Lys(Boc)-OH (4B) et Z-beta-Lys(Boc)-OMe (5B)**

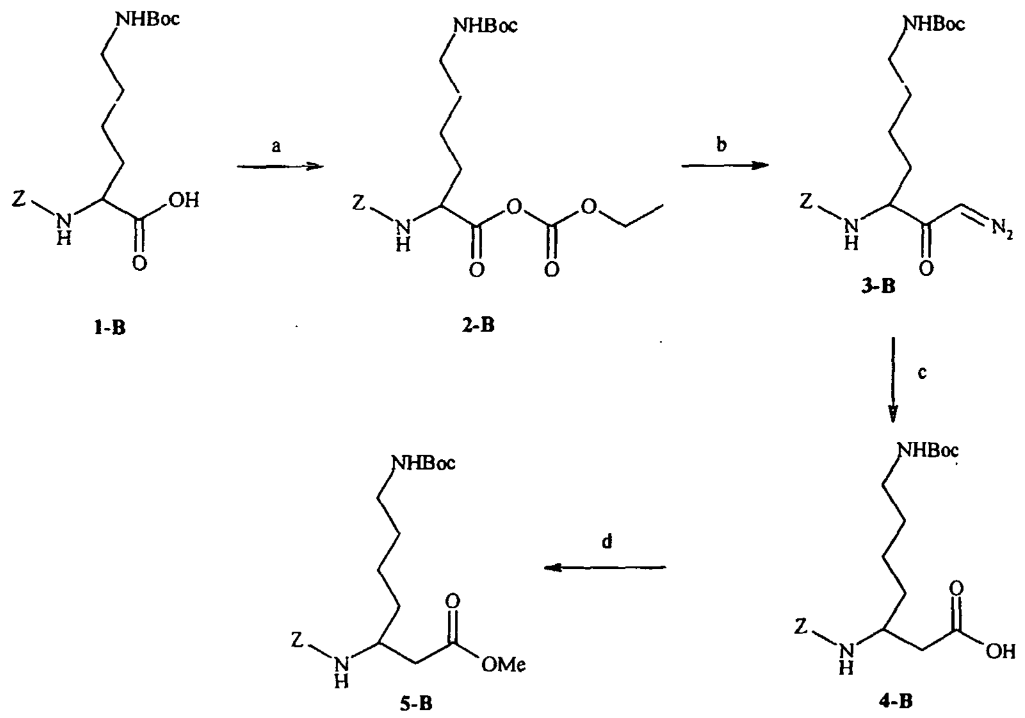


1-B 2-B 3-B 4-B 5-B

a) EtOCOCl, NMM, THF ; b) $CH_2N_2/Et_2O$; c) $CF_3COOAg$, NMM, THF/10% $H_2O$ d) iodométhane, $K_2CO_3$, acétonitrile

a) On a dissous 40 mmol (1 éq.) de Z-Lys(Boc)OH dans 100 ml de THF sous agitation. On a ajouté 5,27 ml

(1,2 éq.) de NMM, le mélange réactionnel a ensuite été refroidi à -20°C. On a dissous 4,59 ml (1,2 éq.) de chloroformate d'éthyle dans 30 ml de THF, et on a ensuite ajouté le tout goutte à goutte dans le système réactionnel. Le mélange réactionnel contenant **2-B** a été maintenu au froid pendant 20 minutes de plus.

b) Préparation du diazométhane :

On a dissous 7,76 g de KOH dans un mélange de 12,5 ml d'eau, 10 ml d'éther et 35 ml de 2-(2-éthoxyéthoxy)éthanol sous agitation. On a chauffé à 75°C. 15,52 g de diazald (N-méthyl-N-nitroso-p-toluènesulfonamide ; Aldrich) ont été dissous dans 140 ml d'éther et on a ajouté le tout goutte à goutte dans le mélange réactionnel. Le diazométhane ainsi produit a été condensé à -75°C dans un bain de glace dans de l'acétone. La solution éthérée de diazométhane ainsi produite a été ajoutée dans la solution réactionnelle de **2-B** et, ensuite, le mélange réactionnel a été maintenu a température ambiante pendant la nuit. On a ajouté 1 ml d'éthylamine (EA) et ensuite une large quantité de $NaHCO_3$ (st.). Après séparation des phases organique et aqueuse, la phase organique a été lavée avec $NaHCO_3$ deux fois et avec NaCl (st) une fois. Après séchage sur du sulfate de sodium, le solvant a été évaporé et la diazocétone **3-B** ainsi produite a été séchée sous vide. Masse : 15,4 g ; Rendement : 95%.

c) Réaction de réarrangement :

7 g (1 éq.) de la diazocétone **3-B** ont été dissous dans un mélange de 110 ml de THF et 18 ml d'eau sous agitation à l'abri de la lumière et l'ensemble a été refroidi à -20°C. 435 mg (0,11 éq.) de benzoate d'argent et 6,07 ml (2,5 éq.) de triéthylamine ont été ajoutés. Le mélange réactionnel a été maintenu à température ambiante pendant la nuit dans l'obscurité. Une partie du THF a été évaporée, on a ajouté 20 ml d'éther qui a été extrait avec $NaHCO_3$ plusieurs fois. La phase aqueuse a été combinée et acidifiée à pH 3 avec de la poudre de $KHSO_4$. La solution a été extraite avec du DCM et séchée sur du sulfate de sodium. Après évaporation du solvant, le produit final **4-B** a été séché sous vide. Masse : 5 g ; Rendement : 73 %.

d) On a dissous 1,67 g (1 éq.) d'acide aminé β **4-B** tel qu'obtenu précédemment dans de l'acétonitrile et on a refroidi l'ensemble à 0°C avec un bain de glace. Au bout d'une heure, on a ajouté 1,32 ml (5 éq.) d'iodométhane. Le mélange réactionnel a été maintenu à température ambiante pendant la nuit. L'acétonitrile a été évaporé et on a ajouté de l'acétate d'éthyle, qui a été lavé séquentiellement avec $NaHCO_3$(st.), $KHSO_4$ (1N) et NaCl (st.). La phase organique a été séchée sur du sulfate de sodium et le solvant a été supprimé par évaporation pour donner **5-B.** Masse : 1,53g ; Rendement : 88%.

**2) Construction de la molécule coeur protégée :**

**Schéma réactionnel :**

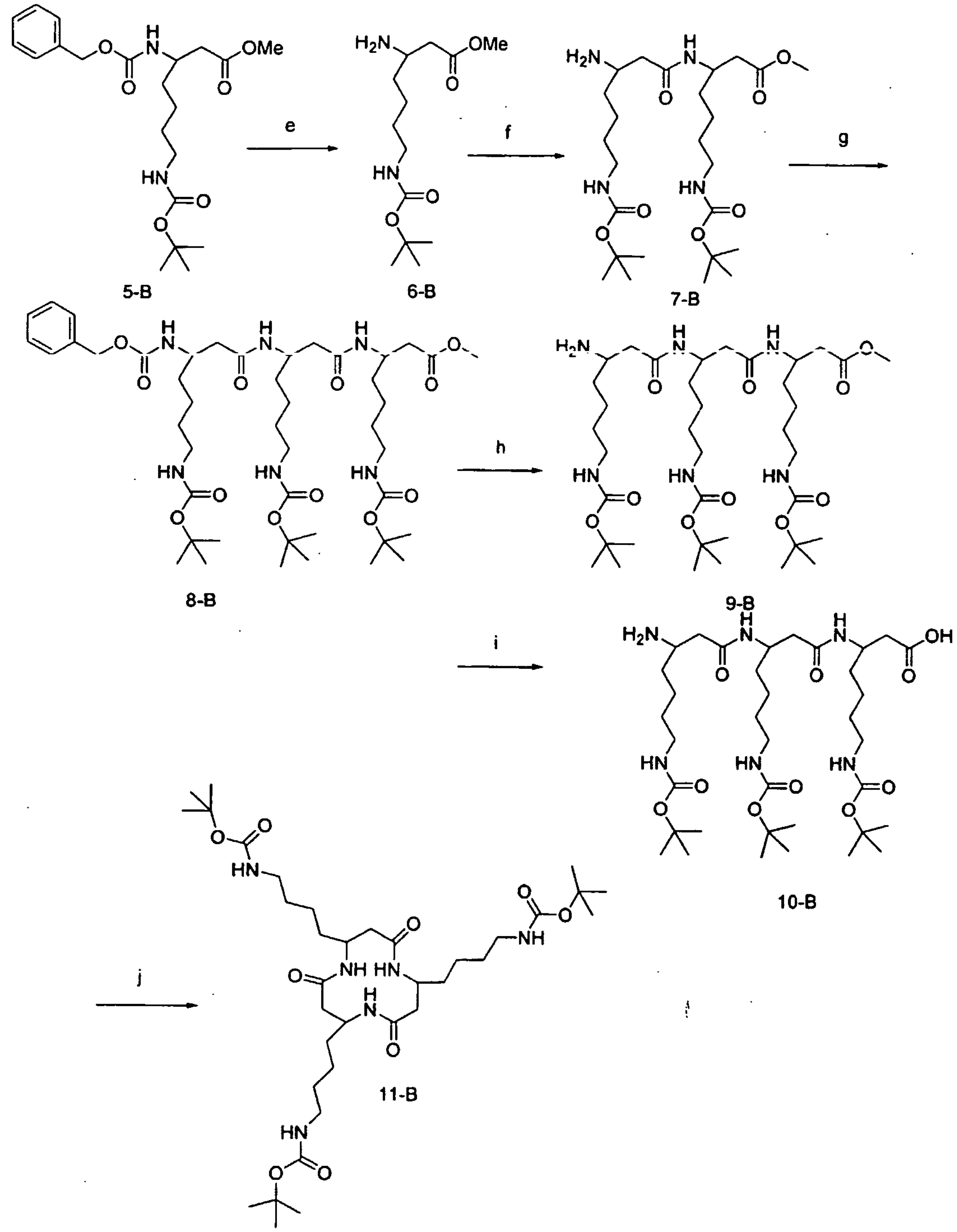


5-B 6-B 7-B 8-B 9-B 10-B 11-B

e) On a dissous 765 mg (1 éq.) de composé **5-B** dans de l'éthanol sous agitation et on a ajouté 10% de Pd/C (en poids). Le flacon a été balayé avec de l'hydrogène trois fois et maintenu sous une atmosphère d'hydrogène pendant 2 heures. Après filtration, le solvant a été supprimé par évaporation et le produit **6-B** restant a été séché sous vide. Masse : 400 mg ; Rendement : 98%.

f) On a dissous 657 mg (1 éq.) de composé **4-B** dans 3 ml de DMF sous agitation et ensuite on a ajouté séquentiellement 40 mg (1,1 éq.) de composé **6-B,** 738 mg (1 éq.) de BOP et 726 µl (2,5 éq.) de DIEA. Deux heures après, le mélange réactionnel a été précipité dans $NaHCO_3$(st). Après filtration, le solide a été lavé avec $H_2O$, $KHSO_4$, NaCl(st.) et ensuite séché sous vide. Masse : 1 g ; Rendement : 84%. Ce composé a été hydrogéné dans MeOH pour donner **7-B** dans un rendement quantitatif.

g) On a dissous 1 équivalent de composé **4-B** dans 3 ml de DMF sous agitation et ensuite on a ajouté séquentiellement 1,1 éq. de composé **7-B,** 1 éq. de BOP, 2,5 éq. de DIEA. Au bout de deux heures, le mélange réactionnel a été précipité dans $NaHCO_3$(st). Après filtration, le solide a été lavé avec $H_2O$, $KHSO_4$, NaCl(st.) et ensuite séché sous vide pour donner **8-B.**

h) On a dissous 100 mg de composé **8-B** dans du méthanol sous agitation et on a ajouté 10% de Pd/C (en poids). Le flacon a été balayé avec de l'hydrogène trois fois et maintenu sous une atmosphère d'hydrogène pendant 2 heures. Après filtration, le solvant a été retiré par évaporation et le produit restant a été séché sous vide pour donner **9-B.** Masse :78 mg ; Rendement : 92%.

i) On a dissous 78 mg (1 éq.) de composé **9-B** dans du méthanol et on a ensuite ajouté 10 éq. de NaOH aqueuse 2N. La réaction a été contrôlée par chromatographie sur couche mince CCM (solvants : acétate d'éthyle/pyridine/acide acétique/eau). A la fin de la réaction, le méthanol a été évaporé et on a ajouté de l'eau. Le mélange a été acidifié avec de l'acide acétique à pH 3 et un précipité s'est formé. Le solide a été récupéré par filtration et séché sous vide pour donner **10-B.** Masse : 50 mg ; Rendement : 65%.

j) On a dissous 50 mg (1 éq.) de composé **10-B** dans 3 ml de DMF et 42 μl (3,5 éq.) de DIEA ont été ajoutés. 36 mg (1,2 éq.) de BOP ont été dissous dans 3 ml de DMF sous agitation. La solution préparée précédemment a été ajoutée goutte à goutte. Le mélange réactionnel a été maintenu à température ambiante pendant 2 jours et ensuite précipité dans $NaHCO_3$(st.). Le précipité a été récupéré et lavé avec $H_2O/KHSO_4$ et $H_2O$, puis séché sous vide pour donner **11-B.** Masse : 37 mg ; Rendement : 76%.

### 3) Déprotection de la molécule coeur et synthèse de L-9

**Schéma réactionnel :**

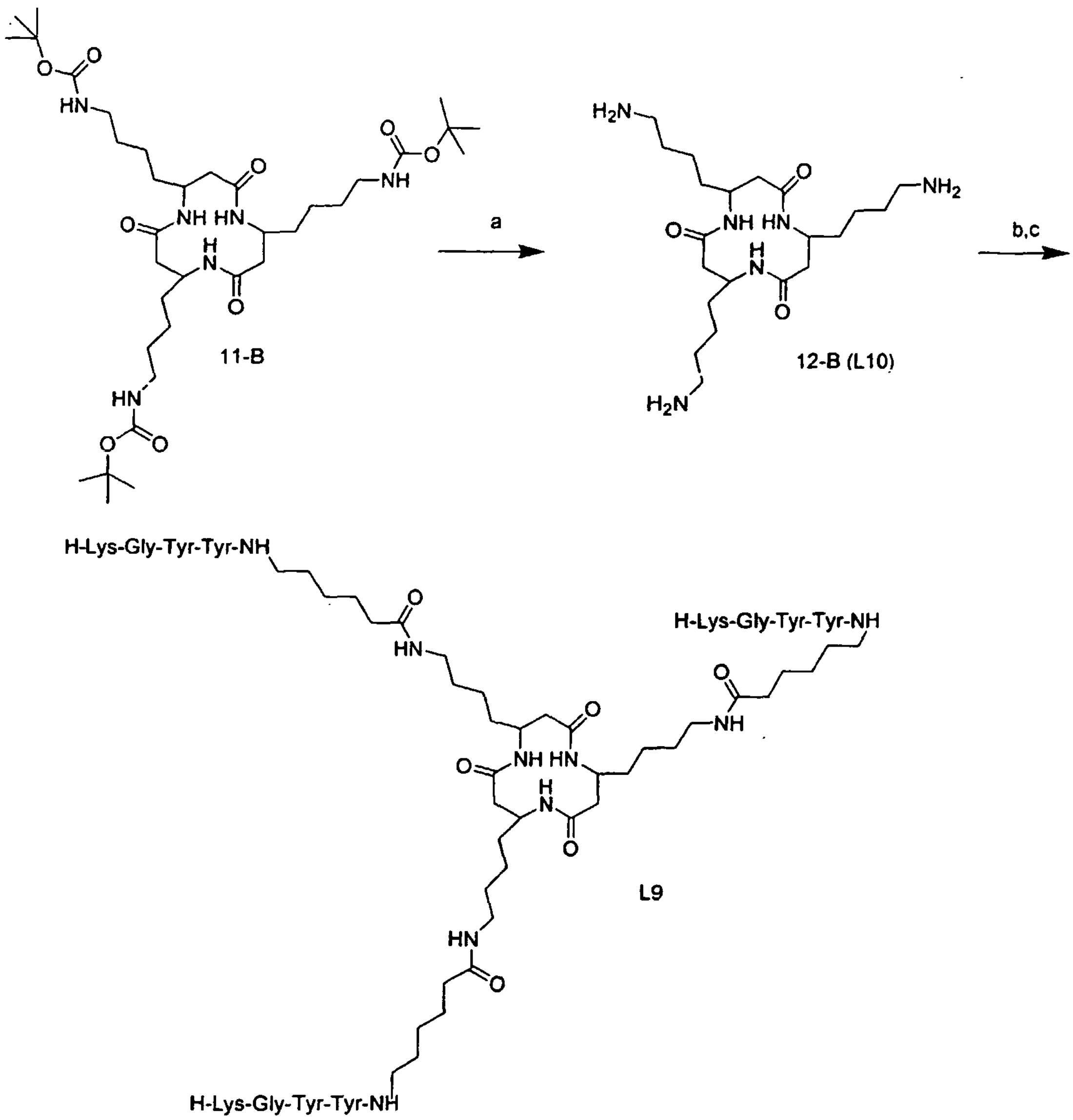


11-B

12-B (L10)

L9

a) 50 mg (1 éq.) de composé **11-B** ont été dissous dans du TFA sous agitation pendant 30 minutes et ensuite le TFA a été retiré par évaporation. Le produit a été séché sous vide pour donner **12-B** (également nommé **L10**).

b) Le composé **12-B** a été dissous dans du DMF sous agitation. 217 mg (3,3 éq.) du pentapeptide **1-A** qui est synthétisé en phase solide comme décrit ci-dessus, 100 mg (3,3 éq.) de BOP, 39,5 µl (10 éq.) de DIEA ont été ajoutés dans le système réactionnel. La réaction a duré pendant 25 heures et ensuite le mélange réactionnel a été précipité dans une grande quantité de $NaHCO_3$(aq.). Le précipité a été filtré et lavé avec $H_2O/KHSO_4$, $H_2O$ et de l'éthylamine (EA). Le solide a été séché sous vide pour donner **13-B.** Masse : 153 mg ; Rendement : 69%.

c) Le traitement de **13-B** avec du TFA a donné le composé du titre dans un rendement quantitatif. Le composé L9 pur a été obtenu par purification par RP-CLHP (chromatographie en phase inverse).

**H) PRÉPARATION DE L12 :**

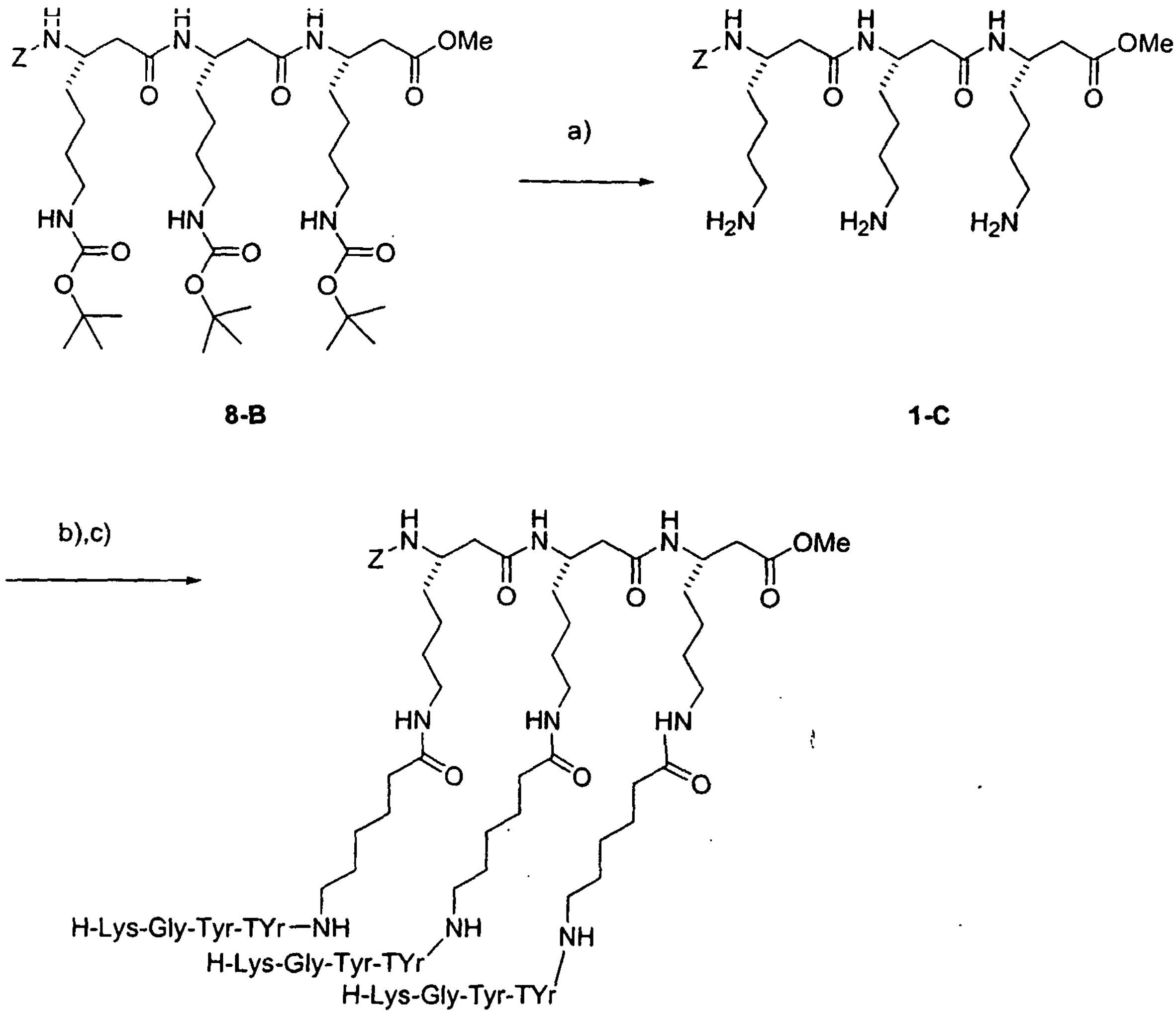


**8-B** **1-C**

**L-12**

a) On a dissous 1 équivalent de composé **8-B** dans du TFA sous agitation pendant 30 minutes et ensuite le TFA a été retiré par évaporation. Le produit a été séché sous vide pour donner **1-C.**

b) Le composé **1C** a été dissous dans du DMF sous agitation. 3,3 éq. du pentapeptide **1-A** qui est synthétisé en phase solide comme décrit ci-dessus, 3,3 éq. de BOP et 10 éq. de DIEA ont été ajoutés dans le système réactionnel. La réaction a duré 25 heures et ensuite le mélange réactionnel a été précipité dans une grande quantité de $NaHCO_3$ (aq.). Le précipité a été filtré et lavé avec $H_2O/KHSO_4$, $H_2O$ et de l'éthylamine. Le solide a été séché sous vide pour donner **2-C.** Masse : 153 mg ; Rendement : 69%.

c) Le traitement de **2-C** avec du TFA a donné le composé **L-12** dans un rendement quantitatif. Le composé **L-12** pur a été obtenu par purification par chromatographie RP-HPLC.

**TESTS BIOLOGIQUES :**

Ces tests s'effectuent en 3 phases. On choisit d'abord les ligands les plus intéressants par des tests simples, puis leurs effets physiologiques sont testés dans des modèles d'activations cellulaires *in vitro.* Enfin, les ligands les plus intéressants sont testés dans des modèles murins *in vivo.*

**I - SÉLECTION DES LIGANDS :**

Une fois l'intégrité structurale de ces ligands analysée, leur liaison est testée sur des molécules CD40 solubles ou membranaires. Pour cela, on mesure l'inhibition de la liaison du CD40L soluble sur la molécule CD40 adsorbée à

du plastique ou présentée par des cellules B normales ou transformées (lymphome de Burkitt). Cette étape est évaluée à l'aide d'un test ELISA et d'un marquage en cytométrie de flux permettant de visualiser la liaison CD40/CD40L.

Ensuite, l'affinité de ces ligands pour la molécule CD40 est évaluée à l'aide d'un biocapteur (BIAcore™). L'effet agoniste ou antagoniste des ligands a été testé grâce à deux tests biologiques simples. Dans le premier, on étudie l'expression de la molécule membranaire CD95 par des cellules B transformées (lymphomes de Burkitt) après activation par l'interaction CD40-CD40L (Schattner et al., 1996). Dans un tel modèle, le partenaire CD40L est exprimé sur des fibroblastes transfectés (3T6-CD40L)(Buelens et al., 1997). Les cellules du lymphome de Burkitt BL41 sont incubées avec des fibroblastes 3T6 non transfectées (Figures 3A et 3C) ou avec des fibroblastes 3T6 transfectés par CD40L (Figures 3B et 3D). Après 48h, l'expression de CD95 est évaluée par cytométrie de flux. L'expression de CD95 est induite en présence de CD40L (Figure 3B). Un anticorps anti-CD40L commercial inhibe les conséquences de l'interaction CD40-CD40L (Figure 3D).

Le second test est basé sur la propriété de certains lymphomes B de cesser de proliférer et d'entrer en apoptose lors du pontage de leur molécule CD40 membranaire (Tong et al., 1999). Dans ce test, on incube les cellules BL41 avec des ligands de CD40 et on mesure après 24h soit l'inhibition de prolifération de ces cellules, en étudiant l'incorporation de thymidine tritiée, soit le pourcentage de cellules apoptotiques par une étude en cytométrie de flux (Figures 4A et 4B).

L'effet agoniste des ligands est évalué par la mesure après incubation des cellules B avec les ligands soit de l'expression de CD95 en cytométrie de flux soit de l'inhibition de la prolifération et/ou de l'augmentation de la mort par apoptose.

L'effet antagoniste est évalué par la mesure de la diminution de l'expression de CD95 induite par le CD40L en présence des différents ligands chimiques (voir Tableau I). Grâce à ces tests, on sélectionne les ligands les plus intéressants pour évaluer leur activité *in vivo* et *in vitro* dans des modèles plus complexes.

### A) PRINCIPE DES TESTS BIOLOGIQUES

#### 1- Expression de CD95 induite par CD40L

Les cellules de lymphomes B expriment la molécule CD95 lorsqu'elles reçoivent un signal par l'intermédiaire de la molécule CD40, qu'elles expriment de manière constitutive. Ce signal est généralement apporté par la molécule CD40L (CD154) exprimée par une autre cellule. Les ligands antagonistes inhibent la liaison de CD40 sur CD40L et bloquent donc l'expression de CD95. Les ligands agonistes miment la molécule CD40L et induisent l'expression de CD95 même en absence de la cellule exprimant CD40L.

<u>Mode opératoire</u>

Les cellules du lymphome de Burkitt BL41 ($5.10^5$/ml) sont cultivées en présence de fibroblastes 3T6 ($10^5$/ml) transfectés (3T6-CD40L) ou non avec CD40L (3T6), et traitées à la mitomycine pour arrêter leur prolifération. Les ligands sont ajoutés au début de la culture à la concentration désirée. Après 48h de culture, l'expression de la molécule CD95 est mesurée en cytométrie de flux. Les cellules BL41 sont distinguées des fibroblastes en utilisant un anticorps anti-CD19 (marqueur spécifique des cellules B).

<u>Résultats</u>

Les cellules BL41 cultivées en présence des cellules 3T6 n'expriment pas la molécule CD95 (Figure 3A). Par contre, l'expression de CD95 est induite à la surface des cellules BL41 en présence de cellules 3T6-CD40L (Figure 3B). Un anticorps anti-CD40L commercial, qui bloque l'interaction CD40/CD40L, inhibe complètement l'expression de CD95 induite sur les cellules BL41 en présence des cellules 3T6-CD40L (Figure 3D).

L'expression de CD95 est inhibée par le ligand L1 à 100 et 50 μM (Tableau 1). Le ligand L3, qui a la même structure coeur que L1, mais qui présente une séquence en acides aminés différente inhibe fortement l'expression de CD95 induite par CD40L de 100 à 25 μM. Le ligand L2, qui correspond à la structure coeur des ligands L1 et L3, n'a aucun effet sur l'expression de CD95. Le ligand L7, constitué d'une structure coeur branchée portant la même séquence peptidique que L3, n'a aucun effet sur l'expression de CD95 induite par CD40L. Par contre, le ligand L4, à structure coeur cyclique portant 3 exemplaires de la séquence peptidique mimant l'interface CD40-CD40L, inhibe l'expression de CD95 induite par CD40L pour une gamme de concentrations allant de 50 à 0,5 μM. Son activité est donc environ 10 fois plus importante que le ligand linéaire qui présente le même peptide (L3). Il est intéressant de noter que l'inhibition maximale est obtenue pour une concentration de 10 μM, la concentration de 50 μM induisant une mortalité cellulaire importante (cf. partie 2). Le ligand L4 bis qui est constitué de la même molécule coeur mais qui ne porte que 2 exemplaires de la séquence peptidique mimant l'interface CD40-CD40L n'inhibe que très faiblement l'expression de CD95 induite par CD40L.

**Tableau I**
**% d'inhibition de l'expression de CD95 induite par CD40L après traitement avec**

| | L1 | L2 | L3 | L4 | L4bis | L7 |
|---|---|---|---|---|---|---|
| **100 μM** | 60 | 0 | 60 | | | |
| **50 μM** | 43 | 0 | 62 | 22 | 13 | 0 |
| **25 μM** | 0 | 0 | 25 | | | |
| **10 μM** | | | 13 | 40 | | |
| **5 μM** | | | 0 | 36 | 8 | 0 |
| **0,5 μM** | | | | 19 | 0 | 0 |

**2- Induction de l'apoptose des lymphomes B par CD40L**

Certains lymphomes B entrent en apoptose lors du pontage de leur molécule CD40 par un anticorps anti-CD40 ou par du CD40L soluble de manière indépendante de la molécule CD95. Cette induction d'apoptose se traduit par une diminution de prolifération mesurée par l'incorporation de thymidine tritiée. Les ligands agonistes de CD40 vont induire une augmentation de l'apoptose et par conséquent une diminution de l'incorporation de thymidine tritiée.

Mode opératoire

Les cellules du lymphome de Burkitt BL41 ($4.10^5$/mL) sont cultivées en présence des différents ligands à la concentration désirée. Après 24h, les cellules sont soit incubées pendant 8h avec de la thymidine tritiée (1μci/puits) pour mesurer la prolifération soit marquées avec de l'Annexine V FITC et de l'iodure de propidium pour évaluer le pourcentage de cellules apoptotiques en cytométrie de Flux.

Résultats

Le ligand L4 (coeur à structure cyclique + 3 exemplaires de la séquence peptidique mimant l'interface CD40-CD40L) tout comme la molécule CD40L soluble inhibe très fortement la prolifération des cellules BL41. Par contre le ligand L7 (coeur à structure branchée portant la même séquence peptidique que L4) et ses dérivés L7-1, L7-2 et L7-3 n'ont que peu ou pas d'effet sur la prolifération des cellules BL41 (Figure 4A).

Cette inhibition de prolifération s'accompagne pour le ligand L4 d'une forte augmentation de l'apoptose alors que le ligand L4bis (structure coeur cyclique + 2 exemplaires de la séquence peptidique) induit une très faible apoptose. Le ligand L8 constitué de la structure coeur seule ou le ligand L11 constitué de la séquence peptidique seule n'ont aucun effet sur la mort par apoptose des cellules BL41 (Figure 4B).

Le ligand L9 qui est constitué d'une structure coeur cyclique différente de celle du ligand L4 portant 3 exemplaires de la même séquence peptidique induit aussi fortement l'apoptose de BL41 (Figure 4B).

En conclusion, les ligands L4 et L9 ont une activité agoniste et miment CD40L.

**B) ÉTUDE DE L'INTERACTION ENTRE LE CD40 ET LE CD40L ET LES LIGANDS L1 à L12 :**

Principe de la méthode

Le Biacore3000 est un appareil qui permet l'étude des interactions entre deux molécules, basé sur la résonance plasmonique de surface. Il permet de mesurer en temps réel, et donc de suivre les cinétiques d'interaction (association et dissociation) d'un analyte (qui se trouve dans une solution injectée) et d'un ligand (immobilisé sur une micropuce supportant la mesure). Les mesures cinétiques à différentes concentrations d'analyte permettent de calculer les constantes d'affinités de l'interaction entre le ligand et les analytes. La micropuce contient quatre cellules de mesure différentes, ce qui permet de comparer directement une cellule de référence sur laquelle une protéine non-pertinente (protéine qui n'a aucune affinité avec le ligand étudié), mais proche du ligand est immobilisée avec la cellule sur laquelle le ligand est immobilisé.

Mode opératoire

Sur une micropuce ont été immobilisés des anticorps de lapin dirigés contre la partie constante d'immunoglobulines de souris. Dans la cellule de référence on immobilise avec un flux de 5 μL/min durant 2 minutes un anticorps

monoclonal de souris d'isotype IgG2a (LG112) à 40 nM ; dans la cellule du ligand on immobilise dans les mêmes conditions le CD40 recombinant associé à la partie constante de la chaîne lourde d'IgG2a de souris (human CD40 mulg fusion protein, ANCELL Corporation, Bayport, MN).

Sur les deux cellules (référence et ligand) sont ensuite injectés, comme analyte, le CD40L (human CD154 muCD8 fusion protein, ANCELL Corporation) à différentes concentrations ou le CD40L à une concentration de 1 μM ou de 125 nM, en présence de différents concentrations de ligands. Le flux en présence des analytes est de 10 μL/min pendant 5 minutes pour étudier l'association et, en absence d'analytes, les conditions sont les mêmes pour étudier la dissociation.

Afin de régénérer les cellules (c'est-à-dire enlever toutes les protéines adsorbées de manière non covalente), une solution de 10 mM de HCl est injectée à 5 μL/min durant 1 minute. Les cellules sont ensuite prêtes pour une nouvelle analyse.

Résultats

Afin d'étudier la constante d'affinité du CD40L pour le CD40, on utilise 4 concentrations de CD40L de 62,5 jusqu'à 500 nM. La constante d'équilibre est calculée à 54 nM.

Pour étudier l'association avec L1, l'analyte était constitué de CD40L à 1μM et de L1 à 50 μM. Bien qu'une inhibition soit observée durant les premières secondes de l'interaction, elle disparaît au cours des cinq minutes d'association, rendant impossible le calcul de la constante d'équilibre de L1. Celle-ci est donc plus grande que 50 μM.

L'association de L3 fut étudiée en présence de 125 nM de CD40L. L'analyse cinétique en présence de 2,5 μM de L3 a donné une constante d'équilibre pour L3 de 10 μM.

L'association de L4, de L7 (et de ses dérivés), de L8, de L9 de L11 et de L12 a été étudiée en présence de 100nM de CD40L. Seules les molécules L4 et L9 se fixent sur CD40 et déplacent CD40L. L'analyse cinétique en présence de 40 et 80nM de L4 donnait une constante d'équilibre pour L4 de 180nM. Le ligand L4 inhibait 50% de la fixation de CD40L (IC50) à 80 nM. Le ligand L9, testé dans les mêmes conditions, a une IC50 qui est inférieure à 50nM, sa constante d'équilibre est donc inférieure à celle de L4. Il est intéressant de remarquer que la molécule qui est une version linéaire de L9 ne se fixe pas à CD40. Ces résultats suggèrent que la conception de ligand actifs de CD40 nécessite l'utilisation d'une molécule coeur (A dans la formule générale : $A\text{-}X_n$) suffisamment rigide et de symétrie C3.

## II - TESTS DES LIGANDS DANS DES MODÈLES *IN VITRO* :

Les cellules dendritiques immatures, différenciées *in vitro* à partir de monocytes humains, sont très sensibles au CD40L qui induit leur maturation. Cette maturation s'accompagne (i) de profonds remaniements phénotypiques, (ii) d'une sécrétion de cytokines et de chimiokines, (iii) d'une résistance à l'apoptose médiée par le CD95 (Koppi et al., 1997 ; Bjorck et al., 1997), (iv) d'une longévité accrue des cellules dendritiques (Miga et al., 1997) et (v) d'une capacité nettement accrue des cellules dendritiques matures à stimuler des lymphocytes T allogéniques. On étudie l'effet de ces ligands sur les CDs. On étudie en premier lieu, par cytométrie en flux, la capacité des ligands agonistes potentiels à modifier le phénotype des CDs immatures. On poursuit cette étude en cherchant à mettre en évidence, à l'aide de tests ELISA, le déclenchement par les ligands agonistes d'une sécrétion de cytokines. Enfin, cette recherche est complétée par l'étude de la capacité des CDs, préincubées en présence des molécules agonistes, à stimuler des cellules T allogéniques. L'effet des ligands antagonistes est évalué par la mesure de la diminution des variations, normalement induites par le CD40L, observée en préincubant les CDs en présence des molécules antagonistes. L'effet des ligands peut aussi être testé (i) sur la commutation de classes des lymphocytes B, qui peut être mimée *in vitro* par activation de cellules B des amygdales par CD40 en présence de cytokines, et (ii) sur la différenciation des lymphocytes T cytotoxiques qui peut être mimée par une co-incubation de cellules dendritiques activées par CD40 et des cellules T pré-cytotoxiques.

## III - TESTS DES LIGANDS DANS DES MODÈLES *IN VIVO* :

Les ligands L4 et L9 s'étant révélés agonistes, ils représentent des touches intéressantes pour tester l'effet thérapeutique de nos molécules. Le lupus érythémateux disséminé est une maladie auto-immune systémique dans laquelle l'importance de l'interaction CD40-CD40L est bien documentée. Le traitement de souris lupiques par des anticorps ani-CD40 agonistes accélère fortement la maladie lupique. Pour tester l'effet *in vivo* des différents ligands, nous les avons injectés à des souris qui développent spontanément une maladie lupique présentant des symptômes proches du lupus érythémateux disséminé humain. Nous avons étudié l'effet des ligands sur le développement de la maladie.

Mode opératoire

Des souris MRL-$^{lpr}$ (Koopman et al., 1988) pré-autoimmunes (âgées de 5 semaines) sont injectées par voie

intraveineuse avec 100 µL par souris de PBS contenant ou non 100 µg de ligand L4. L'injection est répétée 2 fois à 2 semaines d'intervalle. Le sérum de ces souris est prélevé régulièrement par saignée rétro-orbitale. Le développement de la maladie lupique est suivi par une mesure de la protéinurie dans les urines, de la présence d'anticorps anti-ADN dans le sérum par un test ELISA et de la survie des souris. Ces deux marqueurs sont caractéristiques du développement de la, maladie lupique chez les souris MRL-$^{lpr}$.

Résultats

Les souris traitées par le ligand L4 meurent significativement (p= 0,0101) plus vite que les souris traitées par du PBS (Figure 5A). Cette accélération de la mortalité s'accompagne d'une apparition plus précoce des anti-ADN dans le sérum (Figure 5B) et de la protéinurie dans les urines (Figure 5C), montrant que la mortalité est sans doute due à une accélération de la maladie lupique. Ces résultats montrent clairement que le ligand L4 a un effet agoniste *in vivo.*

**RÉFÉRENCES**

- Bjorck, P., Banchereau, J., Flores-Romo, L. (1997) CD40 ligation counteracts Fas-induced apoptosis of human dendritic cells, *Int Immunol.,* **9:** 365-72,
- Buelens, C., Verhasselt, V., De Groote, D., Goldman, M., Willems, F. (1997) Human dendritic cell responses to lipopolysaccharide and CD40 ligation are differentially regulated by interleukin-10, *Eur J Immunol.*, **27:** 1848-52,
- Chaussabel, D., Jacobs, F., de Jonge, J., de Veerman, M., Carlier, Y., Thielemans, K., Goldman, M., Vray, B. (1999) CD40 ligation prevents *Trypanosoma cruzi* infection through interleukin-12 upregulation, *Infect Immun.,* **67:** 1929-34,
- Diehl, L., den Boer, A.T., Schoenberger, S.P., van der Voort, E.I., Schumacher, T.N., Melief, C.J., Offringa, R., Toes, R.E. (1999) CD40 activation *in vivo* overcomes peptide-induced peripheral cytotoxic T-lymphocyte tolerance and augments anti-tumor vaccine efficacy, *Nat Med.,* **5**: 774-9,
- Howard, L.M., Miga, A.J., Vanderlugt, C.L., Dal Canto, M.C., Laman, J.D., Noelle, R.J., Miller, S.D. (1999) Mechanisms of immunotherapeutic intervention by anti-CD40L (CD154) antibody in an animal model of multiple sclerosis, *J Clin Invest.*, **103:** 281-90,
- Kaiser, E. et coll. (1970) *Anal. Biochem.*, **34**, 595-598,
- Kikuchi, T., Moore, M.A., Crystal, R.G. (2000) Dendritic cells modified to express CD40 ligand elicit therapeutic immunity against preexisting murine tumors, *Blood.* **96:** 91-9,
- Kirk, A.D., Burkly, L.C., Batty, D.S., Baumgartner, R.E., Berning, J.D., Buchanan, K., Fechner, J.H., Jr., Germond, R.L., Kampen, R.L., Patterson, N.B., Swanson, S.J., Tadaki, D.K., TenHoor, C.N., White, L., Knechtle, S.J., Harlan, D.M. (1999) Treatment with humanized monoclonal antibody against CD154 prevents acute renal allograft rejection in non-human primates, *Nat Med.*, **5:** 686-93,
- Koopman W.J. and Gay, S. (1988) The MRL-*lpr*/*lpr* mouse. A model for the study of rheumatoid arthritis. *Scand. J. Rheumatol.* **75**, 284-289,
- Koppi, T.A., Tough-Bement, T., Lewinsohn, D.M., Lynch, D.H., Alderson, M.R. (1997) CD40 ligand inhibits Fas/CD95-mediated apoptosis of human blood-derived dendritic cells, *Eur J Immunol.*, **27:** 3161-5,
- Locksley et al. (2001) *Cell,* **104**, 487-501,
- Lode, H.N., Xiang, R., Pertl, U., Forster, E., Schoenberger, S.P., Gillies, S.D., Reisfeld, R.A. (2000) Melanoma immunotherapy by targeted IL-2 depends on CD4(+) T-cell help mediated by CD40/CD40L interaction, *J Clin Invest.*, **105:** 1623-30,
- Miga, A.J., Masters, S.R., Durell, B.G., Gonzalez, M., Jenkins, M.K., Maliszewski, C., Kikutani, H., Wade, W.F., Noelle, R.J. (2001) Dendritic cell longevity and T cell persistence is controlled by CD154-CD40 interactions, *Eur J Immunol.*, **31:** 959-965,
- Schattner, E.J., Mascarenhas, J., Bishop, J., Yoo, D.H., Chadburn, A., Crow, M.K., Friedman, S.M. (1996) $CD4^+$ T-cell induction of Fas-mediated apoptosis in Burkitt's lymphoma B cells, *Blood,* **88**: 1375-82,
- Sotomayor, E.M., Borrello, I., Tubb, E., Rattis, F.M., Bien, H., Lu, Z., Fein, S., Schoenberger, S., Levitsky, H.I. (1999) Conversion of tumor-specific CD4+ T-cell tolerance to T-cell priming through *in vivo* ligation of CD40. *Nat Med.*, **5:** 780-7,
- Tong, A. W., B. Seamour, J. Chen, D. Su, G. Ordonez, L. Frase, G. Netto, and M. J. Stone. 2000. CD40 ligand-induced apoptosis is Fas-independent in human multiple myeloma cells. *Leuk Lymphoma* **36**:543-558.


## Revendications

**1.** Molécule multimérique, **caractérisée en ce qu'**elle répond à la formule générale suivante :

$$A\text{-}X_n$$

dans laquelle :

- n est égal à 3, 4, 5 ou 6,
- A est un groupe chimique, fonctionnalisé par au moins trois fonctions amines ou fonctions COOH ou fonctions

SH ou fonctions S-Npys (S-nitro-pyridinesulfényle) ou fonctions S-Pys (S-pyridinesulfényle), et est notamment différent d'une protéine,

- X représente un groupe -D, -B-D ou -B(D)-D', dans lequel :

  * B est un bras espaceur,
  * -D et -D' représentent des peptides ou pseudopeptides correspondant à une séquence dérivée d'un ligand, choisie parmi les résidus formant l'interface avec le récepteur du ligand, laquelle séquence est susceptible d'interagir avec le récepteur, ledit ligand étant choisi parmi les ligands de récepteurs de la superfamille du TNF, et notamment parmi les ligands suivants : EDA, CD40L, FasL, OX40L, AITRL, CD30L, VEGI, LIGHT, 4-1BBL, CD27L, LTα, TNF, LTβ, TWEAK, APRIL, BLYS, RANKL et TRAIL,

**caractérisée en ce que** :

• soit A est un radical branché de symétrie $C_3$ de formule générale suivante :

$$Y\left[\left(CH_2\right)_m Z \left(CH_2\right)_{m'} V\right]_3$$

dans laquelle :

  * m et m' sont des nombres entiers compris de 1 à 5,
  * V représente un groupe -NH- ou -CO- formant une liaison amide avec X,
  * Z représente un atome d'oxygène ou un groupe $CH_2$,
  * Y représente soit un atome d'azote, soit un groupe R-C- soit un groupe R-CONH-C-, dans lesquels R peut être un groupe alkyle avec 1 à 10 atomes de carbone, un groupe alkényle avec 1 à 10 atomes de carbone, un groupe alkynyle avec 1 à 10 atomes de carbone, un groupe aryle avec 5 à 12 atomes de carbone, un groupe aralkyle avec 5 à 14 atomes de carbone ou un groupe hétéroaryle avec 1 à 10 atomes de carbone, lesdits groupes pouvant être non substitués ou substitués par 1 jusqu'à 6 substituants choisis parmi les groupes -COOH, $-NH_2$, $-CONH_2$ ou alkoxy,

• soit A est un radical $C_3$ cyclique répondant à l'une des formules générales suivantes :

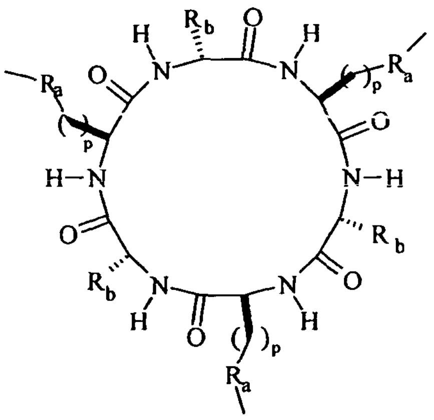

Ia

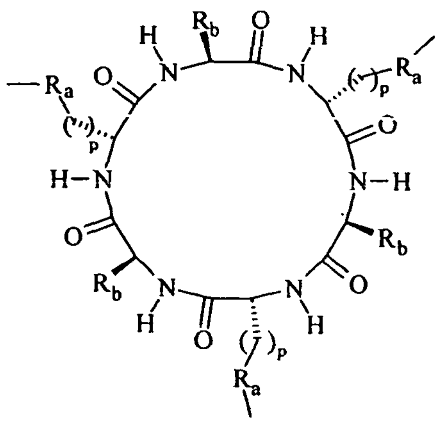

Ib

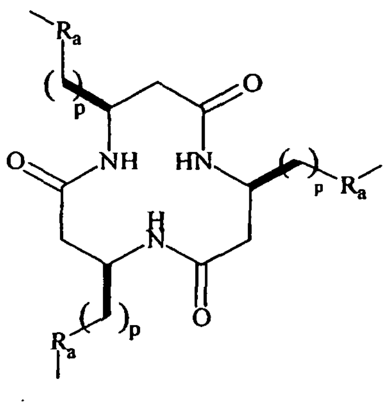

II

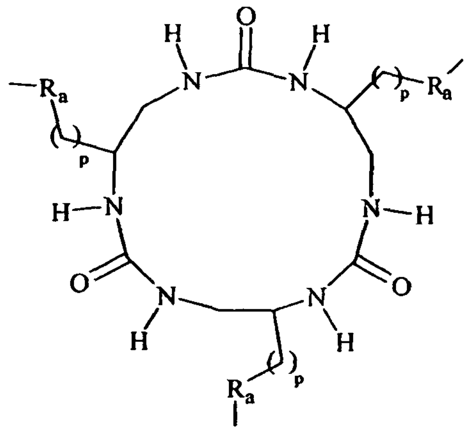

III

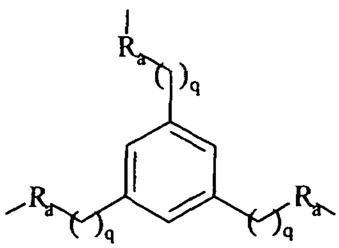

IV

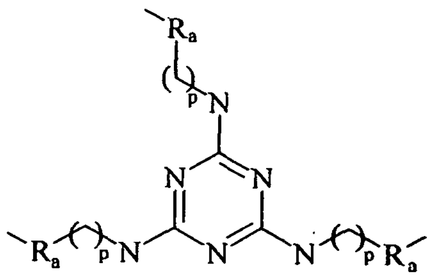

V

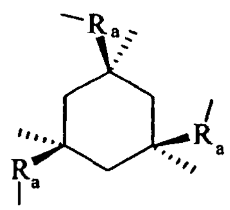

VI

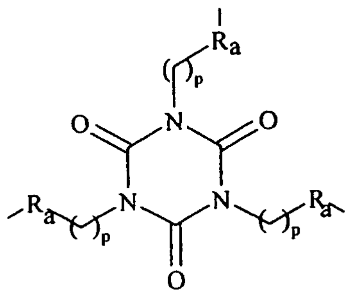

VIa

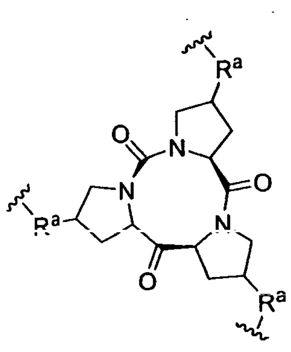

VIb

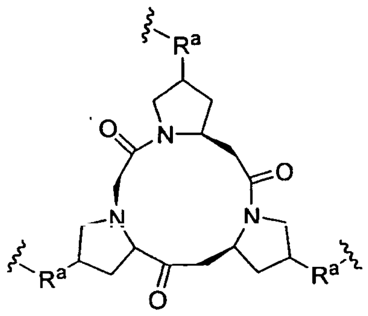

VIc

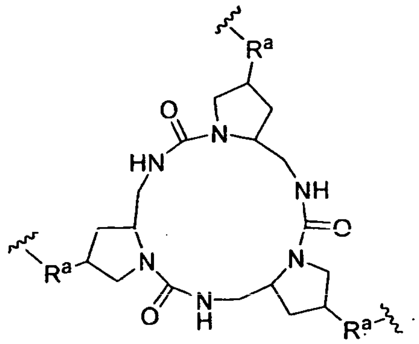

VId

dans lesquelles :

* $R_a$ représente soit un groupe -NH- soit un groupe -CO- formant une liaison amide avec X,
* $R_b$ représente la chaîne latérale d'un acide aminé protéinogénique,
* p est un nombre entier compris de 1 à 4,
* q est un nombre entier compris de 0 à 4,

• soit A est un radical branché non symétrique répondant aux formules générales suivantes :

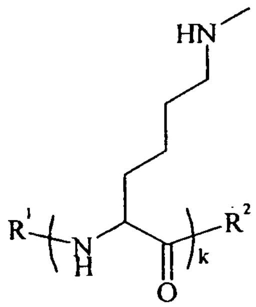

VII

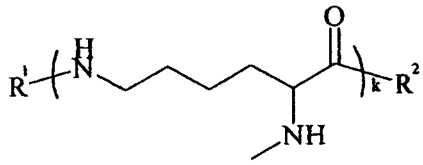

VIII

dans lesquelles :

* k représente 3, 4, 5 ou 6,
* $R^1$ représente soit un atome d'hydrogène, soit un résidu d'acide aminé choisi parmi les acides aminés protéinogéniques, soit un groupement RCO-, ROCO- ou RNHCO-, R étant tel que défini ci-dessus,
* $R^2$ représente soit un groupe $-NH_2$, soit un groupe -NHR, soit un résidu d'acide aminé choisi parmi les acides aminés protéinogéniques, R étant tel que défini ci-dessus.

2. Molécule multimérique selon la revendication 1, **caractérisée en ce que** B répond à l'une des formules générales suivantes :

$$-R^3-Y-R^4$$ ou 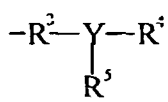

dans lesquelles :

* Y représente une chaîne alkyle $C_1$-$C_{10}$ ou un groupement alkynyle ou alkényle ou aryle ou aralkyle ou hété-

roaryle,

* $R^3$ représente soit un groupe -NH- lorsque V ou $R_a$ est un groupe -CO-, soit un groupe -CO- lorsque V ou $R_a$ est un groupe -NH-,

* $R^4$ et $R^5$ représentent indépendamment l'un de l'autre un groupe -CO- ou un groupe -NH-.

**3.** Molécule selon la revendication 1 ou 2, **caractérisée en ce que** -D et -D' représentent des peptides dérivés du ligand du récepteur CD40 humain ou murin (CD40L), lesdits peptides appartenant à la séquence primaire du ligand CD40L de CD40 et dont le nombre d'acides aminés est compris entre 3 et 10.

**4.** Molécule selon l'une quelconque des revendications 1 à 3, **caractérisée en ce que** les peptides dérivés du ligand du récepteur CD40 humain ou murin (CD40L) sont choisis parmi les suivants :

$Lys^{143}$-Gly-$Tyr^{145}$ (SEQ ID NO : 1), $Tyr^{145}$-Gly-$Lys^{143}$ (SEQ ID NO : 2),
$Lys^{143}$-Gly-Tyr-$Tyr^{146}$ (SEQ ID NO : 3), $Tyr^{146}$-Tyr-Gly-$Lys^{143}$ (SEQ ID NO : 4),
Lys-Pro-Arg (SEQ ID NO : 5), Lys-ψ($CH_2$NH)Pro-Arg,
$Arg^{200}$-Phe-Glu-Arg-Ile-Leu-Leu-$Arg^{207}$ (SEQ ID NO : 6),
$Arg^{207}$-Leu-Leu-Ile-Arg-Glu-Phe-$Arg^{200}$ (SEQ ID NO : 7),
$Arg^{200}$-Phe-Glu-Arg-$Ile^{204}$ (SEQ ID NO : 8), $Ile^{204}$-Arg-Glu-Phe-$Arg^{200}$ (SEQ ID NO : 9),
$Arg^{203}$-Ile-Leu-Leu-$Arg^{207}$ (SEQ ID NO : 10), $Arg^{207}$-Leu-Leu-Ile-$Arg^{203}$ (SEQ ID NO : 11),
$Cys^{218}$-Gly-Gln-Gln-Ser-$Ile^{223}$ (SEQ ID NO : 12), $Ile^{223}$-Ser-Gln-Gln-Gly-$Cys^{218}$ (SEQ ID NO : 13),
$Gly^{200}$-Ser-Glu-Arg-Ile-Leu-Leu-$Lys^{207}$ (SEQ ID NO : 14),
$Lys^{207}$-Leu-Leu-Ile-Arg-Glu-Ser-$Gly^{200}$ (SEQ ID NO : 15),
$Gly^{200}$-Ser-Glu-Arg-$Ile^{204}$ (SEQ ID NO : 16), $Ile^{204}$-Arg-Glu-Ser-$Gly^{200}$ (SEQ ID NO : 17),
$Arg^{203}$-Ile-Leu-Leu-$Lys^{207}$ (SEQ ID NO : 18), $Lys^{207}$-Leu-Leu-Ile-$Arg^{203}$ (SEQ ID NO : 19),
$Cys^{218}$-Glu-Gln-Gln-Ser-$Val^{223}$ (SEQ ID NO : 20),
$Val^{223}$-Ser- Gln- Gln- Glu- $Cys^{218}$ (SEQ ID NO : 21),

ou parmi des peptides hybrides constitués d'au moins deux acides aminés consécutifs de deux des séquences définies ci-dessus, notamment les peptides de séquences $Arg^{203}$-$Ile^{204}$-$Tyr^{145}$-$Tyr^{146}$ (SEQ ID NO : 22) ou $Arg^{203}$-$Ile^{204}$-$Tyr^{146}$-$Tyr^{145}$-$Gly^{144}$-$Lys^{143}$ (SEQ ID NO : 23),
ou parmi des fragments des séquences susmentionnées,
les acides aminés pouvant être indifféremment de configuration L ou D.

**5.** Molécule multimérique selon l'une des revendications 1 à 4, **caractérisée en ce que** A présente une symétrie $C_3$.

**6.** Molécule selon l'une des revendications 1 à 5, **caractérisée en ce que** A répond à l'une des formules suivantes :

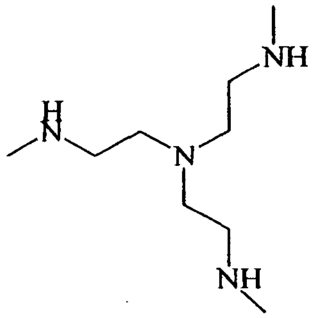
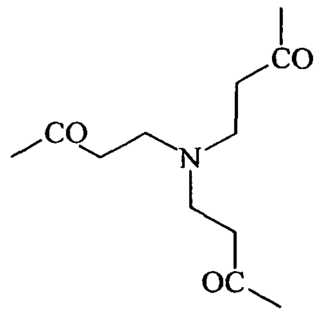
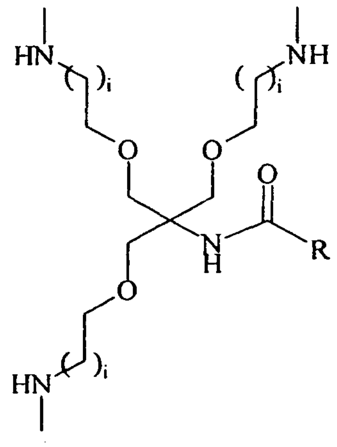
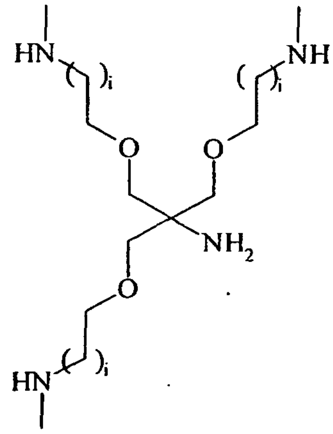

dans lesquelles i représente un nombre entier supérieur ou égal à 1.

**7.** Molécule selon l'une des revendications 1 à 6, de formule suivante :

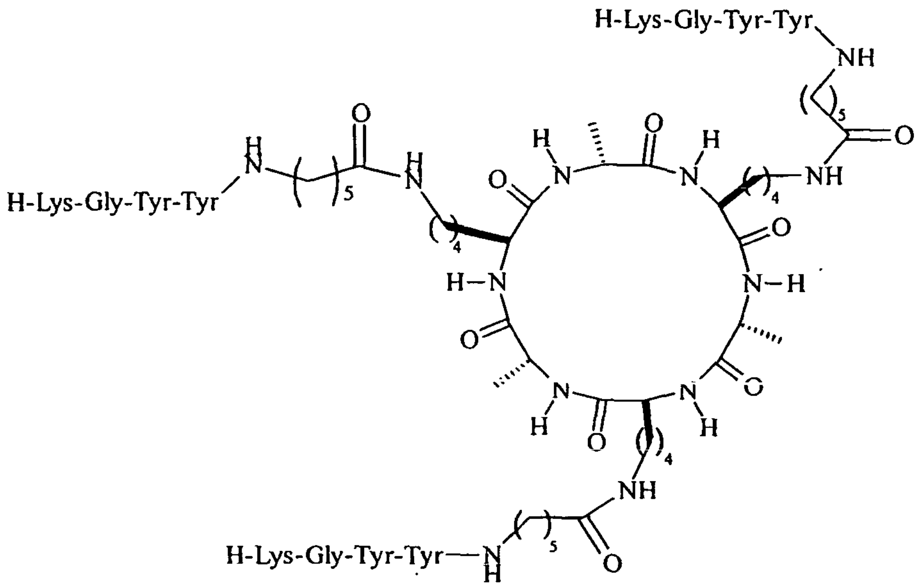

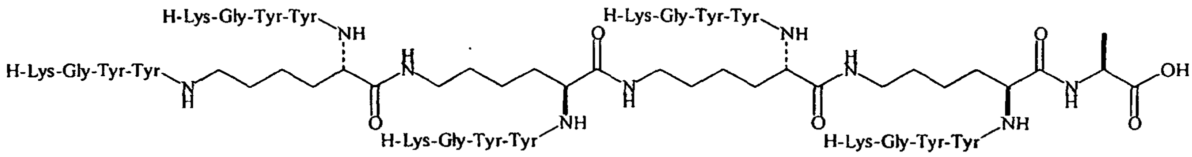

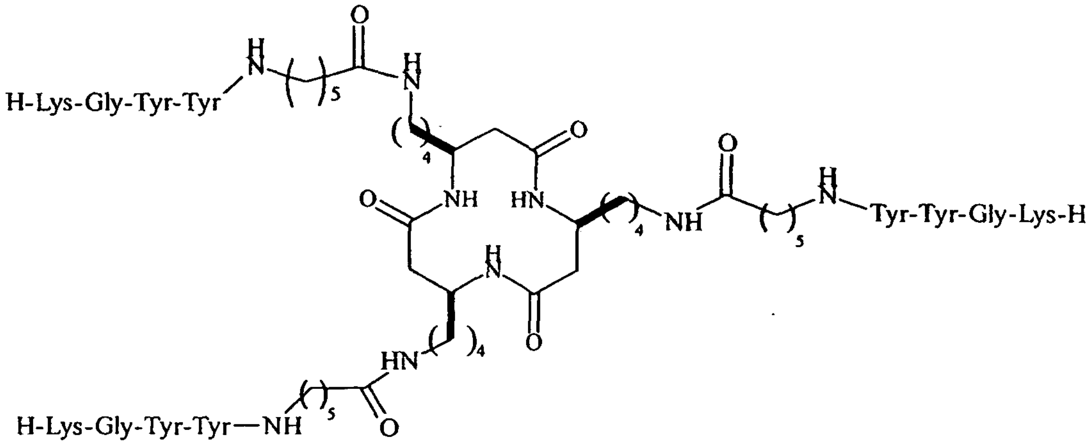

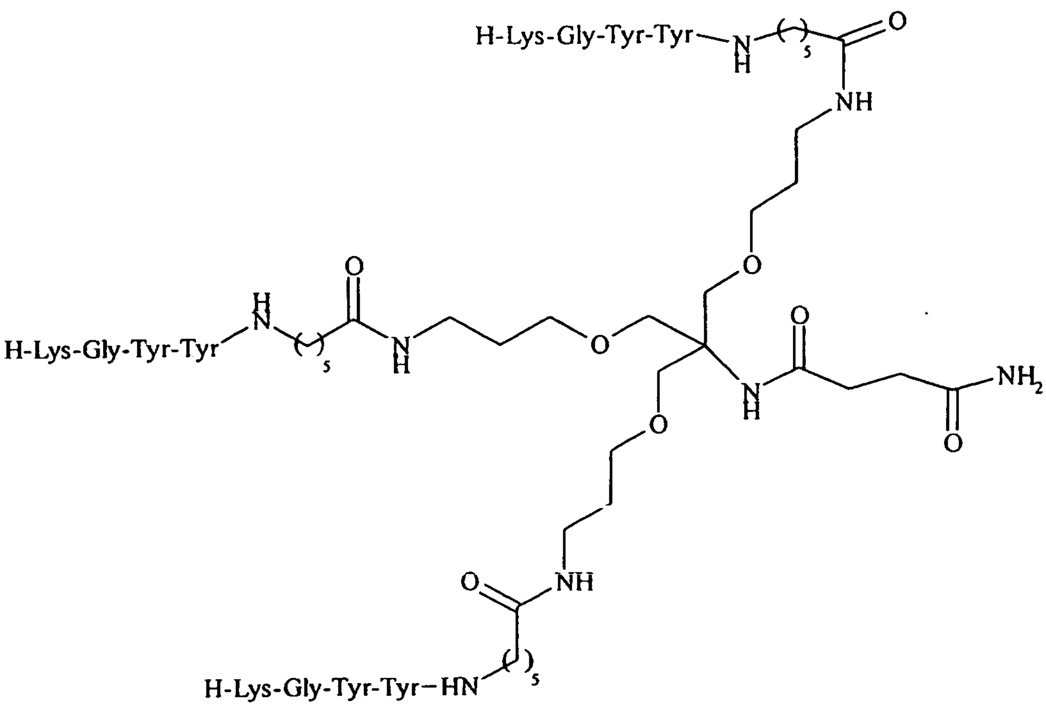

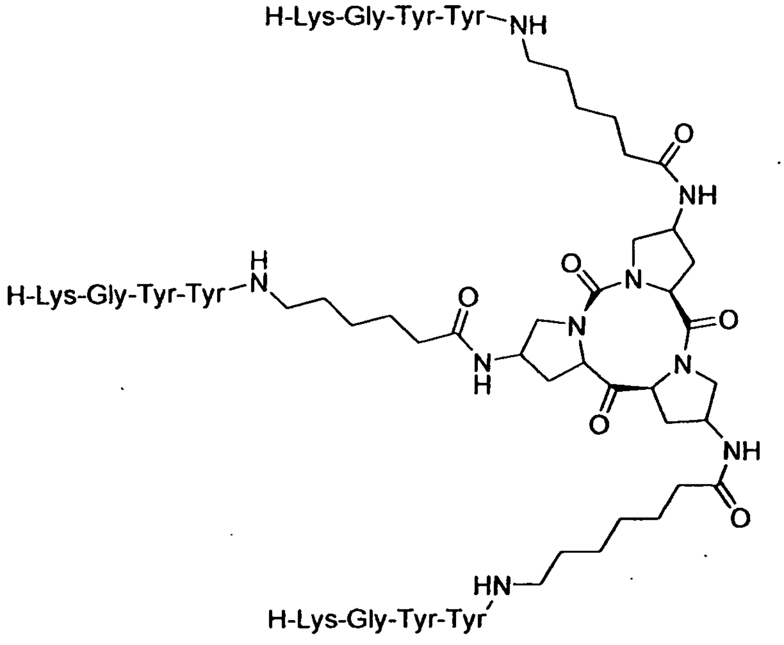

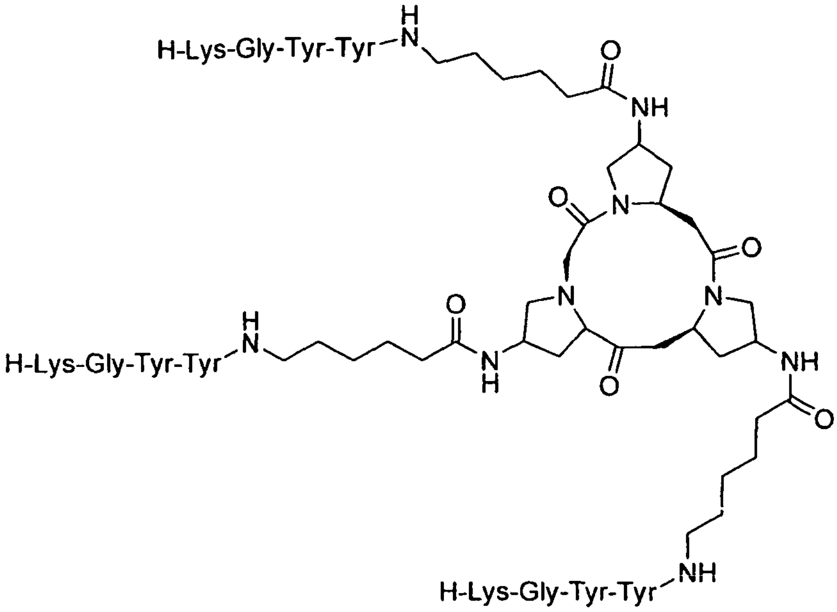

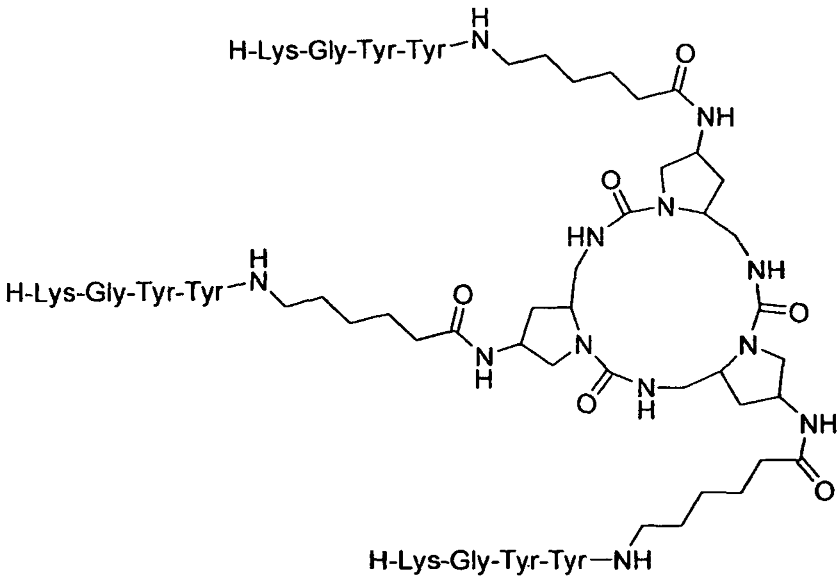

8. Composition pharmaceutique **caractérisée en ce qu'**elle comprend, à titre de substance active une molécule multimérique selon l'une des revendications 1 à 7, en association avec un vecteur pharmaceutiquement acceptable.

9. Composition vaccinale, **caractérisée en ce qu'**elle comprend, à titre de substance active une molécule multimérique selon l'une des revendications 1 à 7, en association avec un adjuvant pharmaceutiquement acceptable.

10. Utilisation de molécules multimériques selon l'une des revendications 1 à 7, pour la préparation d'un médicament destiné au traitement de pathologies impliquant l'inhibition ou l'activation de la réponse immunitaire.

11. Utilisation selon la revendication 10, pour la préparation d'un médicament destiné au traitement de pathologies impliquant l'inhibition de la réponse immunitaire, telles que les rejets de greffes ou les maladies auto-immunes.

12. Utilisation selon la revendication 10, pour la préparation d'un médicament destiné au traitement de pathologies impliquant l'augmentation de la réponse immunitaire, telles que les cancers ou les infections parasitaires, bactériennes ou virales.

13. Procédé de préparation sur support solide d'une molécule multimérique, selon l'une des revendications 1 à 7, dans laquelle A est un radical $C_3$ cyclique et répond à l'une des formules Ia, Ib, II, VIb, VIc ou VId telles que définies dans la revendication 5, ledit procédé étant **caractérisé en ce qu'**il comprend les étapes suivantes :

- la formation d'un précurseur linéaire de A, lequel précurseur est constitué d'un enchaînement d'acides aminés formant une chaîne peptidique en croissance, synthétisée par des cycles successifs de couplage entre des résidus d'acides aminés N-protégés, dont trois portent un groupe $R_a$ de type amine, et la fonction amine de la chaîne peptidique en croissance, et de déprotection, le premier résidu d'acide aminé étant accroché sur un support solide,
- la cyclisation du précurseur linéaire de A protégé susmentionné,
- le clivage des susdits groupes protecteurs, pour libérer les susdites fonctions amines protégées,
- le couplage des trois fonctions amines libérées avec un bras espaceur B N-protégé,
- la déprotection du bras espaceur B et le couplage des fonctions amines libérées du bras espaceur B, avec un peptide D déjà formé ou formé in situ par l'assemblage séquentiel des résidus d'acides aminés correspondant au peptide D, et
- le clivage de la molécule du support solide, après la suppression de tous les groupements protecteurs présents sur les chaînes latérales fonctionnalisées du peptide D, afin d'obtenir la molécule multimérique telle que définie dans l'une des revendications 1 à 7.

14. Procédé de préparation en solution d'une molécule multimérique, selon l'une des revendications 1 à 7, dans laquelle A est un radical $C_3$ cyclique et répond à l'une des formules Ia, Ib, II, VIb, VIc ou VId telles que définies dans la

revendication 5, ledit procédé étant **caractérisé en ce qu'**il comprend les étapes suivantes :

- la formation d'un précurseur linéaire de A, lequel précurseur est constitué d'un enchaînement d'acides aminés formant une chaîne peptidique en croissance, synthétisée par des cycles successifs de couplage entre des résidus d'acides aminés N-protégés, dont trois portent un groupe $R_a$ de type amine, et la fonction amine de la chaîne peptidique en croissance, et de déprotection,
- la cyclisation du précurseur linéaire de A protégé susmentionné,
- le clivage des susdits groupes protecteurs, pour libérer les susdites fonctions amines protégées,
- le couplage des trois fonctions amines libérées avec un peptide -D-B correspondant à un bras espaceur B lié à un peptide D protégé,
- la déprotection des groupements protecteurs présents sur le peptide D, afin d'obtenir la molécule multimérique telle que définie dans l'une des revendications 1 à 7.

**15.** Procédé de préparation d'une molécule multimérique selon l'une des revendications 1 à 7, dans laquelle A est un radical $C_3$ branché et répond à l'une des formules IV, V, VI ou VIa telles que définies dans la revendication 5, ledit procédé étant **caractérisé en ce qu'**il comprend les étapes suivantes :

- le couplage des trois fonctions amines du radical A de formule IV, V, VI ou VIa avec un bras espaceur B protégé,
- la déprotection du bras espaceur B,
- l'assemblage du bras espaceur B déprotégé avec des acides aminés protégés entrant dans la constitution d'un peptide D, par des cycles successifs de couplage, de purification et de déprotection des acides aminés susmentionnés,
- la déprotection du dernier acide aminé entrant dans la constitution du peptide D, afin d'obtenir la molécule multimérique telle que définie dans l'une des revendications 1 à 7.

**16.** Procédé de préparation sur support solide d'une molécule multimérique selon l'une des revendications 1 à 7, dans laquelle A est un radical branché non symétrique répondant à l'une des formules VII ou VIII telles que définies dans la revendication 5, ledit procédé étant **caractérisé en ce qu'**il comprend les étapes suivantes :

- le greffage d'une lysine sur un support solide, chacune des deux fonctions amines de la lysine, respectivement en position $\alpha$ et $\varepsilon$, étant protégée respectivement par des groupes protecteurs différents et orthogonaux,
- l'allongement de la chaîne peptidique formée à partir de la lysine, à la longueur désirée, par couplages et déprotections successifs

    * soit uniquement des fonctions amines en position $\alpha$ afin d'obtenir le radical A de formule VII, avec des fonctions amines protégées en position $\varepsilon$,
    * soit uniquement des fonctions amines en position $\varepsilon$ afin d'obtenir le radical A de formule VIII, avec des fonctions amines protégées en position $\alpha$,

- le couplage des fonctions amines déprotégées en position $\varepsilon$ dans le radical A de formule VII ou en position $\alpha$ dans le radical A de formule VIII, avec un bras B protégé,
- l'assemblage du bras espaceur B déprotégé avec un peptide D déjà formé ou formé in situ par l'assemblage séquentiel des résidus d'acides aminés correspondant au peptide D, et
- le clivage de la molécule ainsi obtenue du support solide, après la suppression de tous les groupements protecteurs présents sur les chaînes latérales fonctionnalisées du peptide D.

## Claims

**1.** A multimeric molecule, **characterized in that** it corresponds to the following general formula:

$$A\text{-}X_n$$

in which:

- n is equal to 3, 4, 5 or 6,
- A is a chemical group, functionalized by at least three amino functions or COOH functions or SH functions or S-Npys (S-nitro-pyridinesulphenyl) functions or S-Pys (S-pyridinesulphenyl) functions, and is in particular different from a protein,

- X represents a -D, -B-D or -B(D)-D' group , in which:

  * B is a spacer arm,
  * -D and -D' represent peptides or pseudopeptides corresponding to a sequence derived from a ligand, chosen from the residues forming the interface with the ligand receptor, which sequence is capable of interacting with the receptor, said ligand being chosen from the ligands of receptors of the TNF superfamily, and in particular from the following ligands: EDA, CD40L, FasL, OX40L, AITRL, CD30L, VEGI, LIGHT, 4-1BBL, CD27L, LTα, TNF, LTβ, TWEAK, APRIL, BLYS, RANKL and TRAIL,

**characterized in that**:

• either A is a branched radical with $C_3$ symmetry with the following general formula:

$$Y\left[\left(CH_2\right)_m - Z - \left(CH_2\right)_{m'} - V - \right]_3$$

in which:

  * m and m' are integers comprised from 1 to 5,
  * V represents an -NH- or -CO- group forming an amide bond with X,
  * Z represents an oxygen atom or a $CH_2$ group,
  * Y represents either a nitrogen atom, or an R-C- group or an R-CONH-C-group, in which R can be an alkyl group with 1 to 10 carbon atoms, an alkenyl group with 1 to 10 carbon atoms, an alkynyl group with 1 to 10 carbon atoms, an aryl group with 5 to 12 carbon atoms, an aralkyl group with 5 to 14 carbon atoms or a heteroaryl group with 1 to 10 carbon atoms, said groups are capable of being non-substituted or substituted by 1 to 6 substituents chosen from the -COOH, $-NH_2$, $-CONH_2$ or alkoxy groups,

• or A is a cyclic $C_3$ radical corresponding to one of the following general formulae:

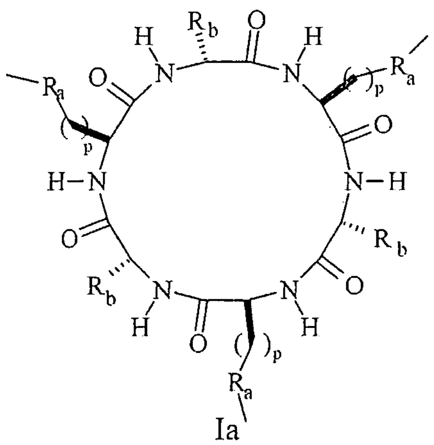

Ia

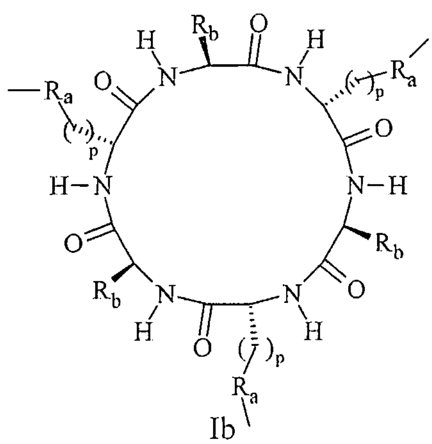

Ib

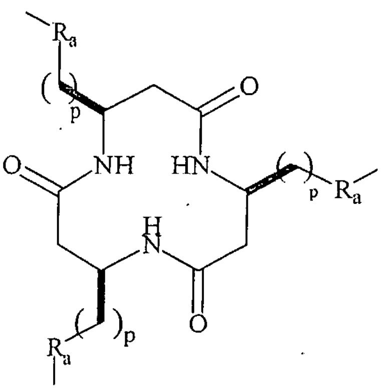

II

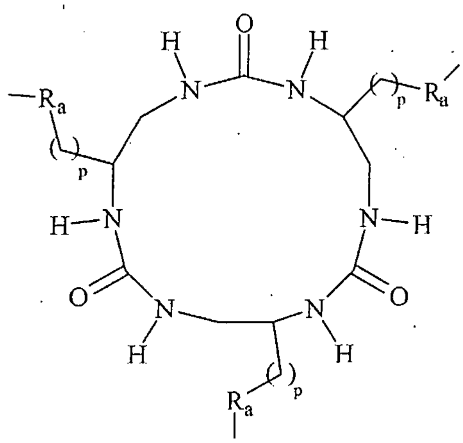

III

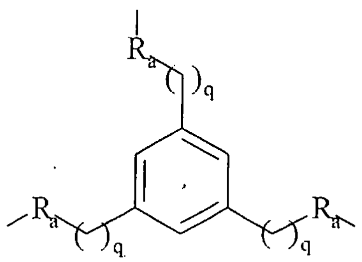

IV

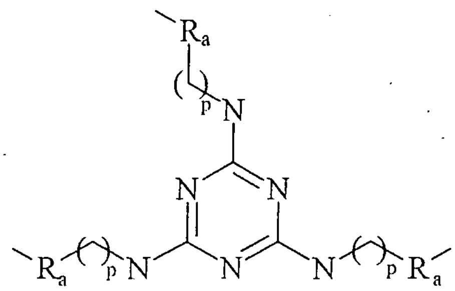

V

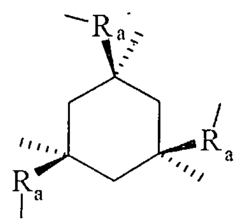

VI

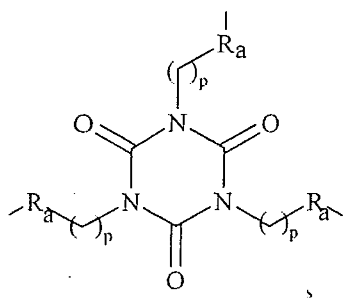

VIa

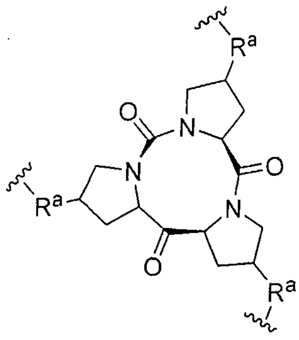

VIb

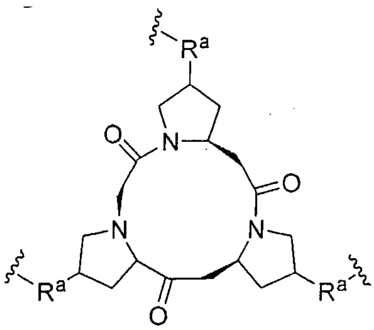

VIc

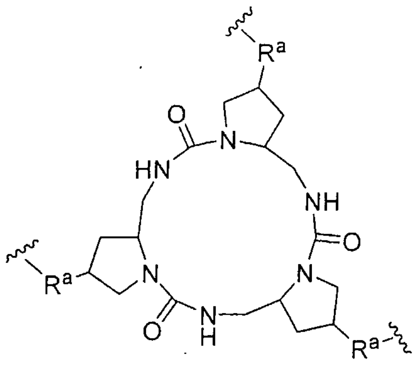

VId

in which:

* $R_a$ represents either an -NH- group or a -CO- group forming an amide bond with X,
* $R_b$ represents the side chain of a proteinogenic amino acid,
* p is an integer comprised from 1 to 4,
* q is an integer comprised from 0 to 4,

• or A is a non-symmetrical branched radical corresponding to the following general formulae:

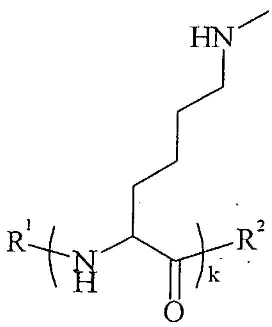

VII

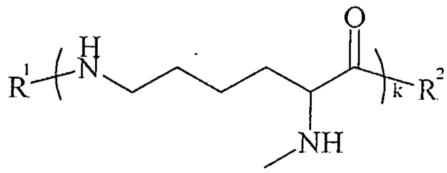

VIII

in which:

* k represents 3, 4, 5 or 6,
* $R^1$ represents either a hydrogen atom, or an amino acid residue chosen from the proteinogenic amino acids, or an RCO-, ROCO- or RNHCO-group, R being as defined above,
* $R^2$ represents either an $-NH_2$ group, or an -NHR group, or an amino acid residue chosen from the proteinogenic amino acids, R being as defined above.

**2.** The multimeric olecule according to claim 1, **characterized in that** B corresponds to one of the following general formulae:

$-R^3-Y-R^4$ or 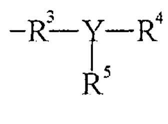

in which:

* Y represents a $C_1$-$C_{10}$ alkyl chain or an alkynyl or alkenyl or aryl or aralkyl or heteroaryl group,
* $R^3$ represents either an -NH- group when V or $R_a$ is a -CO- group, or a -CO-group when V or $R_a$ is an -NH- group,

* $R^4$ and $R^5$ represent independently of one another a -CO- group or an -NH-group,

3. The molecule according to claim 1 or 2, **characterized in that** -D and -D' represent peptides derived from the ligand of the human or murine CD40 receptor (CD40L), said peptides belonging to the primary sequence of the CD40L ligand of CD40 and the number of amino acids of which is comprised between 3 and 10.

4. The molecule according to any one of claims 1 to 3, **characterized in that** the peptides derived from the ligand of the human or murine CD40 receptor (CD40L) are chosen from the following:

   Lys$^{143}$-Gly-Tyr$^{145}$ (SEQ ID NO : 1), Tyr$^{145}$-Gly-Lys$^{143}$ (SEQ ID NO : 2),
   Lys$^{143}$-Gly-Tyr-Tyr$^{146}$ (SEQ ID NO : 3), Tyr$^{146}$-Tyr-Gly-Lys$^{143}$ (SEQ ID NO : 4),
   Lys-Pro-Arg (SEQ ID NO : 5), Lys-$\psi$($CH_2$NH)Pro-Arg,
   Arg$^{200}$-Phe-Glu-Arg-Ile-Leu-Leu-Arg$^{207}$ (SEQ ID NO : 6),
   Arg$^{207}$-Leu-Leu-Ile-Arg-Glu-Phe-Arg$^{200}$ (SEQ ID NO : 7),
   Arg$^{200}$-Phe-Glu-Arg-Ile$^{204}$ (SEQ ID NO : 8), Ile$^{204}$-Arg-Glu-Phe-Arg$^{200}$ (SEQ ID NO : 9),
   Arg$^{203}$-Ile-Leu-Leu-Arg$^{207}$ (SEQ ID NO : 10), Arg$^{207}$-Leu-Leu-Ile-Arg$^{203}$ (SEQ ID NO : 11),
   Cys$^{218}$-Gly-Gln-Gln-Ser-Ile$^{223}$ (SEQ ID NO : 12), Ile$^{223}$-Ser-Gln-Gln-Gly-Cys$^{218}$ (SEQ ID NO: 13),
   Gly$^{200}$-Ser-Glu-Arg-Ile-Leu-Leu-Lys$^{207}$ (SEQ ID NO : 14),
   Lys$^{207}$-Leu-Leu-Ile-Arg-Glu-Ser-Gly$^{200}$ (SEQ ID NO : 15),
   Gly$^{200}$-Ser-Glu-Arg-Ile$^{204}$ (SEQ ID NO: 16), Ile$^{204}$-Arg-Glu-Ser-Gly$^{200}$ (SEQ ID NO : 17),
   Arg$^{203}$-Ile-Leu-Leu-Lys$^{207}$ (SEQ ID NO : 18), Lys$^{207}$-Leu-Leu-Ile-Arg$^{203}$ (SEQ ID NO : 19),
   Cys$^{218}$-Glu-Gln-Gln-Ser-Val$^{223}$ (SEQ ID NO : 20),
   Val$^{223}$-Ser-Gln-Gln-Glu-Cys$^{218}$ (SEQ ID NO : 21),

   or from hybrid peptides constituted by at least two consecutive amino acids of two of the sequences defined above, in particular the peptides with the sequences Arg$^{203}$-Ile$^{204}$-Tyr$^{145}$-Tyr$^{146}$ (SEQ ID NO : 22) or Arg$^{203}$-Ile$^{204}$-Tyr$^{146}$-Tyr$^{145}$-Gly$^{144}$-Lys$^{143}$ (SEQ ID NO : 23),
   or from fragments of the abovementioned sequences,
   the amino acids being equally able to be of L or D-configuration.

5. The multimeric molecule according to one of claims 1 to 4, **characterized in that** A has a $C_3$ symmetry.

6. The molecule according to one of claims 1 to 5, **characterized in that** A corresponds to one of the following formulae:

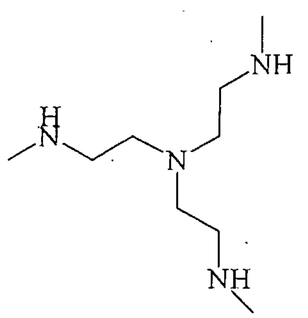

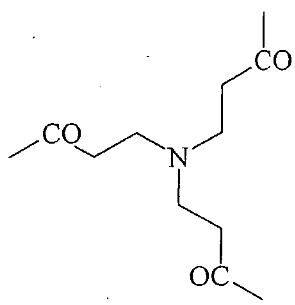

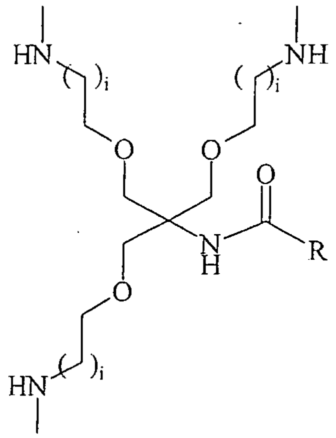

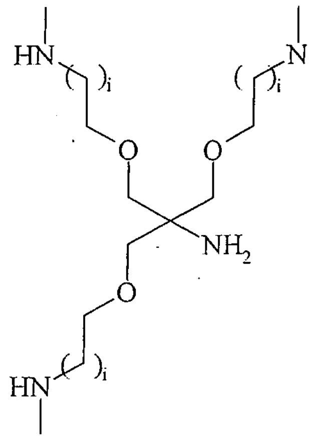

in which i represents an integer greater than or equal to 1.

**7.** The molecule according to one of claims 1 to 6, of the following formula:

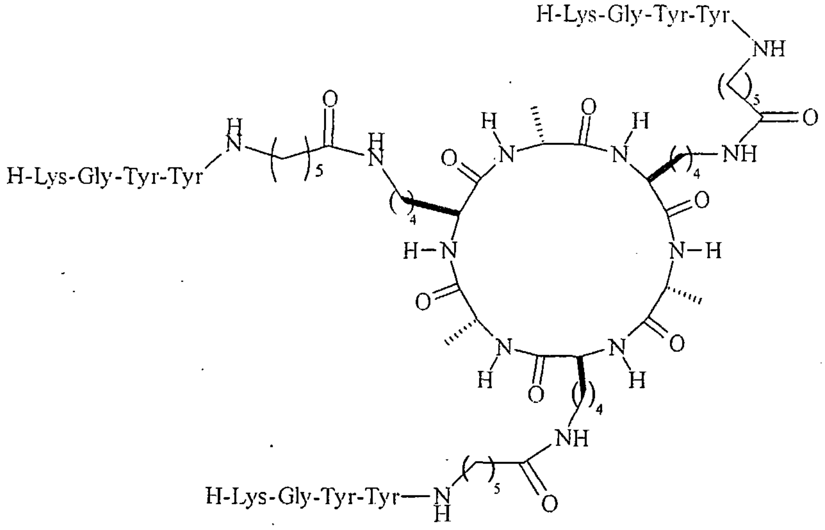

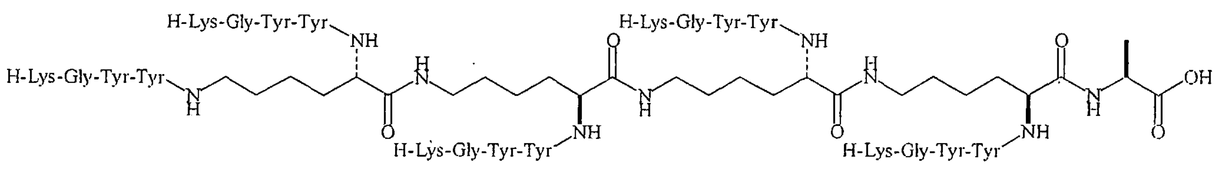

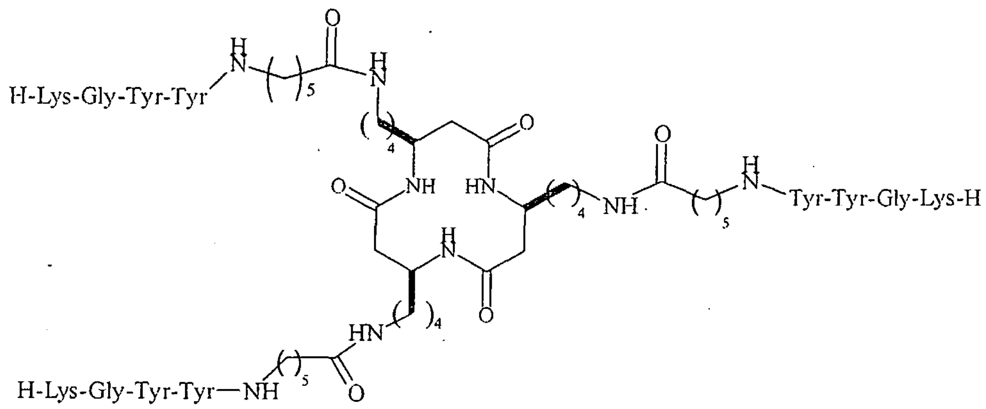

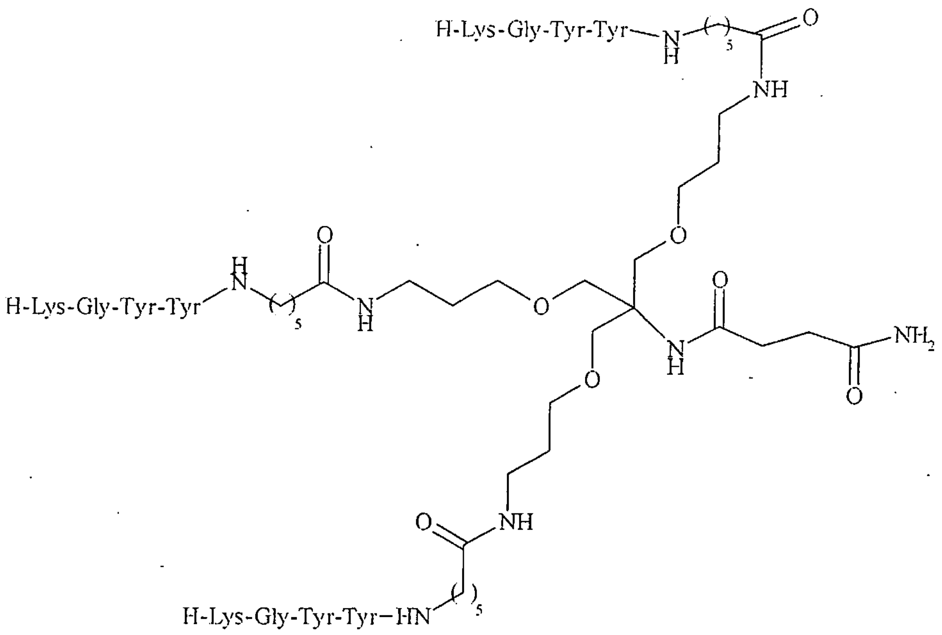

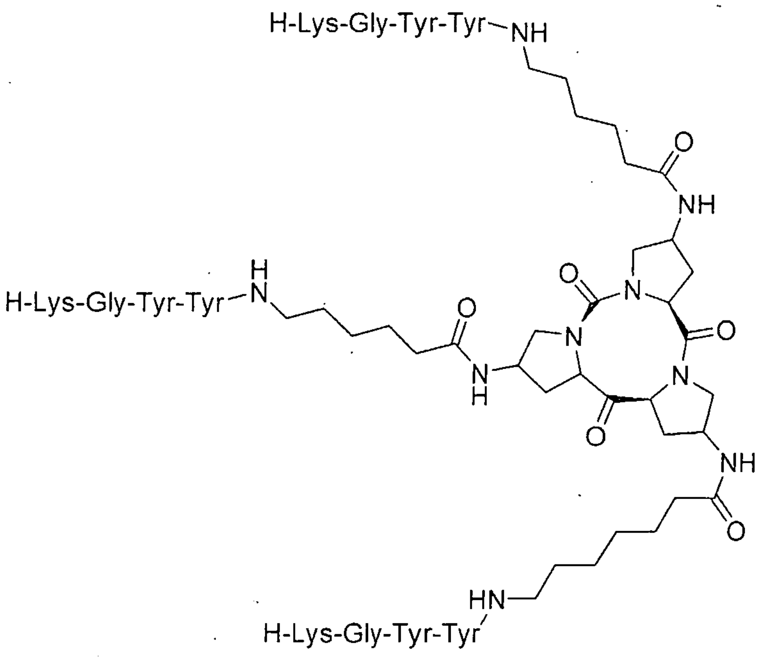

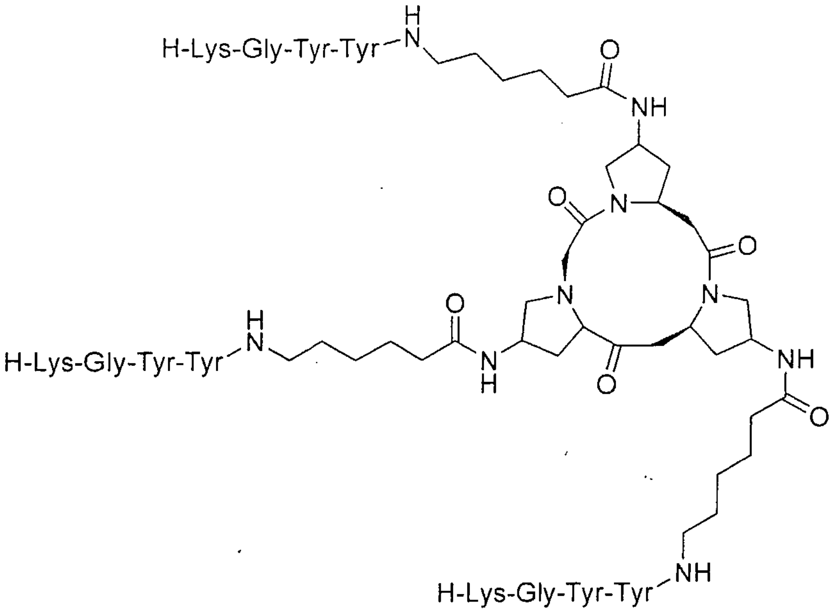

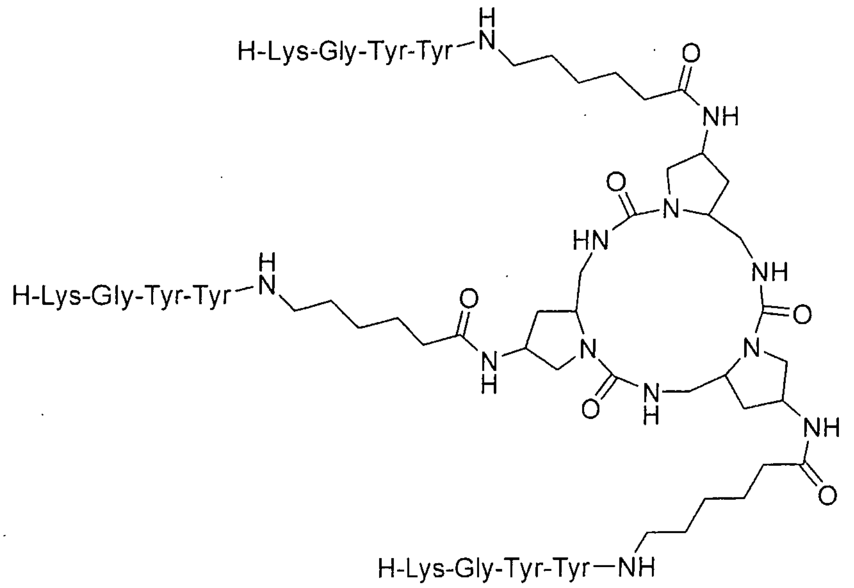

**8.** A pharmaceutical composition **characterized in that** it comprises, as active ingredient, a multimeric molecule according to one of claims 1 to 7, in combination with a pharmaceutically acceptable vector.

**9.** A vaccinal composition, **characterized in that** it comprises, as active ingredient, a multimeric molecule according to one of claims 1 to 7, in combination with a pharmaceutically acceptable adjuvant.

**10.** The use of multimeric molecules according to one of claims 1 to 7, for the preparation of a medicament intended for the treatment of pathologies involving the inhibition or the activation of the immune response.

**11.** The use according to claim 10, for the preparation of a medicament intended for the treatment of pathologies involving inhibition of the immune response, such as the rejection of grafts or auto-immune diseases.

**12.** The use according to claim 10, for the preparation of a medicament intended for the treatment of pathologies involving the increase of immune response, such as cancers or parasitic, bacterial or viral infections.

**13.** A process for the preparation on a solid support of a multimeric molecule, according to one of claims 1 to 7, in which A is a cyclic $C_3$ radical and corresponds to one of formulae Ia, Ib, II, VIb, VIc or VId as defined in claim 5, said process being **characterized in that** it comprises the following stages:

- the formation of a linear precursor of A, which precursor is constituted by an amino acid sequence forming a growing peptide chain, synthesized by successive coupling cycles between residues of N-protected amino acids, three of which carry an $R_a$ group of amine type, and the amine function of the growing peptide chain, and deprotection, the first amino acid residue being attached to a solid support,
- the cyclization of the abovementioned protected linear precursor of A,
- the cleavage of said protective groups, in order to release said protected amine functions,
- the coupling of the three released amine functions with an N-protected spacer arm B,
- the deprotection of the spacer arm B and the coupling of the amine functions released from the spacer arm B, with a D peptide already formed or formed in situ by the sequential assembly of the amino acid residues corresponding to the D peptide, and
- the cleavage of the molecule from the solid support, after the deletion of all the protective groups present on the functionalized side chains of the D peptide, in order to obtain the multimeric molecule as defined in one of claims 1 to 7.

**14.** A process for the preparation in solution of a multimeric molecule, according to one of claims 1 to 7, in which A is a cyclic $C_3$ radical and corresponds to one of formulae Ia, Ib, II, VIb, VIc or VId as defined in claim 5, said process being **characterized in that** it comprises the following stages:

- the formation of a linear precursor of A, which precursor is constituted by an amino acid sequence forming a growing peptide chain, synthesized by successive coupling cycles between N-protected amino acid residues, three of which carry an amine-type $R_a$ group, and the amine function of the growing peptide chain, and deprotection,
- the cyclization of the abovementioned protected linear precursor of A,
- the cleavage of said protective groups, in order to release said protected amine functions,
- the coupling of the three released amine functions with a -D-B peptide corresponding to a spacer arm B linked to a protected D peptide,
- the deprotection of the protective groups present on the D peptide, in order to obtain the multimeric molecule as defined in one of claims 1 to 7.

**15.** A process for the preparation of a multimeric molecule according to one of claims 1 to 7, in which A is a branched $C_3$ radical and corresponds to one of formulae IV, V, VI or VIa as defined in claim 5, said process being **characterized in that** it comprises the following stages:

- the coupling of the three amine functions of the radical A of formula IV, V, VI or VIa with a protected spacer arm B,
- the deprotection of the spacer arm B,
- the assembly of the deprotected spacer arm B with protected amino acids involved in the constitution of a D peptide, by successive cycles of coupling, purification and deprotection of the abovementioned amino acids,
- the deprotection of the last amino acid involved in the constitution of the D peptide, in order to obtain the multimeric molecule as defined in one of claims 1 to 7.

**16.** A process for the preparation on a solid support of a multimeric molecule according to one of claims 1 to 7, in which A is a non-symmetrical branched radical corresponding to one of formulae VII or VIII as defined in claim 5, said process being **characterized in that** it comprises the following stages:

- the grafting of a lysine onto a solid support, each of the two amino functions of the lysine, in positions $\alpha$ and $\varepsilon$ respectively, being protected by different and orthogonal protective groups respectively,
- the extension of the peptide chain formed from the lysine, to the desired length, with successive couplings and deprotections

  * either of the amine functions in position $\alpha$ only, in order to obtain the radical A of formula VII, with protected amine functions in position $\varepsilon$,
  * or of the amine functions in position $\varepsilon$ only, in order to obtain the radical A of formula VIII, with protected amine functions in position $\alpha$,

- the coupling of the deprotected amino functions in position $\varepsilon$ in the radical A of formula VII or in $\alpha$ position in the radical A of formula VIII, with a protected arm B,
- the assembly of the deprotected spacer arm B with a D peptide already formed or formed in situ by the sequential assembly of the amino acid residues corresponding to the D peptide, and
- the cleavage of the molecule thus obtained from the solid support, after the deletion of all the protective groups present on the functionalized side chains of the D peptide.

## Patentansprüche

**1.** Multimeres Molekül, **dadurch gekennzeichnet, dass** es die folgende allgemeine Formel hat:

$$A\text{-}X_n$$

worin

- n gleich 3, 4, 5 oder 6 ist,
- A eine chemische Gruppe ist, die durch mindestens drei Aminfunktionen oder COOH-Funktionen oder SH-Funktiollen oder S-Npys-Funktionen (S-Nitropyridinsulfenyl-Funktionen) oder S-Pys-Funktionen (S-Pyridinsulfenyl-Funktionen) funktionalisiert ist und insbesondere von einem Protein verschieden ist,
- X eine Gruppe -D, -B-D oder -B(D)-D’ darstellt, worin

* B eine Spacer-Gruppe ist,
* -D und -D' Peptide oder Pseudopeptide repräsentieren, die einer von einem Liganden abgeleiteten Sequenz entsprechen, ausgewählt aus den die Grenzfläche mit dem Rezeptor des Liganden bildenden Resten, welche Sequenz zur Wechselwirkung mit dem Rezeptor in der Lage ist, wobei der Ligand ausgewählt ist aus den Liganden von Rezeptoren der TNF-Superfamilie, insbesondere aus den folgenden Liganden: EDA, CD40L, FasL, OX40L, AITRL, CD30L, VEGI, LIGHT, 4-1BBL, CD27L, LTα, TNF, LTβ, TWEAK, APRIL, BLYS, RANKL und TRAIL,

**dadurch gekennzeichnet, dass**

• A ein verzweigter Rest mit $C_3$-Symmetrie und der folgenden allgemeinen Formel ist:

$$Y\left[\left(CH_2\right)_m Z \left(CH_2\right)_{m'} V-\right]_3$$

worin:

* m und m' ganze Zahlen von 1 bis 5 sind,
* V eine Gruppe -NH- oder -CO- ist, die eine Amidbindung mit X bildet,
* Z ein Sauerstoffatom oder eine $CH_2$-Gruppe darstellt,
* Y ein Stickstoffatom, eine R-C- oder R-CONH-C- Gruppe ist, worin R eine Alkylgruppe mit 1 bis 10 Kohlenstoffatomen, eine Alkenylgruppe mit 1 bis 10 Kohlenstoffatomen, eine Alkinylgruppe mit 1 bis 10 Kohlenstoffatomen, eine Arylgruppe mit 5 bis 12 Kohlenstoffatomen, eine Aralkylgruppe mit 5 bis 14 Kohlenstoffatomen oder eine Heteroarylgruppe mit 1 bis 10 Kohlenstoffatomen sein kann, wobei diese Gruppen unsubstituiert oder mit 1 bis 6 Substituenten die aus den Gruppen -COOH, $-NH_2$, $-CONH_2$ oder Alkoxy ausgewählt sind, substituiert sein können,

• oder A ein cyclischer Rest mit C3 gemäß einer der folgenden allgemeinen Formeln ist:

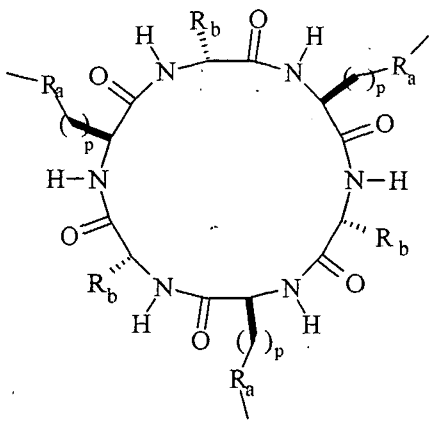

Ia

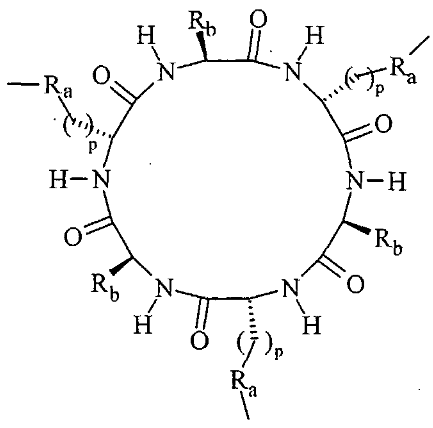

Ib

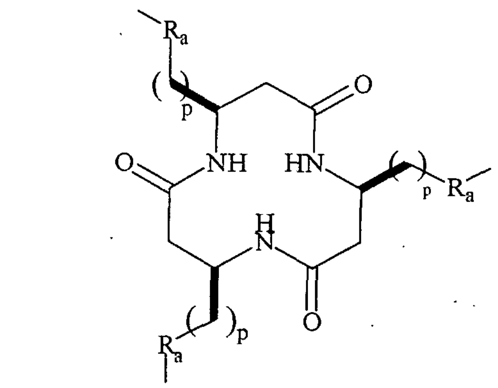

II

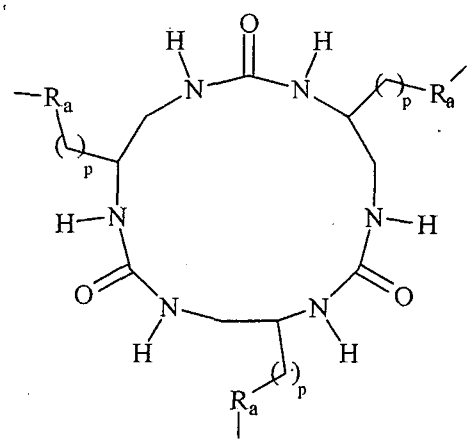

III

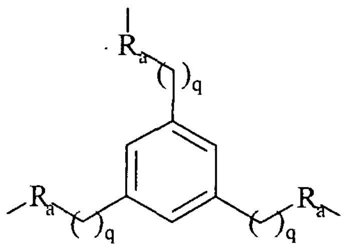

IV

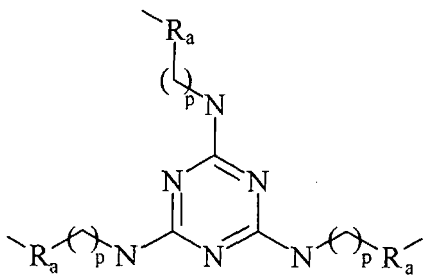

V

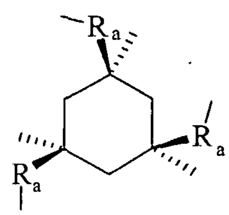

VI

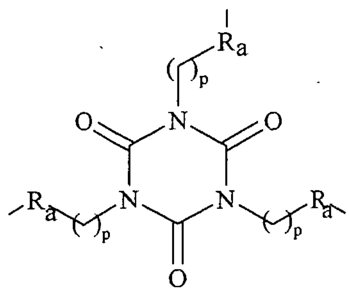

VIa

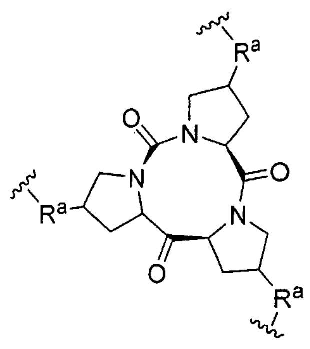

VIb

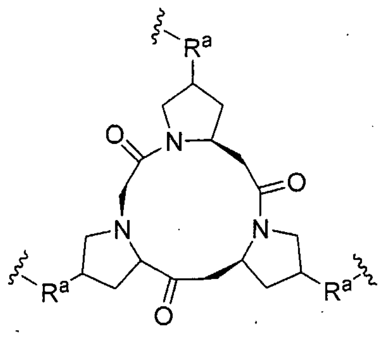

VIc

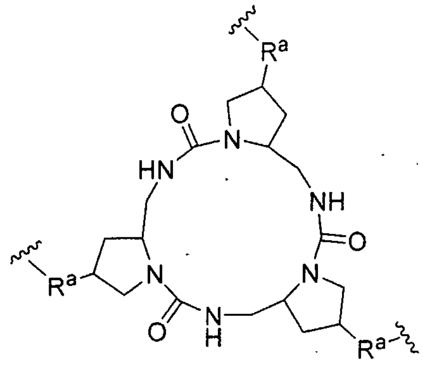

VId

worin

* $R_a$ eine Gruppe -NH- oder -CO- darstellt, die eine Amidbindung mit X bildet,
* $R_b$ eine Seitenkette einer proteinogenen Aminosäure darstellt,
* p eine ganze Zahl von 1 bis 4 ist,
* q eine ganze Zahl von 0 bis 4 ist,

• oder A ein unsymmetrischer verzweigter Rest gemäß den folgenden allgemeinen Formeln ist:

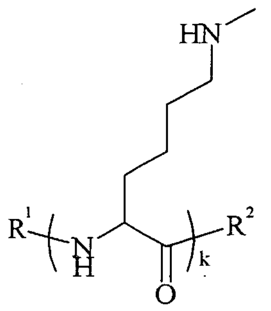

VII

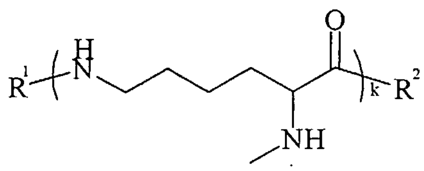

VIII

worin

* k 3, 4, 5 oder 6 ist,
* $R^1$ ein Wasserstoffatom, ein aus den proteinogenen Aminosäuren ausgewählter Amiriosäurerest oder eine Gruppe RCO-, ROCO- oder RNHCO- ist, worin R wie oben definiert ist,
* $R^2$ die $-NH_2$ Gruppe, -NHR Gruppe oder ein aus den proteinogenen Aminosäuren ausgewählter Aminosäurerest ist, worin R wie oben definiert ist.

2. Multimeres Molekül nach Anspruch 1, **dadurch gekennzeichnet, dass** B einer der folgenden allgemeinen Formeln entspricht:

$-R^3-Y-R^4$ oder 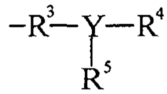

worin

* Y eine $C_1$-$C_{10}$-Alkylkette oder eine Alkinyl- oder Alkenyl- oder Aryl- oder Aralkyl- oder Heteroarylgruppe darstellt,
* $R^3$ eine -NH- Gruppe darstellt, wenn V oder $R_a$ eine -CO- Gruppe ist, oder eine -CO- Gruppe darstellt, wen V oder $R_a$ eine -NH- Gruppe ist,
* $R^4$ und $R^5$ unabhängig voneinander eine -CO- oder -NH- Gruppe darstellen.

**3.** Molekül nach Anspruch 1 oder 2, **dadurch gekennzeichnet, dass** -D und -D' Peptide repräsentieren, die vom humanen oder murinen CD40-Rezeptor Liganden (CD40L) abgeleitet sind, wobei diese Peptide der Primärsequenz des Liganden CD40L von CD40 angehören und ihre Aminosäureanzahl zwischen 3 und 10 liegt.

**4.** Molekül nach einem der Ansprüche 1 bis 3, **dadurch gekennzeichnet, dass** die vom humanen oder murinen CD40-Rezeptor Liganden (CD40L) abgeleiteten Peptide aus folgenden ausgewählt sind:

Lys$^{143}$-Gly-Tyr$^{145}$ (SEQ ID NO: 1), Tyr$^{145}$-Gly-Lys$^{143}$ (SEQ ID NO: 2),
Lys$^{143}$-Gly-Tyr-Tyr$^{146}$ (SEQ ID NO: 3), Tyr$^{146}$-Tyr-Gly-Lys$^{143}$ (SEQ ID NO: 4),
Lys-Pro-Arg (SEQ ID NO: *5)*, Lys-$\psi$($CH_2$NH)Pro-Arg,
Arg$^{200}$-Phe-Glu-Arg-Ile-Leu-Leu-Arg$^{207}$ (SEQ ID NO: 6),
Arg$^{207}$-Leu-Leu-Ile-Arg-Glu-Phe-Arg$^{200}$ (SEQ ID NO: 7),
Arg$^{200}$-Phe-Glu-Ara-Ile$^{204}$ (SEQ ID NO: 8), Ile$^{204}$-Arg-Glu-Phe-Arg$^{200}$ (SEQ ID NO: 9),
Arg$^{203}$-Ile-Leu-Leu-Arg$^{207}$ (SEQ ID NO: 10), Arg$^{207}$-Leu-Leu-Ile-Arg$^{203}$ (SEQ ID NO: 11),
Cys$^{218}$-Gly-Gln-Gln-Ser-Ile$^{223}$ (SEQ ID NO: 12),
Ile$^{223}$-Ser-Gln-Gln-Gly-Cys$^{218}$ (SEQ ID NO: 13),
Gly$^{200}$-Ser-Glu-Arg-Ile-Leu-Leu-Lys$^{207}$ (SEQ ID NO: 14),
Lys$^{207}$-Leu-Leu-Ile-Arg-Glu-Ser-Gly$^{200}$ (SEQ ID NO: 15),
Gly$^{200}$-Ser-Glu-Arg-Ile$^{204}$ (SEQ ID NO: 16), Ile$^{204}$-Arg-Glu-Ser-Gly$^{200}$ (SEQ ID NO: 17),
Arg$^{203}$-Ile-Leu-Leu-Lys$^{207}$ (SEQ ID NO: 18), Lys$^{207}$-Leu-Leu-Ile-Arg$^{203}$ (SEQ ID NO: 19),
Cys$^{218}$-Glu-Gln-Gln-Ser-Val$^{223}$ (SEQ ID NO: 20),
Val$^{223}$-Ser-Gln-Glu-Glu-Cys$^{218}$ (SEQ ID NO: 21),

oder aus Hybridpeptiden, bestehend aus mindestens 2 aufeinander folgenden Aminosäuren von 2 oben definierten Sequenzen, insbesondere den Peptiden mit den Sequenzen Arg$^{203}$-Ile$^{204}$-Tyr$^{145}$-Tyr$^{146}$ (SEQ ID NO: 22) oder Arg$^{203}$-Ile$^{204}$-Tyr$^{146}$-Tyr$^{145}$-Gly$^{144}$-Lys$^{143}$ (SEQ ID NO: 23),
oder aus Fragmenten der genannten Sequenzen,
wobei die Aminosäuren gleichermaßen L- oder D-Konfiguration haben können.

**5.** Multimeres Molekül nach einem der Ansprüche 1 bis 4, **dadurch gekennzeichnet, dass** A $C_3$-Symmetrie hat.

**6.** Molekül nach einem der Ansprüche 1 bis 5, **dadurch gekennzeichnet, dass** A einer der folgenden Formeln entspricht:

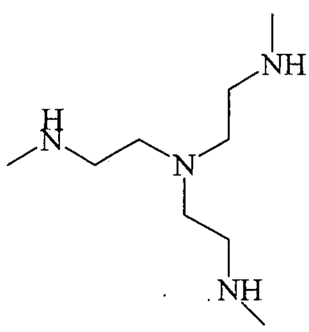

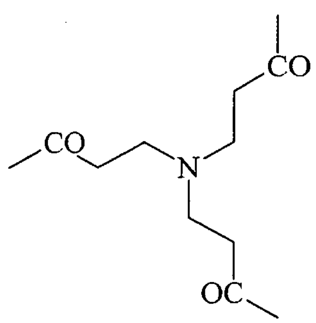

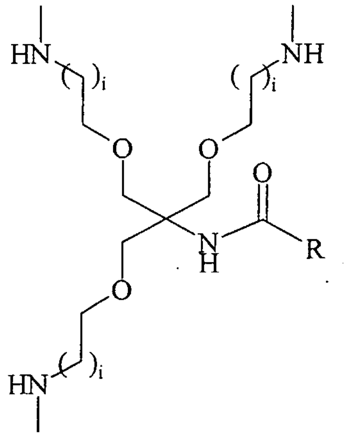

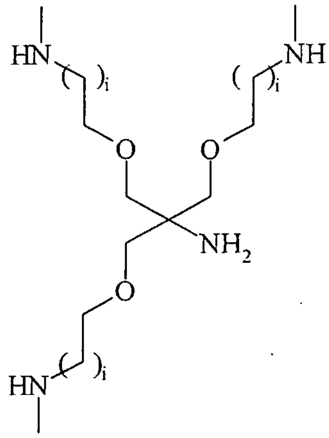

worin i eine ganze Zahl darstellt, die größer oder gleich 1 ist.

7. Molekül nach einem der Ansprüche 1 bis 6 mit der folgenden Formel:

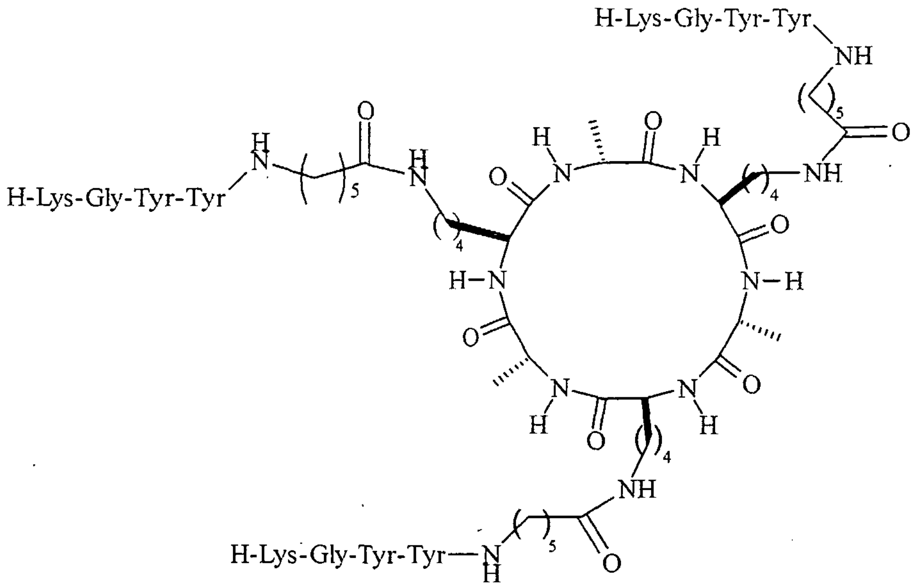

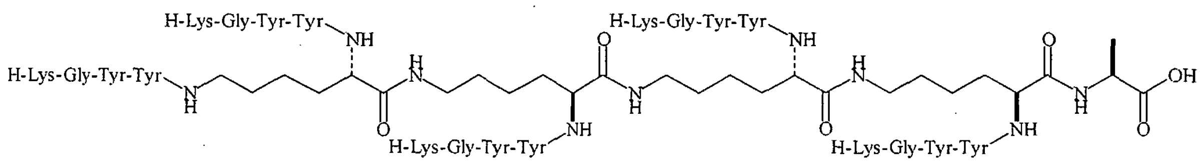

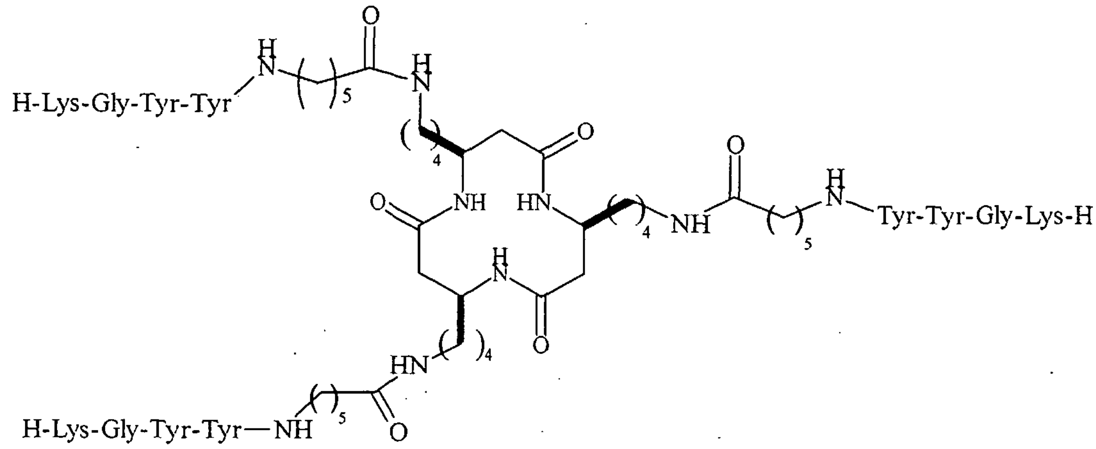

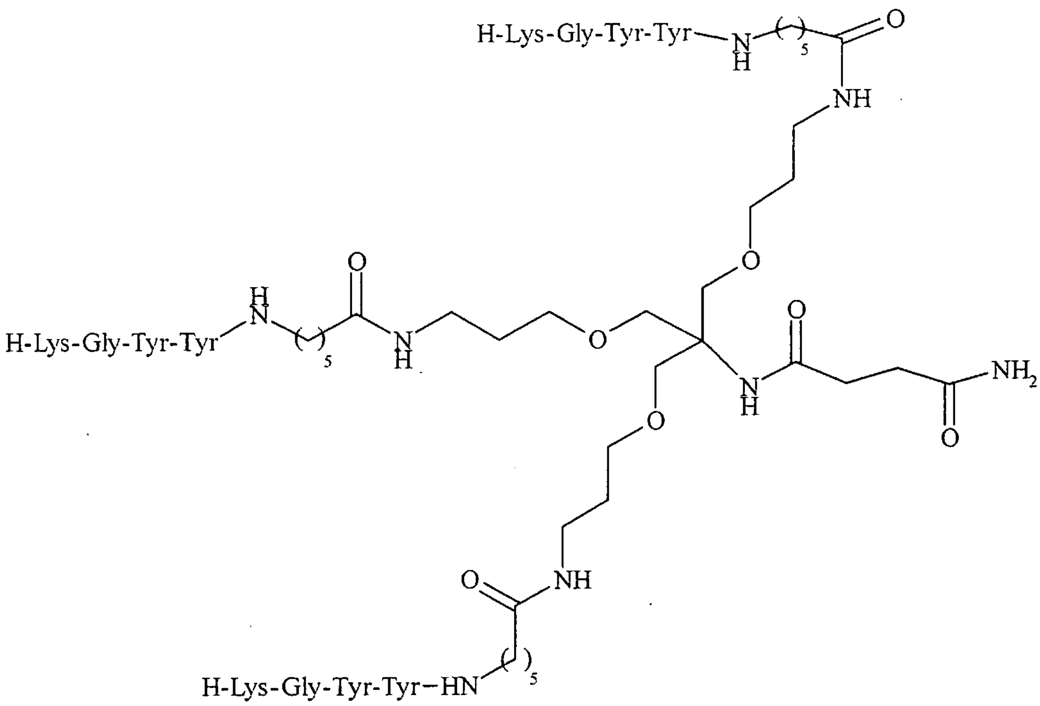

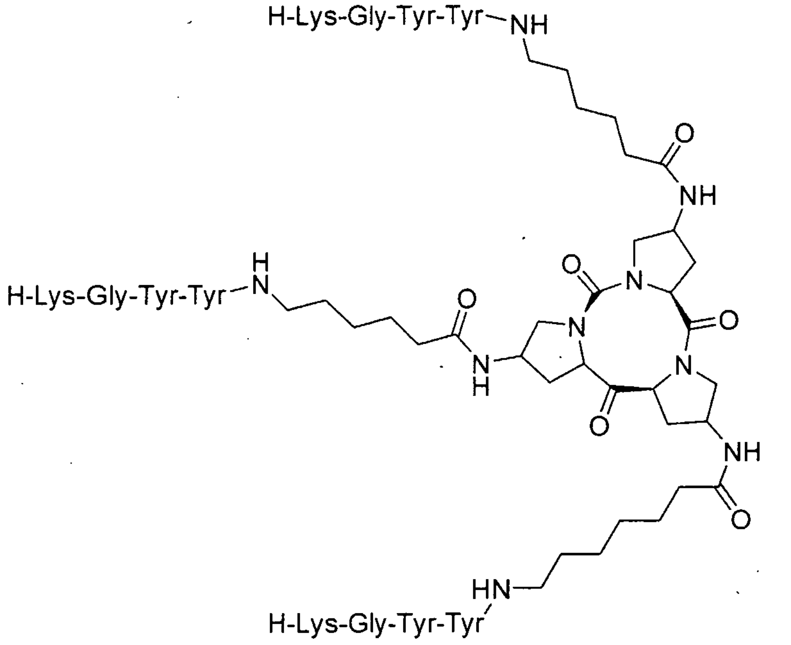

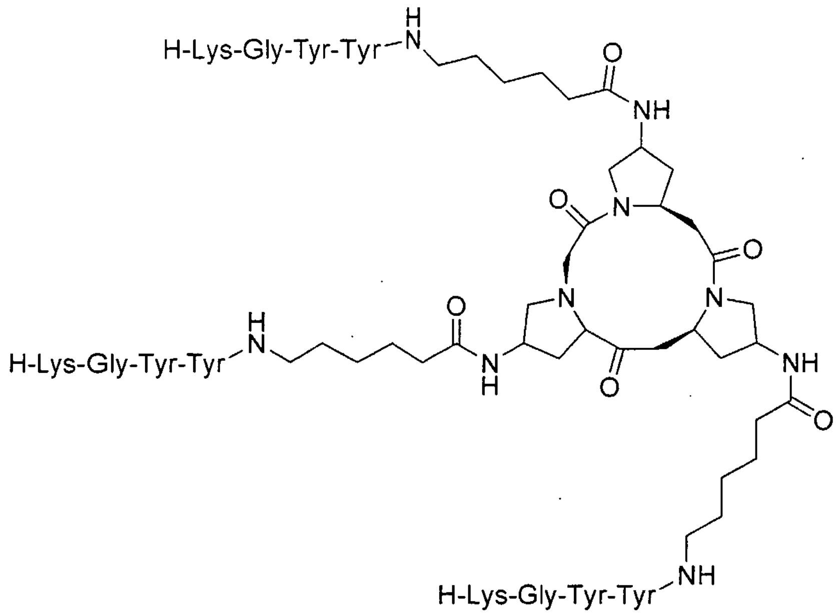

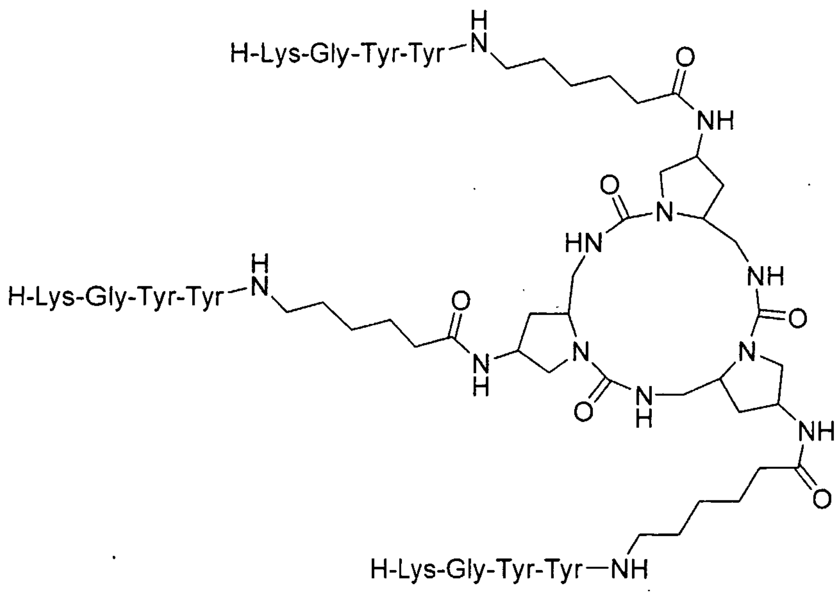

**8.** Pharmazeutische Zusammensetzung, **dadurch gekennzeichnet, dass** sie als Wirkstoff ein multimeres Molekül nach einem der Ansprüche 1 bis 7 zusammen mit einem pharmazeutisch akzeptablen Träger enthält.

**9.** Impfzusammensetzung, **dadurch gekennzeichnet, dass** sie als Wirkstoff ein multimeres Molekül nach einem der Ansprüche 1 bis 7 zusammen mit einem pharmazeutisch akzeptablen Adjuvans enthält.

**10.** Verwendung von multimeren Molekülen nach einem der Ansprüche 1 bis 7 zur Herstellung eines Medikaments zur Behandlung von Pathologien, wobei die Hemmung oder Aktivierung der Immunantwort involviert ist.

**11.** Verwendung nach Anspruch 10 zur Herstellung eines Medikaments zur Behandlung von Pathologien, wobei die Hemmung der Immunantwort involviert ist, wie Abstoßung von Transplantaten oder Autoimmunerkrankungen.

**12.** Verwendung nach Anspruch 10 zur Herstellung eines Medikaments zur Behandlung von Pathologien, wobei die Verstärkung der Immunantwort involviert ist, wie Krebs oder parasitäre, bakterielle oder virale Infektionen.

**13.** Verfahren zur Herstellung eines multimeren Moleküls nach einem der Ansprüche 1 bis 7, worin A ein cyclischer Rest mit $C_3$ gemäß einer der Formeln Ia, Ib, II, VIb, VIc oder VId ist, wie im Anspruch 5 definiert, auf einem festen Träger, welches Verfahren **dadurch gekennzeichnet ist, dass** es folgende Schritte umfasst:

- Bildung eines linearen Vorläufers von A, wobei der Vorläufer aus einer Aneinanderreihung von Aminosäuren besteht, die eine wachsende Peptidkette bilden, die synthetisiert wird in aufeinander folgenden Zyklen von Kupplung zwischen N-geschützten Aminosäureresten, von denen drei eine Gruppe $R_a$ vom Amintyp aufweisen, und der Aminfunktion der wachsenden Peptidkette und Entschützen, wobei der erste Aminosäurerest an einen festen Träger gekuppelt ist,
- Cyclisieren des oben genannten geschützten linearen Vorläufers von A,
- Abspalten der Schutzgruppen zum Freisetzen der geschützten Aminfunktionen,
- Kupplung der drei freigesetzten Aminfunktionen mit einer N-geschützten Spacer-Gruppe B,
- Entschützen der Spacer-Gruppe B und Kupplung der freigesetzten Aminfunktionen der Spacer-Gruppe B mit einem Peptid D, das bereits gebildet wurde oder *in situ* durch sequentielles Aneinanderfügen der dem Peptid D entsprechenden Aminosäurereste gebildet wird, und
- Abspalten des Moleküls von dem festen Träger nach der Beseitigung aller Schutzgruppen in den funktionalisierten Seitenketten des Peptids D, um das Multimer, wie es in einem der Ansprüche 1 bis 7 definiert ist, zu erhalten.

**14.** Verfahren zur Herstellung eines multimeren Moleküls nach einem der Ansprüche 1 bis 7, worin A ein cyclischer Rest mit $C_3$ gemäß einer der Formeln Ia, Ib, II, VIb, VIc oder VId ist, wie im Anspruch 5 definiert, in Lösung, welches Verfahren **dadurch gekennzeichnet ist, dass** es folgende Schritte umfasst:

- Bildung eines linearen Vorläufers von A, wobei der Vorläufer aus einer Aneinanderreihung von Aminosäuren besteht, die eine wachsende Peptidkette bilden, die synthetisiert wird in aufeinander folgenden Zyklen von Kupplung zwischen N-geschützten Aminosäureresten, von denen drei eine Gruppe $R_a$ vom Amintyp aufweisen, und der Aminfunktion der wachsenden Peptidkette und Entschützen,
- Cyclisieren des oben genannten geschützten Vorläufers von A,
- Abspalten der Schutzgruppen zum Freisetzen der geschützten Aminfunktionen,
- Kupplung der drei freigesetzten Aminfunktionen mit einem Peptid -D-B, das einer an ein geschütztes Peptid D gebundenen Spacer-Gruppe B entspricht,
- Entfernen der auf dem Peptid D vorhandenen Schutzgruppen, um das multimer Molekül, wie es in einem der Ansprüche 1 bis 7 definiert ist, zu erhalten.

**15.** Verfahren zur Herstellung eines multimeren Moleküls nach einem der Ansprüche 1 bis 7, worin A ein verzweigter Rest mit $C_3$ gemäß einer der Formeln IV, V, VI oder VIa ist, wie im Anspruch 5 definiert, welches Verfahren **dadurch gekennzeichnet ist, dass** es folgende Schritte umfasst:

- Kupplung der drei Aminfunktionen des Restes A der Formel IV, V, VI oder VIa mit einer geschützten Spacer-Gruppe B,
- Entschützen der Spacer-Gruppe B,
- Zusammenfügen der entschützten Spacer-Gruppe B mit geschützten Aminosäuren, die in die Bildung eines Peptids D eintreten, in aufeinander folgenden Zyklen von Kupplung, Reinigung und Entschützen der genannten Aminosäuren,
- Entschützen der letzten Aminosäure, die in die Bildung des Peptids D eintritt, um das multimer Molekül, wie es in einem der Ansprüche 1 bis 7 definiert ist, zu erhalten.

**16.** Verfahren zur Herstellung eines multimeren Moleküls nach einem der Ansprüche 1 bis 7, worin A ein unsymmetrischer verzweigter Rest gemäß einer der Formeln VII oder VIII ist, wie im Anspruch 5 definiert, auf einem festen Träger, welches Verfahren **dadurch gekennzeichnet ist, dass** es folgende Schritte umfasst:

- Aufpfropfen von Lysin auf einen festen Träger, wobei die beiden Aminfunktionen des Lysins, nämlich in $\alpha$- und $\varepsilon$-Position, mit unterschiedlichen und orthogonalen Schutzgruppen geschützt sind,
- Verlängerung der vom Lysin ausgehend gebildeten Peptidkette auf die gewünschte Länge durch aufeinander folgende Kupplungen und Schutzgruppenentfernungen,

  * nur bei Aminfunktionen in $\alpha$-Position, um den Rest A mit der Formel VII mit geschützten Aminfunktionen in $\varepsilon$-Position zu erhalten, oder
  * nur bei Aminfunktionen in $\varepsilon$-Position, um den Rest A mit der Formel VIII mit geschützten Aminfunktionen in $\alpha$-Position zu erhalten,

- Kupplung der entschützten Aminfunktionen in $\varepsilon$-Position im Rest A der Formel VII oder in $\alpha$-Position im Rest A der Formel VIII mit einer geschützten Gruppe B,
- Zusammenfügen der entschützten Spacer-Gruppe B mit einem Peptid D, das bereits gebildet wurde oder *in situ* durch sequentielles Aneinanderfügen der dem Peptid D entsprechenden Aminosäurereste gebildet wird, und
- Abspalten des so erhaltenen Moleküls von dem festen Träger nach der Beseitigung aller Schutzgruppen in den funktionalisierten Seitenketten des Peptids D.

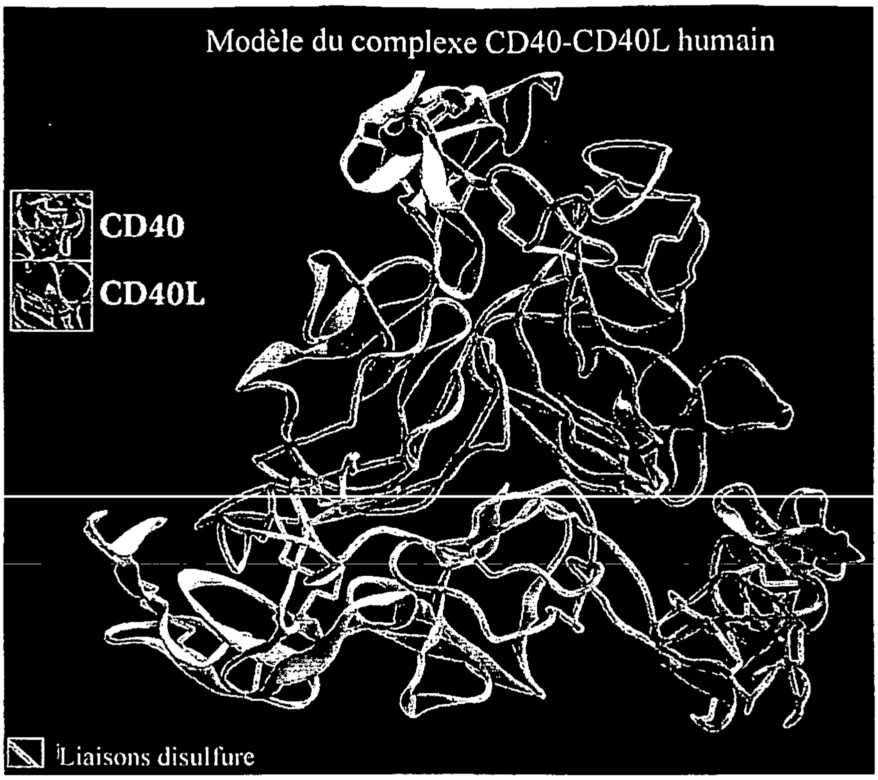


FIGURE 1A

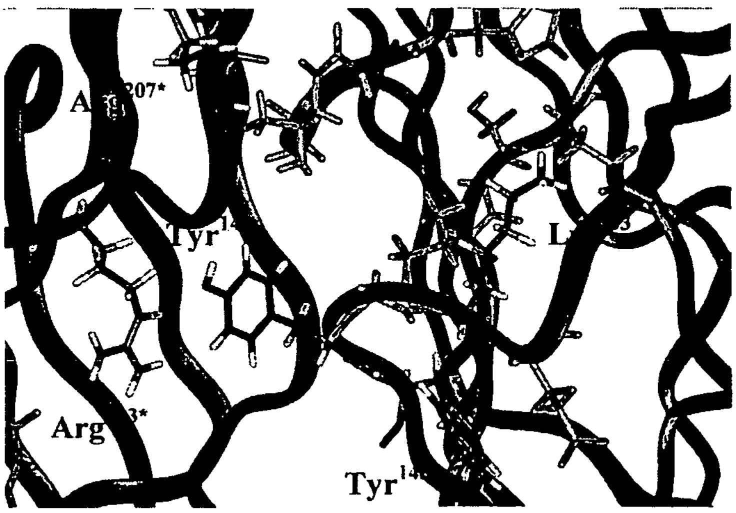


FIGURE 1B

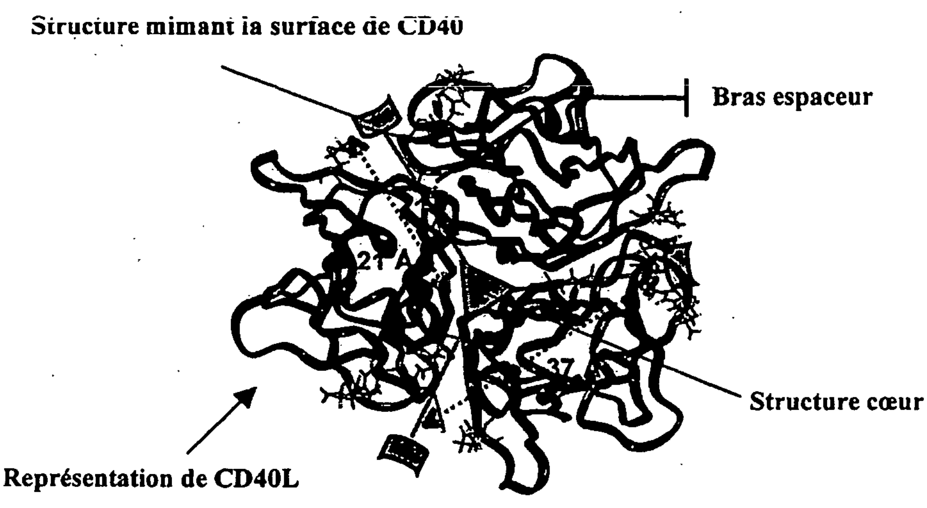
Structure mimant la surface de CD40
Bras espaceur
21 A
37
Structure cœur
Représentation de CD40L

FIGURE 2

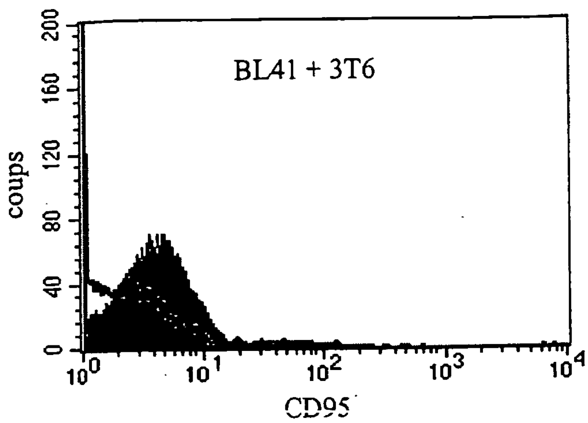


FIGURE 3A

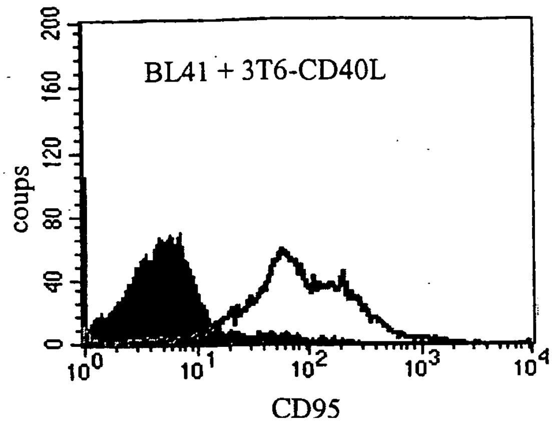


FIGURE 3B

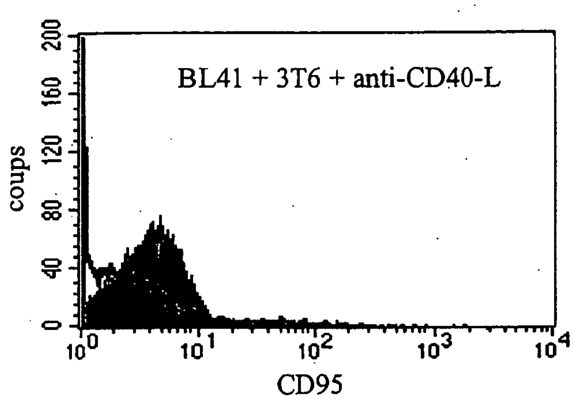


FIGURE 3C

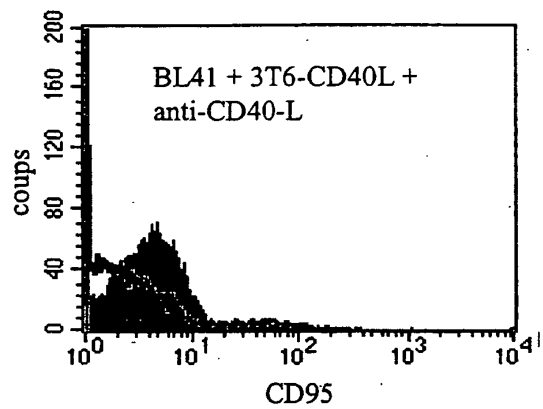


FIGURE 3D

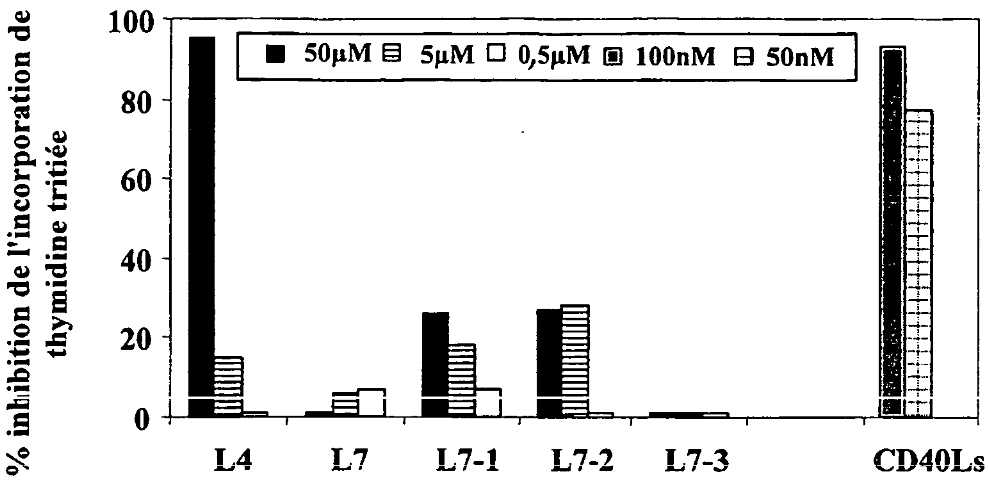


**Figure 4A**

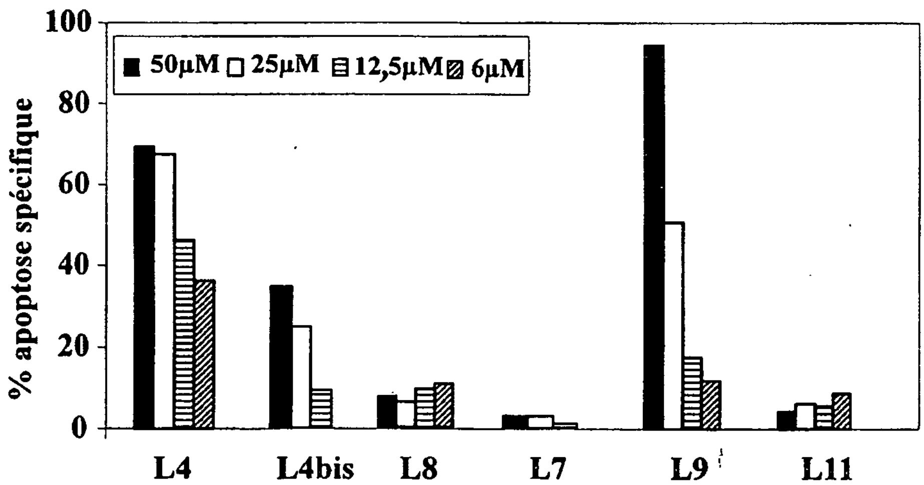


**Figure 4B**

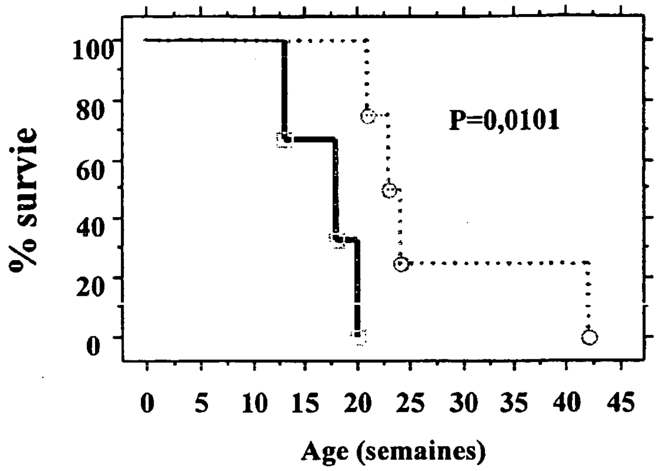


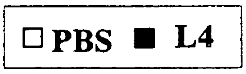


Figure 5A

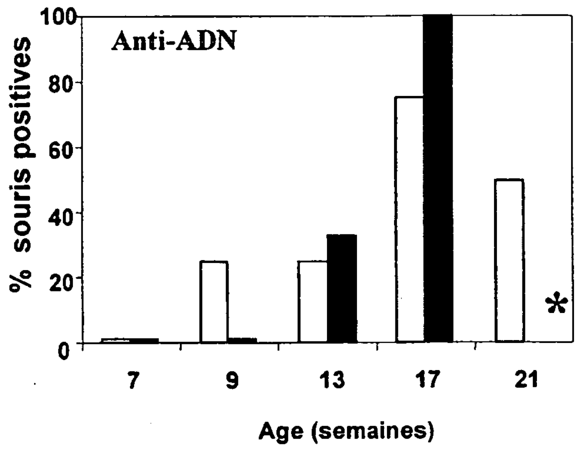


Figure 5B

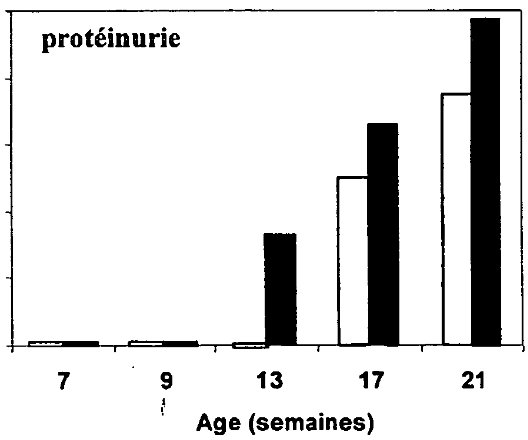


Figure 5C

LISTE DE SÉQUENCES

<110> CENTRE NATIONAL DE LA RECHERCHE SCIENTIFIQUE

<120> NOUVELLES MOLÉCULES MULTIMÉRIQUES, LEUR PROCÉDÉ DE PRÉPARATION, ET LEURS UTILISATIONS POUR LA PRÉPARATION DE MÉDICAMENTS

<130> WOB 02 AE CNR MULT

<140> PCT/FR03/01613
<141> 2003-05-28

<150> FR 02/06631
<151> 2002-05-30

<160> 23

<170> PatentIn version 3.1

<210> 1
<211> 3
<212> PRT
<213> séquence artificielle

<220>
<223> peptide dérivé du ligand du récepteur CD40

<400> 1

```
Lys Gly Tyr
1
```

<210> 2
<211> 3
<212> PRT
<213> séquence artificielle

<220>
<223> peptide dérivé du ligand du récepteur CD40

<400> 2

```
Tyr Gly Lys
1
```

<210> 3
<211> 4
<212> PRT
<213> séquence artificielle

<220>
<223> peptide dérivé du ligand du récepteur CD40

<400> 3

```
Lys Gly Tyr Tyr
1
```

<210> 4
<211> 4
<212> PRT
<213> séquence artificielle

<220>
<223> peptide dérivé du ligand du récepteur CD40

<400> 4

Tyr Tyr Gly Lys
1

<210> 5
<211> 3
<212> PRT
<213> séquence artificielle

<220>
<223> peptide dérivé du ligand du récepteur CD40

<400> 5

Lys Pro Arg
1

<210> 6
<211> 8
<212> PRT
<213> séquence artificielle

<220>
<223> peptide dérivé du ligand du récepteur CD40

<400> 6

Arg Phe Glu Arg Ile Leu Leu Arg
1 5

<210> 7
<211> 8
<212> PRT
<213> séquence artificielle

<220>
<223> peptide dérivé du ligand du récepteur CD40

<400> 7

Arg Leu Leu Ile Arg Glu Phe Arg
1 5

<210> 8
<211> 5

<212> PRT
<213> séquence artificielle

<220>
<223> peptide dérivé du ligand du récepteur CD40

<400> 8

```
Phe Arg Glu Arg Ile
1               5
```

<210> 9
<211> 5
<212> PRT
<213> séquence artificielle

<220>
<223> peptide dérivé du ligand du récepteur CD40

<400> 9

```
Ile Arg Glu Phe Arg
1               5
```

<210> 10
<211> 5
<212> PRT
<213> séquence artificielle

<220>
<223> peptide dérivé du ligand du récepteur CD40

<400> 10

```
Arg Ile Leu Leu Arg
1               5
```

<210> 11
<211> 5
<212> PRT
<213> séquence artificielle

<220>
<223> peptide dérivé du ligand du récepteur CD40

<400> 11

```
Arg Leu Leu Ile Arg
1               5
```

<210> 12
<211> 6
<212> PRT
<213> séquence artificielle

<220>
<223> peptide dérivé du ligand du récepteur CD40

<400> 12

```
Cys Gly Gln Gln Ser Ile
1               5
```

<210> 13
<211> 6
<212> PRT
<213> séquence artificielle

<220>
<223> peptide dérivé du ligand du récepteur CD40

<400> 13

```
Ile Ser Gln Gln Glu Gly
1               5
```

<210> 14
<211> 8
<212> PRT
<213> séquence artificielle

<220>
<223> peptide dérivé du ligand du récepteur CD40

<400> 14

```
Gly Ser Glu Arg Ile Leu Leu Lys
1               5
```

<210> 15
<211> 8
<212> PRT
<213> séquence artificielle

<220>
<223> peptide dérivé du ligand du récepteur CD40

<400> 15

```
Lys Leu Leu Ile Arg Glu Ser Gly
1               5
```

<210> 16
<211> 5
<212> PRT
<213> séquence artificielle

<220>
<223> peptide dérivé du ligand du récepteur CD40

<400> 16

Gly Ser Glu Arg Ile
1 5

<210> 17
<211> 5
<212> PRT
<213> séquence artificielle

<220>
<223> peptide dérivé du ligand du récepteur CD40

<400> 17

Ile Arg Glu Ser Gly
1 5

<210> 18
<211> 5
<212> PRT
<213> séquence artificielle

<220>
<223> peptide dérivé du ligand du récepteur CD40

<400> 18

Arg Ile Leu Leu Lys
1 5

<210> 19
<211> 5
<212> PRT
<213> séquence artificielle

<220>
<223> peptide dérivé du ligand du récepteur CD40

<400> 19

Lys Leu Leu Ile Arg
1 5

<210> 20
<211> 6
<212> PRT
<213> séquence artificielle

<220>
<223> peptide dérivé du ligand du récepteur CD40

<400> 20

Cys Glu Gln Gln Ser Val
1 5

<210> 21
<211> 6
<212> PRT
<213> séquence artificielle

<220>
<223> peptide dérivé du ligand du récepteur CD40

<400> 21

Val Ser Gln Gln Glu Cys
1 5

<210> 22
<211> 4
<212> PRT
<213> séquence artificielle

<220>
<223> peptide dérivé du ligand du récepteur CD40

<400> 22

Arg Ile Tyr Tyr
1

<210> 23
<211> 6
<212> PRT
<213> séquence artificielle

<220>
<223> peptide dérivé du ligand du récepteur CD40

<400> 23

Arg Ile Tyr Tyr Gly Lys
1 5